(19) Europäisches Patentamt
European Patent Office
Office européen des brevets

(11) EP 4 382 604 A1

(12) **EUROPEAN PATENT APPLICATION**
published in accordance with Art. 153(4) EPC

(43) Date of publication:
**12.06.2024 Bulletin 2024/24**

(21) Application number: **22852368.4**

(22) Date of filing: **05.08.2022**

(51) International Patent Classification (IPC):
***C12N 15/115*** *(2010.01)* ***C09K 11/06*** *(2006.01)*
***C12N 15/10*** *(2006.01)* ***G01N 21/64*** *(2006.01)*

(52) Cooperative Patent Classification (CPC):
**C09K 11/06; C12N 15/10; C12N 15/115; G01N 21/64; G01N 33/53**

(86) International application number:
**PCT/CN2022/110700**

(87) International publication number:
**WO 2023/011657 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.08.2021 CN 202110902666**

(71) Applicant: **Fluorescence Diagnosis (shanghai) Biotech Company Ltd.**
**Shanghai 200231 (CN)**

(72) Inventor: **ZHANG, Dasheng**
**Shanghai 200030 (CN)**

(74) Representative: **Bandpay & Greuter**
**11 rue Christophe Colomb**
**75008 Paris (FR)**

# (54) METHOD FOR DETECTING AND QUANTIFYING RNA

(57) An aptamer nucleic acid molecule, a complex containing the aptamer and a small fluorophore molecule, a method for detecting intracellular or extracellular RNA, DNA or other target molecules using the aptamer nucleic acid molecule, and a kit containing the aptamer. The aptamer of the present disclosure can specifically bind with a small fluorophore molecule, and can significantly improve the fluorescence intensity thereof under the excitation of light of suitable wavelength.

EP 4 382 604 A1

## Description

### Technical Field

The present disclosure relates to an aptamer nucleic acid molecule, a method for detecting intracellular or extracellular RNA, DNA or other target molecules using the aptamer nucleic acid molecule, and a kit containing the aptamer. The aptamer of the present disclosure can specifically bind to a small fluorophore molecule, and can significantly improve the fluorescence intensity thereof under the excitation of light of suitable wavelength.

### Background Art

RNA in cells has great varieties and complex functions. Developed in recent years, fluorescent RNA can achieve high spatiotemporal resolution imaging of RNA in living cells, thereby helping researchers to understand dynamics of RNA in cells more intuitively, while providing a powerful new tool for disease diagnosis.

At present, relatively mature technical means of RNA imaging mainly include: RNA fluorescence in situ hybridization technology, molecular beacon technology, RNA binding protein-fluorescent protein MCP-FPs system and fluorescent RNA technology:

1. RNA fluorescence in situ hybridization technology is a method that has been widely used for a long time in the study of the level and distribution of RNA in cells, and is a method for fluorescent labeling and then imaging of specific RNA molecules by using molecular hybridization. However, the operation thereof is relatively complicated and contains an elution step, so it can only be used for the study of immobilized cells, i.e. dead cells, rather than for real-time monitoring of RNA dynamic changes in living cells.
2. Molecular beacon is the first developed RNA imaging technology of living cells. It is a stem-loop double-labeled oligonucleotide probe with an auto hairpin structure formed at the 5' and 3' ends; when it binds to the target RNA, the quenching effect of a quenching group labeled at one end on the fluorescence group is eliminated and the fluorescent group produces fluorescence, or FRET of fluorescent groups at both ends disappears. However, Molecular beacons have disadvantages such as low fluorescence signals, difficulty in entering cells, being easily degradable, serious non-specific aggregation in the nucleus, being susceptible to RNA secondary structure and the need of a custom oligonucleotide probe for each RNA, etc., and these shortcomings limit the wide application of this technology.
3. The principal method of RNA imaging for living cells is to use the MCP-FPs system, which can specifically recognize and bind to mRNA molecules fused with multi-copy MS2 sequence, and monitor mRNA synthesis and distribution in real time by means of detecting fluorescent protein signals (Ozawa et al., Nature Methods, 2007. 4:413-419). However, due to the high background fluorescence generated by MCP-FPs not binding to mRNA molecules, the signal-to-noise ratio of this method is very low. Then, scientists added nuclear localization signals to MCP-FPs fusion protein so that GFP-MS2 not binding to mRNA molecules were localized in the nucleus, thereby reducing the non-specific fluorescence in cytoplasm to a certain extent and increasing the signal-to-noise ratio of the detection.
4. Fluorescent RNA technology can be traced back to 2003. Professor Roger Tsien, one of the Nobel Prize winners in chemistry of fluorescent proteins, proposed that RNA aptamers could be used for specifically identifying, binding to and activating dye molecules, thereby achieving visualization of RNA. Based on this principle, his research group obtained, by means of screening, malachite green (MG) dye molecules capable of binding to and activating triphenylmethane dye, and the fluorescence enhancement multiple reached as high as 2360. However, MG itself is a biological stain, and thus cannot be used for RNA labeling in living cells. This technology was actually applied to living cells in 2011 when S. Jaffrey's research group used a SELEX method, namely, DFHBI (3,5-difluoro-4-hydroxybenzyli-dene imidazolinone), a derivative of green fluorescent protein chromophore HBI, was taken as a screening target and a nucleic acid aptamer named as "Spinach" was obtained. Binding with DFHBI, Spinach could significantly enhance fluorescence signals of DFHBI. By using Spinach-DFHBI complexes, they realized tracing of target RNA in mammalian cells for the first time (Paige et al. Science. 2011. 333: 642-646). So far, this method has been successfully applied to the monitoring and analysis of RNA dynamic changes in bacteria, yeast and mammalian cells. Later, this research group respectively optimized nucleic acid aptamer molecules and the fluorophore, and obtained Spinach2-DFHBI-1T, Broccoli-DFHBI-1T (Filonov et al. Journal of the American Chemical Society. 2014. 136: 16299-16308) and Broccoli-BI (Li et al. Angewandte Chemie International Edition in English. 2020. 59: 4511-4518). In 2017, this research group further developed a Corn-DFHO complex used for detecting activity of RNA polymerase III promoters in mammalian cells (Song et al. Nature Chemical Biology. 2017. 13: 1187-1194). In 2019, a joint research team composed of Professor Yi YANG and Professor Linyong ZHU from China made a breakthrough in the field of fluorescent RNA. Based on a brand-new concept of molecular design, they designed

and synthesized a new fluorophore molecule HBC, and screened and obtained a nucleic acid aptamer Pepper, which is highly compatible therewith. At the same time, a series of derivatives were obtained after chemical modification based on HBC, and the spectrum of complex binding with Pepper covered cyan, green, orange and red. The Pepper-HBC620 complex can emit bright red fluorescence, and has good light stability, and thus can realize SIM super-resolution imaging.

To sum up, current RNA labeling technologies have their own shortcomings. Caused by non-specific binding, MCP-FPs labeling technology has strong background fluorescence and low signal-to-noise ratio. RNA labeling technology based on the complex formed of aptamer-fluorophore-quencher currently can be used for RNA labeling only in bacteria, and cannot yet label RNA in mammalian cells. RNA labeling technology based on single fluorophore-nucleic acid aptamer seems to be perfect, but it has not been widely used because there are currently few types of fluorescent RNA capable of biorthogonal and most complexes are located in the green band with strong magnesium ion dependence and poor light stability. Therefore, more effective fluorophore-nucleic acid aptamer complexes, which can overcome shortcomings of previous fluorophore-nucleic acid aptamer complexes in real-time labeling of RNA or DNA in living cells, have always been needed in the scientific and industrial communities.

## Summary

The present disclosure provides a nucleic acid aptamer molecule, a DNA molecule encoding the nucleic acid aptamer molecule, a complex of the nucleic acid aptamer molecule with fluorophore molecules, and use of the complex.

The present disclosure provides a technical solution below.

1. A nucleic acid aptamer molecule, comprising following nucleotide sequences (a), (b) or (c):

(a): a nucleotide sequence $N_1$CUCCAGGUG$N_{11}$-$N_{12}$-$N_{13}$GUAACGGACUUAGAUCCACUGUACG$N_{39}$, wherein $N_1$, $N_{11}$, $N_{12}$, $N_{13}$ and $N_{39}$ represent nucleotide fragments greater than or equal to 1 in length, and at least one base pair in $N_1$ and $N_{39}$ nucleotide sequences forms a complementary pair, and at least one base pair in $N_{11}$ and $N_{13}$ nucleotide sequences forms a complementary pair;
(b): a nucleotide sequence with an identity of at least 71% to the nucleotide sequence limited by (a); and
(c): a nucleic acid aptamer molecule derived from (a) at a position not including $N_1$, $N_{11}$, $N_{12}$, $N_{13}$ and $N_{39}$ in the nucleotide sequence limited by (a), with substitution, missing and/or addition of one or several nucleotides, and having aptamer functions.

2. The nucleic acid aptamer molecule according to claim 1, a sequence having an identity of at least 71%, 74%, 76%, 79%, 82%, 85%, 88%, 91%, 94%, 97% or 100% to the N16 structure nucleotide sequence of (a).
3. The nucleic acid aptamer molecule according to claim 1, wherein the nucleotide sequence (c) is nucleic acid aptamer molecules obtained with substitution, missing and/or addition of 10, 9, 8, 7, 6, 5, 4, 3, 2 or 1 nucleotide at a position in the N16 structure nucleotide sequence limited by (a) not including $N_1$, $N_{11}$, $N_{12}$, $N_{13}$ and $N_{39}$.
4. The nucleic acid aptamer molecule according to claim 3, wherein the nucleotide sequence (c) is nucleic acid aptamer molecules obtained with substitution of 7, 6, 5, 4, 3, 2 or 1 nucleotide at a position in the nucleotide sequence limited by (a) not including $N_1$, $N_{11}$, $N_{12}$, $N_{13}$ and $N_{39}$.
5. The nucleic acid aptamer molecule according to any one of claims 1 to 4, wherein: when $N_1$ and $N_{39}$ in the nucleotide sequence (a) form a complementary pair, a direction of $N_1$ nucleotide sequence is 5'-3', and a direction of $N_{39}$ nucleotide sequence is 3'-5'; and, when $N_{11}$ and $N_{13}$ form a complementary pair, a direction of $N_{11}$ nucleotide sequence is 5'-3', and a direction of $N_{13}$ nucleotide sequence is 3'-5'.
6. The nucleic acid aptamer molecule according to claim 5, wherein: when at least one fragment of $N_1$ and $N_{39}$ is greater than or equal to 5 nucleotide bases in length, at least two pairs of nucleotide bases in $N_1$ and $N_{39}$ nucleotide sequences form complementary pairs; when at least one fragment of $N_{11}$ and $N_{13}$ is greater than or equal to 5 nucleotide bases in length, at least two pairs of nucleotide bases in $N_{11}$ and $N_{13}$ nucleotide sequences form complementary pairs.
7. The nucleic acid aptamer molecule according to any one of claims 1 to 6, wherein the nucleotide substitution for the General Formula N16 structure is selected from one of the following groups: C2A, C2U, C2G, U3A, U3C, U3G, C4A, C4U, C4G, C5A, C5U, C5G, A6U, A6C, A6G, G7A, G7U, G7C, G8A, G8U, G8C, U9A, U9C, U9G, G10A, G10U, G10C, G14A, G14U, G14C, U15A, U15C, U15G, A16U, A16C, A16G, A17U, A17C, A17G, C18A, C18U, C18G, G19A, G19U, G19C, G20A, G20U, G20C, A21U, A21C, A21G, C22A, C22U, C22G, U23A, U23C, U23G, U24A, U24C, U24G, A25U, A25C, A25G, G26A, G26U, G26C, A27U, A27C, A27G, U28A, U28C, U28G, C29A, C29U, C29G, C30A, C30U, C30G, A31U, A31C, A31G, C32A, C32U, C32G, U33A, U33C, U33G, G34A, G34U, G34C, U35A, U35C, U35G, A36U, A36C, A36G, C37A, C37U, C37G, G38A, G38U, G38C, C4G/U9A, C4G/A16G,

C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U3 5G.

8. The nucleic acid aptamer molecule according to claim 7, wherein the nucleotide substitution for the General Formula N16 structure is selected from one of the following groups: C2U, U3A, U3C, U3G, C4A, C4U, C4G, C5A, C5U, C5G, A6U, A6C, A6G, G8A, U9A, U9C, U9G, G10A, U15C, A16U, A16C, A16G, A17G, C18A, C18U, C18G, A21C, A21G, C22A, C22U, C22G, U23A, U23C, U23G, U24A, U24C, U24G, A25U, A25C, A25G, G26A, G26U, G26C, A27U, A27C, A27G, U28A, U28C, U28G, C29U, C29G, C30U, A31U, A31C, A31G, C32A, C32U, C32G, U33A, U33G, G34A, G34U, G34C, U35A, U35C, U35G, A36U, A36C, A36G, C37A, C37U, C37G, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U3 5G.

9. The nucleic acid aptamer molecule according to claim 8, wherein the nucleotide substitution for the General Formula N16 structure is selected from one of the following groups: U3A, U3C, U3G, C4A, C4U, C4G, C5A, C5U, C5G, A6U, A6G, G8A, U9A, U9C, U9G, G10A, U15C, A16U, A16C, A16G, A17G, C18A, C18G, A21C, A21G, C22A, C22U, C22G, U23A, U23C, U24A, U24C, U24G, A25U, A25C, A25G, G26A, G26U, G26C, A27U, A27C, A27G, U28A, U28C, U28G, C29U, C29G, A31C, A31G, C32A, C32U, C32G, G34A, G34U, G34C, U35A, U35C, U35G, A36U, A36C, A36G, C37A, C37U, C37G, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U3 5G.

10. The nucleic acid aptamer molecule according to any one of claims 1 to 9, wherein nucleotide sequences at $N_1$ and $N_{39}$ in the nucleotide sequence (a) are F30 or tRNA scaffold RNA sequences.

11. The nucleic acid aptamer molecule according to any one of the preceding claims, wherein the aptamer molecules are RNA molecules or base-modified RNA molecules.

12. The nucleic acid aptamer molecule according to any one of the preceding claims, wherein the aptamer molecules are DNA-RNA hybrid molecules or base-modified DNA-RNA molecules.

13. The nucleic acid aptamer molecule according to any one of the preceding claims, wherein the aptamer function refers to that the nucleic acid aptamer can enhance fluorescence intensity of fluorophore molecules under excitation light of appropriate wavelength by at least 2 times, at least 5 to 10 times, at least 20 to 50 times, at least 100 to 200 times or at least 200 to 1,000 times.

14. The nucleic acid aptamer molecule according to claim 1, further comprising concatemers that can bind with multiple fluorophore molecules, wherein the concatemers are connected by spacer sequences of appropriate length that may be 2, 3, 4, 5, 6, 7, 8 or more. Nucleotides of the concatemers can be selected from but are not limited to sequences SEQ ID No: 1, 2, 3, 4, 5, 6, 7, 8, 9, 10, 11, 12 and 13.

15. A complex of nucleic acid aptamer molecules and fluorophore molecules, wherein the nucleic acid aptamer molecule is the nucleic acid aptamer molecule according to any one of claims 1 to 17, and the fluorophore molecule has a structure shown by Formula (I) below:

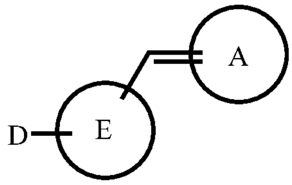

(I)

an electron donor part-D is -NX1-X2, X1 is selected from hydrogen, alkyl or modified alkyl, and X2 is selected from hydrogen, alkyl or modified alkyl; X1 and X2 are optionally interconnected, and form an aliphatic heterocycle with N atoms;
a conjugate system E is formed by at least one conjugate connection selected from double bond, triple bond, aromatic ring and aromatic heterocycle, wherein each hydrogen atom contained therein is optionally and independently substituted by a substituent group selected from halogen atom, hydroxyl group, amino group, primary amino group, secondary amino group, hydrophilic group, alkyl and modified alkyl, and the substituent group is optionally interconnected to form an aliphatic ring or an aliphatic heterocycle;
X1 and X2 are independently connected with the conjugate system E to form an aliphatic heterocycle;
an electron acceptor A optionally forms a ring structure represented by Formula (I-1-a) below:

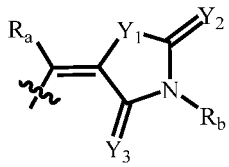

(I-1-a)

Ra is independently selected from hydrogen, halogen atom, nitro group, alkyl group, aryl group, heteroaryl group, hydrophilic group or modified alkyl; $R_b$ is independently selected from hydrogen, halogen atom, hydroxyl group, carboxyl group, amino group, nitro group, alkyl group, aryl group, heteroaryl group, hydrophilic group or modified alkyl, or is a group formed by conjugate connection of the double bond with at least one of the aromatic ring and aromatic heterocycle;
each $Y_1$ is independently selected from -O-, -S-, -(S=O)- and -($NR_i$)-, wherein $R_i$ is selected from hydrogen, amino group, alkyl or modified alkyl;
each $Y_2$ is independently selected from =O, =S, =S=O and =NRi, wherein $R_i$ is selected from hydrogen, alkyl or modified alkyl;
each $Y_3$ is independently selected from =O, =S, =S=O and =NRi, wherein $R_i$ is selected from hydrogen, alkyl or modified alkyl;
or, each $Y_3$ independently is =C ($R_e$)(CN); wherein $R_e$ is selected from hydrogen, ester group, amide group, sulfonic group, sulfamine, and sulfonate ester group;
wherein:

the "alkyl" is $C_1$-$C_{30}$ straight or branched alkyl; preferably, the "alkyl" is $C_1$-$C_{10}$ straight or branched alkyl; preferably, the "alkyl" is $C_1$-$C_7$ straight or branched alkyl; preferably, the "alkyl" is $C_1$-$C_5$ straight or branched alkyl; preferably, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;
the "modified alkyl group" is a group obtained by replacement of any carbon atom of an alkyl group with at least one group selected from a halogen atom, -O-, -OH, -CO-, -CS-, $-NO_2$, -CN, -S-, $-SO_2$-, -(S=O)-,

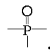,

, phenyl group, phenylene group, primary amino group, secondary amino group, tertiary amino group, quaternary ammonium group, saturated or unsaturated monocyclic or bicyclic cyclic-hydrocarbon group, biaryl heterocyclic group, and a bridged aliphatic heterocyclic group, the modified alkyl group having 1 to 30 carbon atoms, and the carbon-carbon single bond is optionally and independently replaced with a carbon-carbon double bond or a carbon-carbon triple bond;

the replacement of carbon atoms refers to that carbon atoms or the carbon atoms and hydrogen atoms thereon together are replaced by corresponding groups;

the "aliphatic ring" is a saturated or unsaturated 4- to 10-membered monocyclic or polycyclic aliphatic ring;

the "aliphatic heterocycle" is a saturated or unsaturated 4- to 10-membered monocyclic or polycyclic aliphatic heterocycle containing at least one heteroatom selected from N, O, S, or Si; if contained on the aliphatic heterocycle, S atoms are in the form of -S-, -SO-, or $-SO_2-$; the aliphatic heterocycle is optionally substituted with halogen atom, nitro group, alkyl group, aromatic group, hydrophilic group, and modified alkyl group;

the "aromatic group or aromatic ring" is a 5- to 10-membered monocyclic or fused bicyclic aromatic group;

the "heteroaryl or aromatic heterocyclic ring" is a 5- to 10-membered monocyclic or fused bicyclic heteroaromatic group containing at least one heteroatom selected from N, O, S or Si on the ring;

the "halogen atom" is respectively and independently selected from F, Cl, Br, I;

the "hydrophilic group" is hydroxyl group, sulfonic acid group, carboxyl group, phosphite group, primary amino group, secondary amino group, or tertiary amino group;

the "bridged aliphatic heterocycle" is a 5- to 20-membered bridged aliphatic heterocycle containing at least one heteroatom selected from N, O, or S on the ring;

the "ester group" is R(C=O)OR' group;

the "phosphite group" is $RP(=O)(OH)_2$ group;

the "sulfonic group" is $RSO_3H$ group;

the "sulfonate ester group" is $RSO_3R'$ group;

the "sulfamine" is $RSO_2NR'R''$ group;

the "primary amino group" is $RNH_2$ group;

the "secondary amino group" is RNHR' group;

the "tertiary amino group" is RNR'R" group;

the "quaternary ammonium group" is R R'R" R‴$N^+$ group;

each R, R', R", R‴ respectively and independently is single bond, alkyl group, alkylene group, modified alkyl group, or modified alkylene group, and the modified alkyl group or modified alkylene group is a group obtained by replacement of any carbon atom of $C_1$-$C_{10}$ (preferably $C_1$-$C_6$) alkyl or alkylene group with a group selected from -O-, -OH, -CO-, -CS-, -(S=O)-;

optionally, the modified alkyl group or modified alkylene group respectively and independently is a group containing at least one group selected from -OH, -O-, ethylene glycol unit ($-(CH_2CH_2O)n-$), $C_1$-$C_8$ alkyl group, $C_1$-$C_8$ alkoxy group, $C_1$-$C_8$ acyloxy group, $C_1$-$C_8$ haloalkyl group, monosaccharide group, disaccharide group, polysaccharide group, -O-CO-, -NH-CO-, $-(-NH-CHR''''-CO-)_n-$, $-SO_2-O-$, -SO-, $-SO_2-NH-$, -S-S-, -CH=CH-, halogen atom, cyano group, nitro group, o-nitrophenyl group, benzoylmethyl group, and phosphate easter group, wherein n is 1 to 100, preferably 1 to 50, more preferably 1 to 30, more preferably 1 to 10; R'''' is H or residue of $\alpha$ amino acid; the "phosphate easter group" is defined as above;

the "monosaccharide unit" is a saccharide unit that can no longer be simply hydrolyzed into smaller sugar molecules;

the "disaccharide unit" is a saccharide unit formed by dehydration of two monosaccharides;

the "polysaccharide unit" is a saccharide unit formed by dehydration of 10 or more monosaccharides;

optionally, the $C_1$-$C_8$ alkyl group is methyl, ethyl, propyl, or isopropyl, the $C_1$-$C_8$ alkoxy group is methoxy, ethoxy, propoxy, or isopropoxy, the $C_1$-$C_8$ acyloxy is acetoxy, ethyl, propyl, or isopropyl, and the $C_1$-$C_8$ haloalkyl is trifluoromethyl, chloromethyl, or bromomethyl;

optionally, the aliphatic heterocycle is selected from azetidine, pyrrolidine, piperidine, tetrahydrofuran, tetrahydropyran, morpholine, and thiomorpholine;

optionally, the conjugate system E is a structure selected from following Formulae (I-2-1) to (I-2-39):

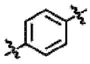 (I-2-1), 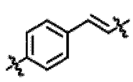 (I-2-2), 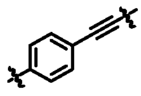 (I-2-3), 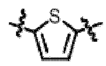 (I-2-4),

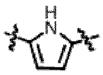 (I-2-5), 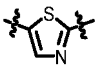 (I-2-6), 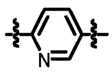 (I-2-7), 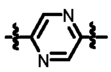 (I-2-8),

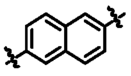 (I-2-9), 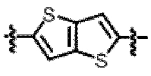 (I-2-10), 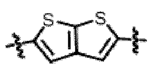 (I-2-11), 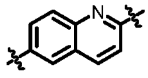 (I-2-12),

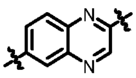 (I-2-13), 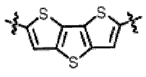 (I-2-14), 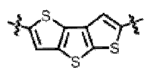 (I-2-15),

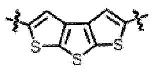 (I-2-16), 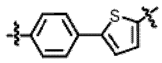 (I-2-17), 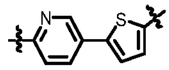 (I-2-18),

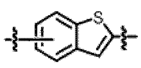 (I-2-19), 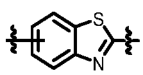 (I-2-20), 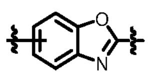 (I-2-21),

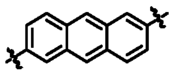 (I-2-22), 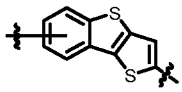 (I-2-23), 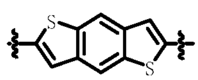 (I-2-24),

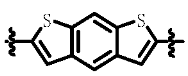 (I-2-25), 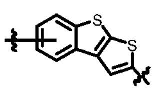 (I-2-26), 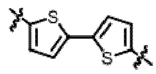 (I-2-27),

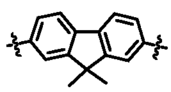 (I-2-28), 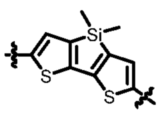 (I-2-29), 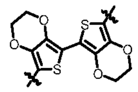 (I-2-30),

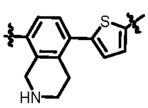 (I-2-31), 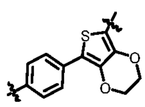 (I-2-32), 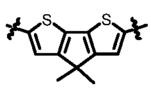 (I-2-33),

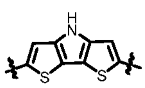 (I-2-34), 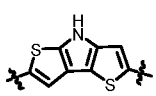 (I-2-35), 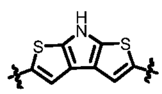 (I-2-36),

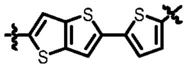 (I-2-37), 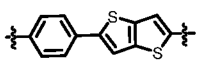 (I-2-38), 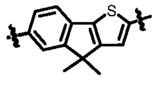 (I-2-39);

optionally, the conjugate system E and $-NX_1-X_2$ form an aliphatic heterocycle represented by Formulae (I-3-1) to (I-3-5):

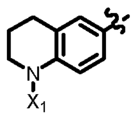 (I-3-1), 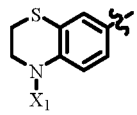 (I-3-2), 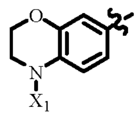 (I-3-3),

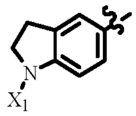 (I-3-4), 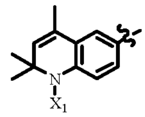 (I-3-5)

or, the conjugate system E and $-NX_1-X_2$ form a structure represented by Formula (I-3-6):

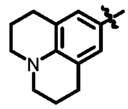 (I-3-6);

and

optionally, the electron acceptor part is one selected from Formulae (I-4-1) to (I-4-42) below:

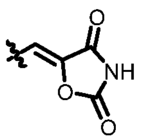 (I-4-1) 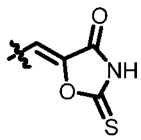 (I-4-2) 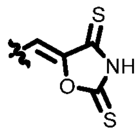 (I-4-3) 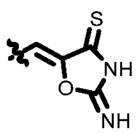 (I-4-4) 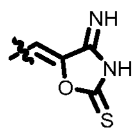 (I-4-5) 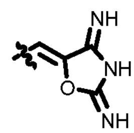 (I-4-6)

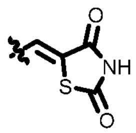 (I-4-7) 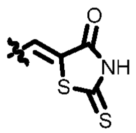 (I-4-8) 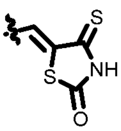 (I-4-9) 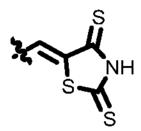 (I-4-10) 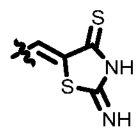 (I-4-11) 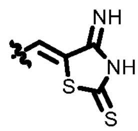 (I-4-12)

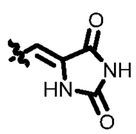 (I-4-13) 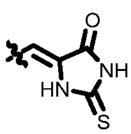 (I-4-14) 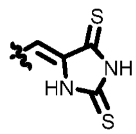 (I-4-15) 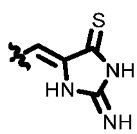 (I-4-16) 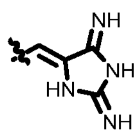 (I-4-17) 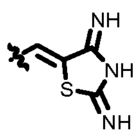 (I-4-18)

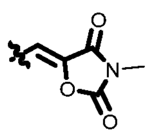 (I-4-19) 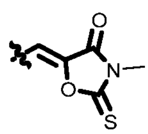 (I-4-20) 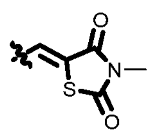 (I-4-21) 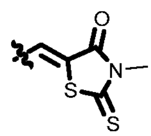 (I-4-22) 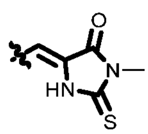 (I-4-23) 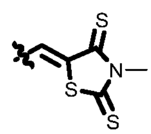 (I-4-24)

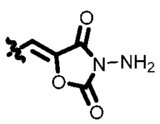 (I-4-25) 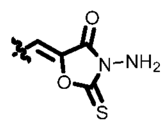 (I-4-26) 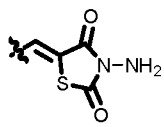 (I-4-27) 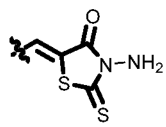 (I-4-28) 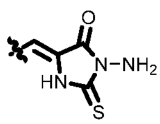 (I-4-29) 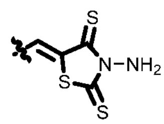 (I-4-30)

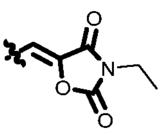
(I-4-31)

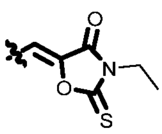
(I-4-32)

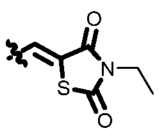
(I-4-33)

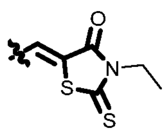
(I-4-34)

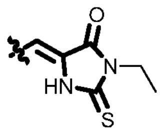
(I-4-35)

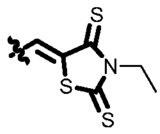
(I-4-36)

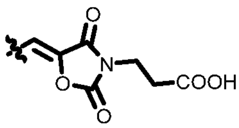
(I-4-37)

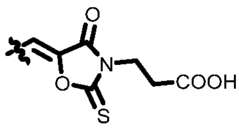
(I-4-38)

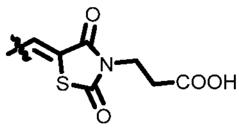
(I-4-39)

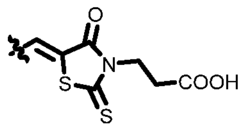
(I-4-40)

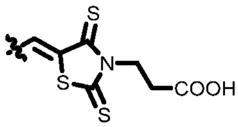
(I-4-41)

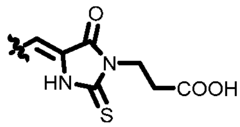
(I-4-42).

16. Optionally, the above-mentioned fluorescent probe, wherein the fluorescent probe formula (I) is selected from compounds represented by the formulae below:

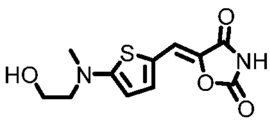
(I-5-1)

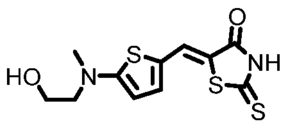
(I-5-2)

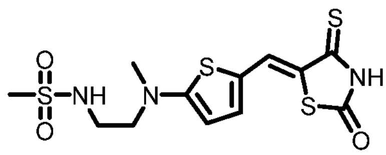
(I-5-3)

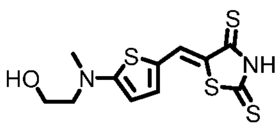
(I-5-4)

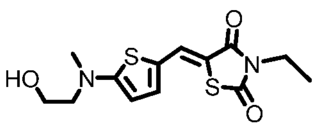
(I-5-5)

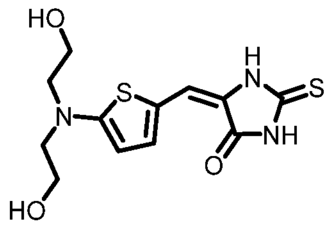
(I-5-6)

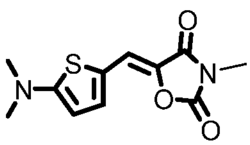
(I-5-7)

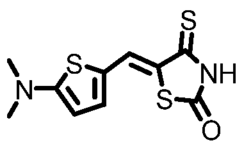
(I-5-8)

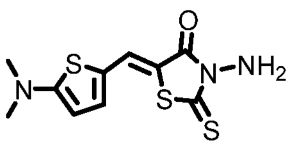
(I-5-9)

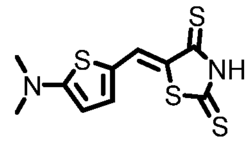

(I-5-10)

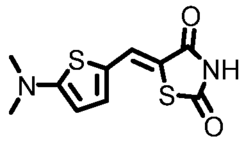

(I-5-11)

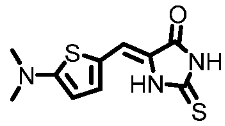

(I-5-12)

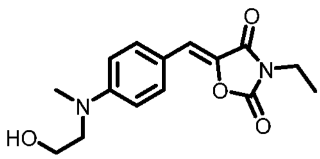

(I-5-13)

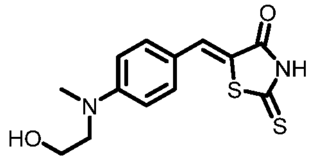

(I-5-14)

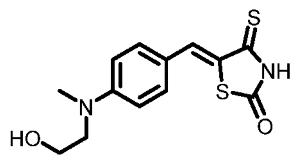

(I-5-15)

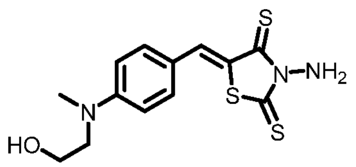

(I-5-16)

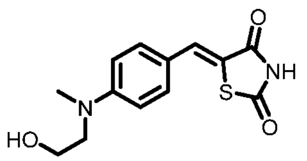

(I-5-17)

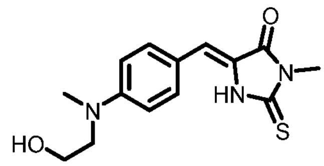

(I-5-18)

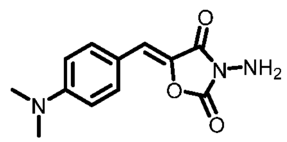

(I-5-19)

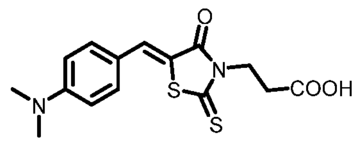

(I-5-20)

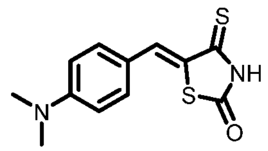

(I-5-21)

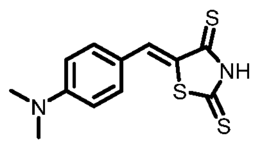

(I-5-22)

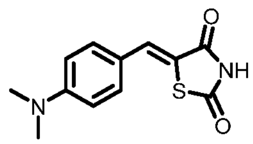

(I-5-23)

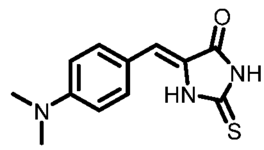

(I-5-24)

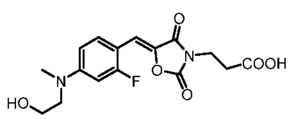

(I-5-25)

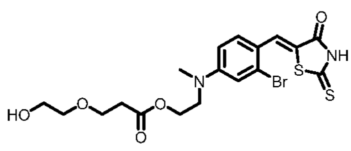

(I-5-26)

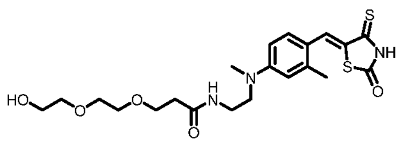

(I-5-27)

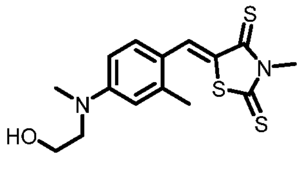

(I-5-28)

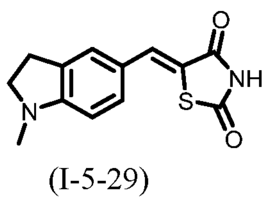

(I-5-29)

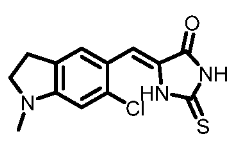

(I-5-30)

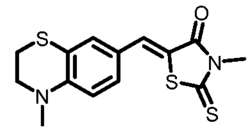
(I-5-31)

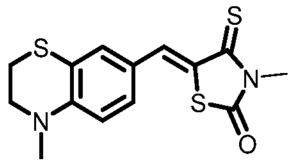
(I-5-32)

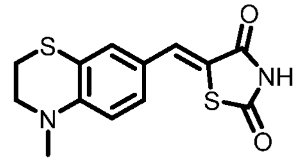
(I-5-33)

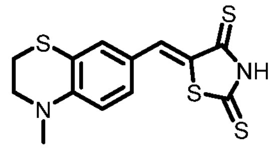
(I-5-34)

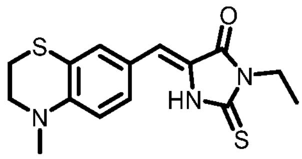
(I-5-35)

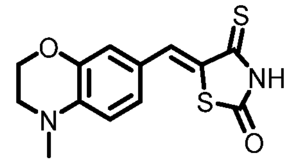
(I-5-36)

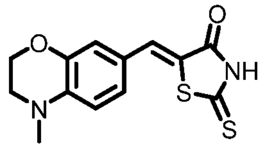
(I-5-37)

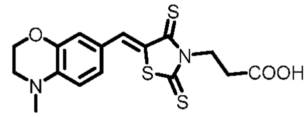
(I-5-38)

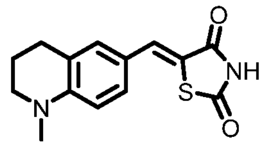
(I-5-39)

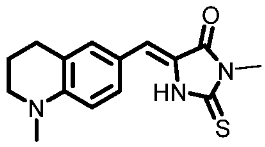
(I-5-40)

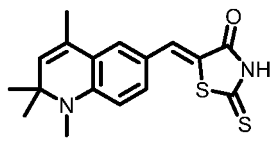
(I-5-41)

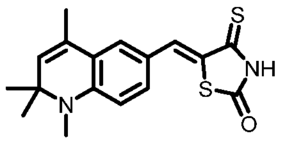
(I-5-42)

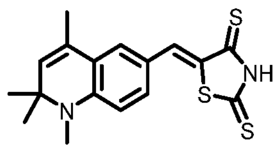
(I-5-43)

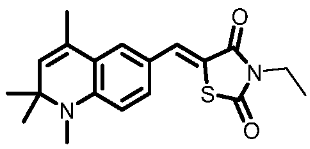
(I-5-44)

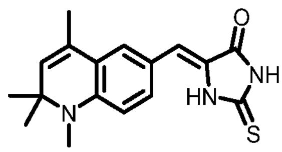
(I-5-45)

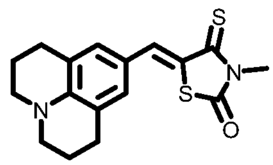
(I-5-46)

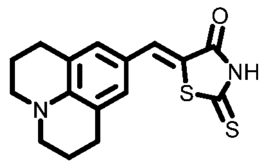
(I-5-47)

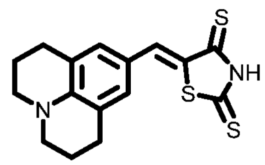
(I-5-48)

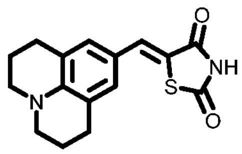
(I-5-49)

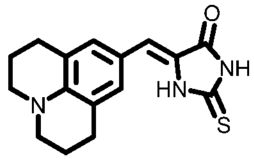
(I-5-50)

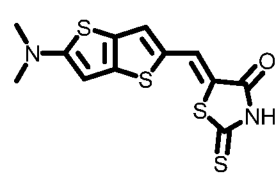
(I-5-51)

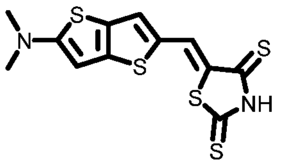
(I-5-52)

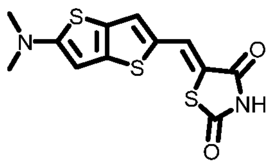
(I-5-53)

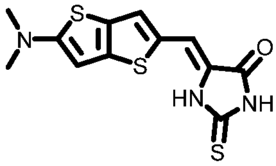
(I-5-54)

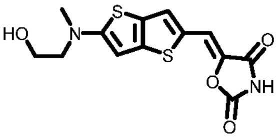
(I-5-55)

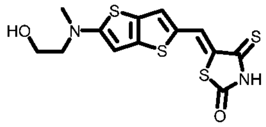
(I-5-56)

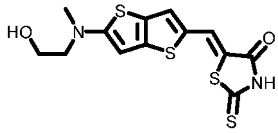
(I-5-57)

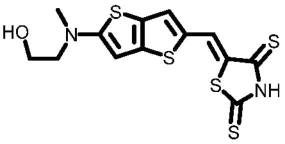
(I-5-58)

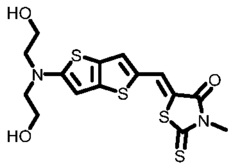
(I-5-59)

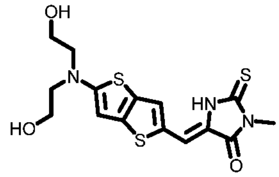
(I-5-60)

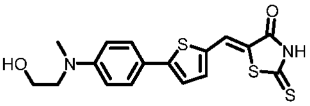
(I-5-61)

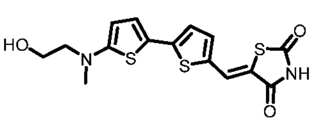
(I-5-62)

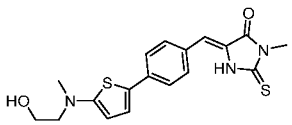
(I-5-63)

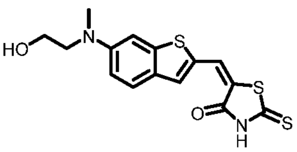
(I-5-64)

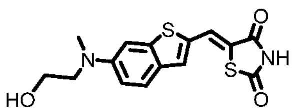
(I-5-65)

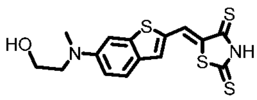
(I-5-66)

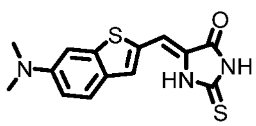
(I-5-67)

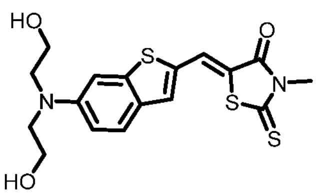
(I-5-68)

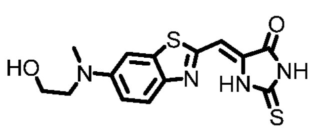
(I-5-69)

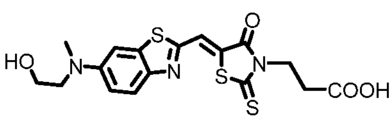
(I-5-70)

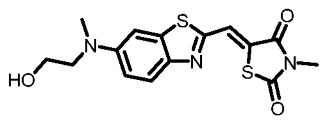
(I-5-71)

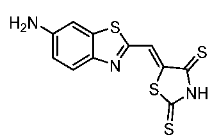
(I-5-72)

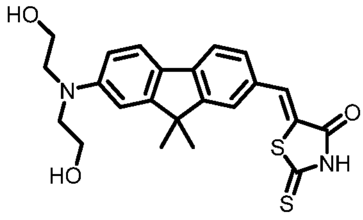
(I-5-73)

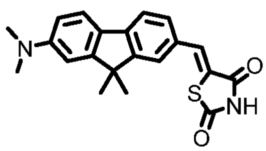
(I-5-74)

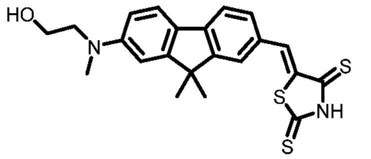
(I-5-75)

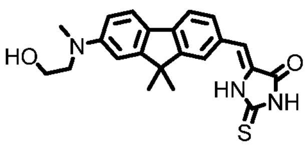
(I-5-76)

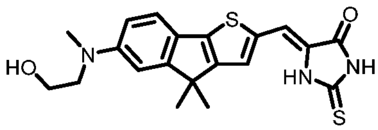
(I-5-77)

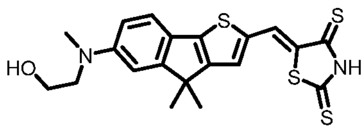
(I-5-78)

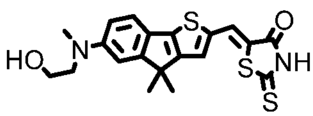
(I-5-79)

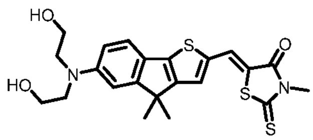
(I-5-80)

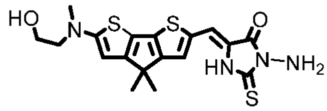
(I-5-81)

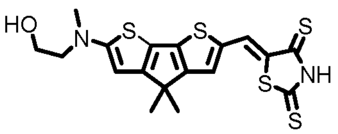
(I-5-82)

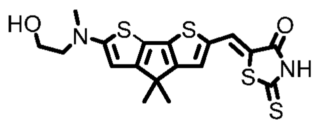
(I-5-83)

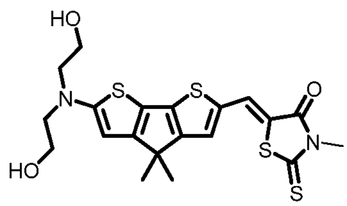
(I-5-84).

17. The complex according to claim 16, wherein the fluorophore molecules in the complex are selected from I-5-1, I-5-2, I-5-3, I-5-4, I-5-5, I-5-6, I-5-7, I-5-8, I-5-9, I-5-10, I-5-11, I-5-12, I-5-13, I-5-14, I-5-15, I-5-16, I-5-17, I-5-18, I-5-19, I-5-20, I-5-21, I-5-22, I-5-23, I-5-24, I-5-25, I-5-26, I-5-27, I-5-28, I-5-29, I-5-30, I-5-31, I-5-32, I-5-33, I-5-34, I-5-35, I-5-36, I-5-37, I-5-38, I-5-39, I-5-40, I-5-41, I-5-42, I-5-43, I-5-44, I-5-45, I-5-46, I-5-47, I-5-48, I-5-49, I-5-50, I-5-51, I-5-52, I-5-53, I-5-54, I-5-55, I-5-56, I-5-57, I-5-58, I-5-59, I-5-60, I-5-61, I-5-62, I-5-63, I-5-64, I-5-65, I-5-66, I-5-67, I-5-68, I-5-69, I-5-70, I-5-71, I-5-72, I-5-73, I-5-74, I-5-75, I-5-76, I-5-77, I-5-78, I-5-79, I-5-80, I-5-81, I-5-82, I-5-83 and I-5-84.

18. The complex according to any one of claims 15 to 17, wherein the aptamer molecules in the complex contain a nucleotide sequence SEQ ID No: 1, 2, 3, 4, 5, 6, 7, 8, 9, 10, 11, 12 and 13.

19. The complex according to any one of claims 15 to 18 for detection or labeling of objective nucleic acid molecules in vitro or in vivo.

20. A DNA molecule, which transcribes the nucleic acid aptamer molecules according to any one of claims 1 to 14.

21. An expression vector containing the DNA molecule according to claim 20.

22. A host cell containing the expression system according to claim 21.

23. A kit, containing the nucleic acid aptamer molecule according to any one of claims 1 to 14 and/or the expression vector according to claim 21 and/or the host cell according to claim 22 and/or the complex according to any one of claims 15 to 18.

24. A method for detecting target molecules, including:

a) adding the complex according to any one of claims 15 to 18 to a solution containing the target molecules;
b) exciting the complex with light of appropriate wavelength; and
c) detecting fluorescence of the complex.

25. A method for extracting and purifying RNA, including extracting and purifying RNA with the complex according to any one of claims 15 to 18.

The inventor designed brand-new nucleic acid aptamer molecules, so as to form a brand-new fluorophore-nucleic acid aptamer complex. After binding of the aptamer molecules with fluorophore molecules, the fluorescence intensity of fluorophore molecules can be significantly increased under the excitation light of appropriate wavelength. They overcome shortcomings of previous fluorophore-nucleic acid aptamer complexes, and can be used for effective and real-time labeling of RNA/DNA in living cells. The nucleic acid aptamer of the present disclosure has rather strong affinity for fluorophore molecules, and shows fine light stability and temperature stability. These nucleic acid aptamer-fluorophore molecule complexes can be used for real-time labeling and imaging of RNA/DNA in prokaryotic and eukaryotic cells, detecting protein-RNA interactions, exploring the relationship between mRNA content and protein in cells, or as labels for RNA extraction and purification.

**Brief Description of Drawings**

FIG. 1 Secondary structure prediction of nucleic acid aptamer molecules. (A) is the predicted general structure of N16, comprising $N_1$ and $N_{39}$ that can form a stem structure, and $N_{11}$, $N_{12}$ and $N_{13}$ that can form a stem-loop structure. (B) is the predicted structure of N16-1, wherein the base sequence of $N_1$ and $N_{39}$ is shown in the dotted box corresponding to Stem 1 in the drawing, and the base sequence of $N_{11}$, $N_{12}$ and $N_{13}$ is shown in the dotted box corresponding to the "stem-loop".

FIG. 2 Secondary structure prediction of N16-1. (A) is the secondary structure prediction of F30-N16-1; (B) is the secondary structure prediction of tRNA-N16-1.

FIG. 3 Spectral property identification of the complex N16-1-I-5-14. (A) is the imaging of the complex N16-1-I-5-14 in NMR tube; (B) is the fluorescence excitation spectrum and emission spectrum of the complex N16-1-I-5-14; (C) is the absorption spectra of the complex N16-1-I-5-14 and the fluorophore molecule I-5-14; (D) is the dissociation constant determination of the binding of N16-1 to I-5-14.

FIG. 4 Property identification of stability of the complex N16-1-I-5-14. (A) is the pH stability determination of the complex N16-1-I-5-14; (B) is the dependence determination of the complex N16-1-I-5-14 on $Mg^{2+}$; (C) is the temperature stability determination of the complex N16-1-I-5-14 (an additional 5 mM of $Mg^{2+}$ is added); (D) is the dependence determination of the complex N16-1-I-5-14 on $K^+$.

FIG. 5 Activation effect for I-5-14 by N16-2 and N16-3, which have different modifications and are on the sequence N16-1. (A) is the secondary structure diagram of N16-2 aptamer containing deoxyribonucleotides (labelled with dark color in the drawing); (B) is the secondary structure diagram of N16-3 aptamer containing 2'F modification (labelled with dark color in the drawing); (C) is the quantitative analysis of the activation effect for I-5-14 by N16-2 and N16-3. "Control" is to replace N16-2 or N16-3 aptamer with buffer.

FIG. 6 Activation effects of different N16-1 concatemers for I-5-14. (A) is obtaining of N16-4 concatemer based on N16-1 by means of "Tandem 1"; (B) is obtaining of N16-5 concatemer based on N16-1 by means of "Tandem 2"; (C) is obtaining of N16-4 concatemer by means of "Tandem 3"; (D) is activation effects of different N16-4 concatemers obtained by means of "Tandem 1" for I-5-14; (E) is activation effects of different N16-5 concatemers obtained by means of "Tandem 2" for I-5-14; (F) is activation effects of different N16-4 concatemers obtained by means of "Tandem 3" for I-5-14.

FIG. 7 Labeling effect of the complex F30-N16-1-I-5-14 on RNA in bacteria (scale: 10 μm).

FIG. 8 Expression of the complex of circular-N16-1 and I-5-14 in mammalian cells (scale: 50 μm).

FIG. 9 Expression of the complex of F30-N16-1 and I-5-14 in mammalian cells (scale: 50 μm).

FIG. 10 Application of the complex N16-1-I-5-14 to the labeling of the mRNA localization of f-actin ACTB (scale: 10 μm).

FIG. 11 Application of N16-1 to quantification of RNA extraction and purification.

**Details**

The following definitions and embodiments referred to in the present disclosure will be described in details here. The contents of all patents and published documents referred to herein, including all sequences disclosed in these patents and published documents, are expressly incorporated herein by reference. Hereinafter, "nucleotides" and "nucleotide bases" are used interchangeably and stand for the same meaning.

Following are detailed explanations of some terms used in the present disclosure.

Nucleic acid aptamer molecules

The "nucleic acid aptamer molecules" of the present disclosure are also referred to as "aptamer molecules". The nucleic acid aptamer molecule contains (a) a nucleotide sequence $N_1$CUCCAGGUG$N_{11}$-$N_{12}$-$N_{13}$GUAACGGACUUAGAUCCACUGUACG$N_{39}$ (corresponding to the General Formula N16 structure in FIG. 1A); or (b) which is a sequence with an identity of at least 71% to the nucleotide sequence of (a); wherein at least one base pair in $N_1$ and $N_{39}$ nucleotide sequences forms a reverse complementary pair, namely, the direction of $N_1$ nucleotide sequence is 5'-3', and the direction of $N_{39}$ nucleotide sequence is 3'-5'. When the length of at least one nucleotide base of $N_1$ and $N_{39}$ is smaller than or equal to 4, at least one base pair is needed for forming the complementary pair; and when the length of at least one nucleotide base of $N_1$ and $N_{39}$ is greater than or equal to 5, at least two base pairs are needed for forming the complementary pair. Wherein, at least one base pair in $N_{11}$ and $N_{13}$ nucleotide sequences forms a reverse complementary pair, namely, the direction of $N_{11}$ nucleotide sequence is 5'-3', and the direction of $N_{13}$ nucleotide sequence is 3'-5'. When the length of at least one nucleotide base of $N_{11}$ and $N_{13}$ is smaller than or equal to 4, at least one base pair is needed for forming the complementary pair; and, when the length of at least one nucleotide base of $N_{11}$ and $N_{13}$ is greater than or equal to 5, at least two base pairs are needed for forming the complementary pair. $N_{12}$ therein is a nucleotide base of any length or composition; or (c) is at any position in the nucleotide sequence (a) with the substitution, missing and/or addition of 1 to 10 nucleotides.

The nucleic acid aptamer molecules contain substitution of the nucleotides in General Formula N16 structure, and the substitution is selected from one of the following groups: C2A, C2U, C2G, U3A, U3C, U3G, C4A, C4U, C4G, C5A, C5U, C5G, A6U, A6C, A6G, G7A, G7U, G7C, G8A, G8U, G8C, U9A, U9C, U9G, G10A, G10U, G10C, G14A, G14U, G14C, U15A, U15C, U15G, A16U, A16C, A16G, A17U, A17C, A17G, C18A, C18U, C18G, G19A, G19U, G19C, G20A, G20U, G20C, A21U, A21C, A21G, C22A, C22U, C22G, U23A, U23C, U23G, U24A, U24C, U24G, A25U, A25C, A25G, G26A, G26U, G26C, A27U, A27C, A27G, U28A, U28C, U28G, C29A, C29U, C29G, C30A, C30U, C30G, A31U, A31C, A31G, C32A, C32U, C32G, U33A, U33C, U33G, G34A, G34U, G34C, U35A, U35C, U35G, A36U, A36C, A36G, C37A, C37U, C37G, G38A, G38U, G38C, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U35G (which are the aptamer molecule structures in Table 1). These mutants can specifically bind to fluorophore molecules, and can significantly increase fluorescence intensity of fluorophore molecules under excitation light of appropriate wavelength after binding. The nucleotide position sequence corresponds to the position shown in FIG. 1A.

The afore-mentioned mutants indicate that nucleotide substitution occurs at corresponding sites of the aptamer nucleotide sequence of the General Formula N16 structure. For example, C2A indicates that the cytosine nucleotide C at the second position of N16 is substituted by adenine nucleotide A, i.e. N16-C2A in Table 1; N16-C4G/U9A indicates that the cytosine nucleotide C at the fourth position of N16 is substituted by guanine nucleotide G, and the uracil nucleotide U at the ninth position of N16 is substituted by adenine nucleotide A, i.e. N16-C4G/U9A in Table 1.

Table 1: Aptamer structure of N16 general formula structure after substitution of 5, 4, 3, 2 or 1 nucleotide

| Substitutions of N16 general formula structure | Aptamer structure general formula after substitution (underlined are bases after substitution) |
|---|---|
| N16 (C2A) | $N_1$<u>A</u>UCCAGGUG$N_{11}$-$N_{12}$-$N_{13}$GUAACGGACUUA GAUCCACUGUACG$N_{39}$ |
| N16 (C2U) | $N_1$<u>U</u>UCCAGGUG$N_{11}$-$N_{12}$-$N_{13}$GUAACGGACUUA GAUCCACUGUACG$N_{39}$ |
| N16 (C2G) | $N_1$<u>G</u>UCCAGGUG$N_{11}$-$N_{12}$-$N_{13}$GUAACGGACUUA GAUCCACUGUACG$N_{39}$ |
| N16 (U3A) | $N_1$C<u>A</u>CCAGGUG$N_{11}$-$N_{12}$-$N_{13}$GUAACGGACUUA GAUCCACUGUACG$N_{39}$ |

(continued)

| Substitutions of N16 general formula structure | Aptamer structure general formula after substitution (underlined are bases after substitution) |
|---|---|
| N16 (U3C) | $N_1$CCCCAGGUG$N_{11}$-$N_{12}$-$N_{13}$GUAACGGACUUAGAUCCACUGUACG$N_{39}$ |
| N16 (U3G) | $N_1$CGCCAGGUG$N_{11}$-$N_{12}$-$N_{13}$GUAACGGACUUAGAUCCACUGUACG$N_{39}$ |
| N16 (C4A) | $N_1$CUACAGGUG$N_{11}$-$N_{12}$-$N_{13}$GUAACGGACUUAGAUCCACUGUACG$N_{39}$ |
| N16 (C4U) | $N_1$CUUCAGGUG$N_{11}$-$N_{12}$-$N_{13}$GUAACGGACUUAGAUCCACUGUACG$N_{39}$ |
| N16 (C4G) | $N_1$CUGCAGGUG$N_{11}$-$N_{12}$-$N_{13}$GUAACGGACUUAGAUCCACUGUACG$N_{39}$ |
| N16 (C5A) | $N_1$CUCAAGGUG$N_{11}$-$N_{12}$-$N_{13}$GUAACGGACUUAGAUCCACUGUACG$N_{39}$ |
| N16 (C5U) | $N_1$CUCUAGGUG$N_{11}$-$N_{12}$-$N_{13}$GUAACGGACUUAGAUCCACUGUACG$N_{39}$ |
| N16 (C5G) | $N_1$CUCGAGGUG$N_{11}$-$N_{12}$-$N_{13}$GUAACGGACUUAGAUCCACUGUACG$N_{39}$ |
| N16 (A6U) | $N_1$CUCCUGGUG$N_{11}$-$N_{12}$-$N_{13}$GUAACGGACUUAGAUCCACUGUACG$N_{39}$ |
| N16 (A6C) | $N_1$CUCCCGGUG$N_{11}$-$N_{12}$-$N_{13}$GUAACGGACUUAGAUCCACUGUACG$N_{39}$ |
| N16 (A6G) | $N_1$CUCCGGGUG$N_{11}$-$N_{12}$-$N_{13}$GUAACGGACUUAGAUCCACUGUACG$N_{39}$ |
| N16 (G7A) | $N_1$CUCCAAGUG$N_{11}$-$N_{12}$-$N_{13}$GUAACGGACUUAGAUCCACUGUACG$N_{39}$ |
| N16(G7U) | $N_1$CUCCAUGUG$N_{11}$-$N_{12}$-$N_{13}$GUAACGGACUUAGAUCCACUGUACG$N_{39}$ |
| N16(G7C) | $N_1$CUCCACGUG$N_{11}$-$N_{12}$-$N_{13}$GUAACGGACUUAGAUCCACUGUACG$N_{39}$ |
| N16 (G8A) | $N_1$CUCCAGAUG$N_{11}$-$N_{12}$-$N_{13}$GUAACGGACUUAGAUCCACUGUACG$N_{39}$ |
| N16 (G8U) | $N_1$CUCCAGUUG$N_{11}$-$N_{12}$-$N_{13}$GUAACGGACUUAGAUCCACUGUACG$N_{39}$ |

(continued)

| Substitutions of N16 general formula structure | Aptamer structure general formula after substitution (underlined are bases after substitution) |
|---|---|
| N16 (G8C) | $N_1$CUCCAGCUG$N_{11}$-$N_{12}$-$N_{13}$GUAACGGACUUA GAUCCACUGUACG$N_{39}$ |
| N16 (U9A) | $N_1$CUCCAGGAG$N_{11}$-$N_{12}$-$N_{13}$GUAACGGACUUA GAUCCACUGUACG$N_{39}$ |
| N16 (U9C) | $N_1$CUCCAGGCG$N_{11}$-$N_{12}$-$N_{13}$GUAACGGACUUA GAUCCACUGUACG$N_{39}$ |
| N16 (U9G) | $N_1$CUCCAGGGG$N_{11}$-$N_{12}$-$N_{13}$GUAACGGACUUA GAUCCACUGUACG$N_{39}$ |
| N16 (G10A) | $N_1$CUCCAGGUA$N_{11}$-$N_{12}$-$N_{13}$GUAACGGACUUA GAUCCACUGUACG$N_{39}$ |
| N16 (G10U) | $N_1$CUCCAGGUU$N_{11}$-$N_{12}$-$N_{13}$GUAACGGACUUA GAUCCACUGUACG$N_{39}$ |
| N16 (G10C) | $N_1$CUCCAGGUC$N_{11}$-$N_{12}$-$N_{13}$GUAACGGACUUA GAUCCACUGUACG$N_{39}$ |
| N16 (G14A) | $N_1$CUCCAGGUG$N_{11}$-$N_{12}$-$N_{13}$AUAACGGACUUA GAUCCACUGUACG$N_{39}$ |
| N16 (G14U) | $N_1$CUCCAGGUG$N_{11}$-$N_{12}$-$N_{13}$UUAACGGACUUA GAUCCACUGUACG$N_{39}$ |
| N16 (G14C) | $N_1$CUCCAGGUG$N_{11}$-$N_{12}$-$N_{13}$CUAACGGACUUA GAUCCACUGUACG$N_{39}$ |
| N16(U15A) | $N_1$CUCCAGGUG$N_{11}$-$N_{12}$-$N_{13}$GAAACGGACUUA GAUCCACUGUACG$N_{39}$ |
| N16(U15C) | $N_1$CUCCAGGUG$N_{11}$-$N_{12}$-$N_{13}$GCAACGGACUUA GAUCCACUGUACG$N_{39}$ |
| N16(U15G) | $N_1$CUCCAGGUG$N_{11}$-$N_{12}$-$N_{13}$GGAACGGACUUA GAUCCACUGUACG$N_{39}$ |
| N16 (A16U) | $N_1$CUCCAGGUG$N_{11}$-$N_{12}$-$N_{13}$GUUACGGACUUA GAUCCACUGUACG$N_{39}$ |
| N16 (A16C) | $N_1$CUCCAGGUG$N_{11}$-$N_{12}$-$N_{13}$GUCACGGACUUA GAUCCACUGUACG$N_{39}$ |

(continued)

| Substitutions of N16 general formula structure | Aptamer structure general formula after substitution (underlined are bases after substitution) |
|---|---|
| N16 (A16G) | $N_1$CUCCAGGUG$N_{11}$-$N_{12}$-$N_{13}$GU$\underline{G}$ACGGACUUA GAUCCACUGUACG$N_{39}$ |
| N16 (A17U) | $N_1$CUCCAGGUG$N_{11}$-$N_{12}$-$N_{13}$GUA$\underline{U}$CGGACUUA GAUCCACUGUACG$N_{39}$ |
| N16 (A17C) | $N_1$CUCCAGGUG$N_{11}$-$N_{12}$-$N_{13}$GUA$\underline{C}$CGGACUUA GAUCCACUGUACG$N_{39}$ |
| N16 (A17G) | $N_1$CUCCAGGUG$N_{11}$-$N_{12}$-$N_{13}$GUA$\underline{G}$CGGACUUA GAUCCACUGUACG$N_{39}$ |
| N16 (C18A) | $N_1$CUCCAGGUG$N_{11}$-$N_{12}$-$N_{13}$GUAA$\underline{A}$GGACUUA GAUCCACUGUACG$N_{39}$ |
| N16 (C18U) | $N_1$CUCCAGGUG$N_{11}$-$N_{12}$-$N_{13}$GUAA$\underline{U}$GGACUUA GAUCCACUGUACG$N_{39}$ |
| N16 (C18G) | $N_1$CUCCAGGUG$N_{11}$-$N_{12}$-$N_{13}$GUAA$\underline{G}$GGACUUA GAUCCACUGUACG$N_{39}$ |
| N16 (G19A) | $N_1$CUCCAGGUG$N_{11}$-$N_{12}$-$N_{13}$GUAAC$\underline{A}$GACUUA GAUCCACUGUACG$N_{39}$ |
| N16 (G19U) | $N_1$CUCCAGGUG$N_{11}$-$N_{12}$-$N_{13}$GUAAC$\underline{U}$GACUUA GAUCCACUGUACG$N_{39}$ |
| N16 (G19C) | $N_1$CUCCAGGUG$N_{11}$-$N_{12}$-$N_{13}$GUAAC$\underline{C}$GACUUA GAUCCACUGUACG$N_{39}$ |
| N16(G20A) | $N_1$CUCCAGGUG$N_{11}$-$N_{12}$-$N_{13}$GUAACG$\underline{A}$ACUUA GAUCCACUGUACG$N_{39}$ |
| N16(G20U) | $N_1$CUCCAGGUG$N_{11}$-$N_{12}$-$N_{13}$GUAACG$\underline{U}$ACUUA GAUCCACUGUACG$N_{39}$ |
| N16(G20C) | $N_1$CUCCAGGUG$N_{11}$-$N_{12}$-$N_{13}$GUAACG$\underline{C}$ACUUA GAUCCACUGUACG$N_{39}$ |
| N16 (A21U) | $N_1$CUCCAGGUG$N_{11}$-$N_{12}$-$N_{13}$GUAACGG$\underline{U}$CUUA GAUCCACUGUACG$N_{39}$ |
| N16 (A21C) | $N_1$CUCCAGGUG$N_{11}$-$N_{12}$-$N_{13}$GUAACGG$\underline{C}$CUUA GAUCCACUGUACG$N_{39}$ |

(continued)

| Substitutions of N16 general formula structure | Aptamer structure general formula after substitution (underlined are bases after substitution) |
|---|---|
| N16 (A21G) | $N_1$CUCCAGGUG$N_{11}$-$N_{12}$-$N_{13}$GUAACGG$\underline{G}$CUUAGAUCCACUGUACG$N_{39}$ |
| N16 (C22A) | $N_1$CUCCAGGUG$N_{11}$-$N_{12}$-$N_{13}$GUAACGGA$\underline{A}$UUAGAUCCACUGUACG$N_{39}$ |
| N16 (C22U) | $N_1$CUCCAGGUG$N_{11}$-$N_{12}$-$N_{13}$GUAACGGA$\underline{U}$UUAGAUCCACUGUACG$N_{39}$ |
| N16 (C22G) | $N_1$CUCCAGGUG$N_{11}$-$N_{12}$-$N_{13}$GUAACGGA$\underline{G}$UUAGAUCCACUGUACG$N_{39}$ |
| N16 (U23A) | $N_1$CUCCAGGUG$N_{11}$-$N_{12}$-$N_{13}$GUAACGGAC$\underline{A}$UAGAUCCACUGUACG$N_{39}$ |
| N16 (U23C) | $N_1$CUCCAGGUG$N_{11}$-$N_{12}$-$N_{13}$GUAACGGAC$\underline{C}$UAGAUCCACUGUACG$N_{39}$ |
| N16 (U23G) | $N_1$CUCCAGGUG$N_{11}$-$N_{12}$-$N_{13}$GUAACGGAC$\underline{G}$UAGAUCCACUGUACG$N_{39}$ |
| N16 (U24A) | $N_1$CUCCAGGUG$N_{11}$-$N_{12}$-$N_{13}$GUAACGGACU$\underline{A}$AGAUCCACUGUACG$N_{39}$ |
| N16 (U24C) | $N_1$CUCCAGGUG$N_{11}$-$N_{12}$-$N_{13}$GUAACGGACU$\underline{C}$AGAUCCACUGUACG$N_{39}$ |
| N16 (U24G) | $N_1$CUCCAGGUG$N_{11}$-$N_{12}$-$N_{13}$GUAACGGACU$\underline{G}$AGAUCCACUGUACG$N_{39}$ |
| N16(A25U) | $N_1$CUCCAGGUG$N_{11}$-$N_{12}$-$N_{13}$GUAACGGACUU$\underline{U}$GAUCCACUGUACG$N_{39}$ |
| N16(A25C) | $N_1$CUCCAGGUG$N_{11}$-$N_{12}$-$N_{13}$GUAACGGACUU$\underline{C}$GAUCCACUGUACG$N_{39}$ |
| N16(A25G) | $N_1$CUCCAGGUG$N_{11}$-$N_{12}$-$N_{13}$GUAACGGACUU$\underline{G}$GAUCCACUGUACG$N_{39}$ |
| N16 (G26A) | $N_1$CUCCAGGUG$N_{11}$-$N_{12}$-$N_{13}$GUAACGGACUUA$\underline{A}$AUCCACUGUACG$N_{39}$ |
| N16 (G26U) | $N_1$CUCCAGGUG$N_{11}$-$N_{12}$-$N_{13}$GUAACGGACUUA$\underline{U}$AUCCACUGUACG$N_{39}$ |

(continued)

| Substitutions of N16 general formula structure | Aptamer structure general formula after substitution (underlined are bases after substitution) |
|---|---|
| N16 (G26C) | $N_1$CUCCAGGUG$N_{11}$-$N_{12}$-$N_{13}$GUAACGGACUUACAUCCACUGUACG$N_{39}$ |
| N16 (A27U) | $N_1$CUCCAGGUG$N_{11}$-$N_{12}$-$N_{13}$GUAACGGACUUAGUUCCACUGUACG$N_{39}$ |
| N16 (A27C) | $N_1$CUCCAGGUG$N_{11}$-$N_{12}$-$N_{13}$GUAACGGACUUAGCUCCACUGUACG$N_{39}$ |
| N16 (A27G) | $N_1$CUCCAGGUG$N_{11}$-$N_{12}$-$N_{13}$GUAACGGACUUAGGUCCACUGUACG$N_{39}$ |
| N16 (U28A) | $N_1$CUCCAGGUG$N_{11}$-$N_{12}$-$N_{13}$GUAACGGACUUAGAACCACUGUACG$N_{39}$ |
| N16 (U28C) | $N_1$CUCCAGGUG$N_{11}$-$N_{12}$-$N_{13}$GUAACGGACUUAGACCCACUGUACG$N_{39}$ |
| N16 (U28G) | $N_1$CUCCAGGUG$N_{11}$-$N_{12}$-$N_{13}$GUAACGGACUUAGAGCCACUGUACG$N_{39}$ |
| N16 (C29A) | $N_1$CUCCAGGUG$N_{11}$-$N_{12}$-$N_{13}$GUAACGGACUUAGAUACACUGUACG$N_{39}$ |
| N16 (C29U) | $N_1$CUCCAGGUG$N_{11}$-$N_{12}$-$N_{13}$GUAACGGACUUAGAUUCACUGUACG$N_{39}$ |
| N16 (C29G) | $N_1$CUCCAGGUG$N_{11}$-$N_{12}$-$N_{13}$GUAACGGACUUAGAUGCACUGUACG$N_{39}$ |
| N16(C30A) | $N_1$CUCCAGGUG$N_{11}$-$N_{12}$-$N_{13}$GUAACGGACUUAGAUCAACUGUACG$N_{39}$ |
| N16 (C30U) | $N_1$CUCCAGGUG$N_{11}$-$N_{12}$-$N_{13}$GUAACGGACUUAGAUCUACUGUACG$N_{39}$ |
| N16(C30G) | $N_1$CUCCAGGUG$N_{11}$-$N_{12}$-$N_{13}$GUAACGGACUUAGAUCGACUGUACG$N_{39}$ |
| N16(A31U) | $N_1$CUCCAGGUG$N_{11}$-$N_{12}$-$N_{13}$GUAACGGACUUAGAUCCUCUGUACG$N_{39}$ |
| N16 (A31C) | $N_1$CUCCAGGUG$N_{11}$-$N_{12}$-$N_{13}$GUAACGGACUUAGAUCCCCUGUACG$N_{39}$ |

(continued)

| Substitutions of N16 general formula structure | Aptamer structure general formula after substitution (underlined are bases after substitution) |
|---|---|
| N16 (A31G) | $N_1$CUCCAGGUG$N_{11}$-$N_{12}$-$N_{13}$GUAACGGACUUA GAUCCGCUGUACG$N_{39}$ |
| N16 (C32A) | $N_1$CUCCAGGUG$N_{11}$-$N_{12}$-$N_{13}$GUAACGGACUUA GAUCCAAUGUACG$N_{39}$ |
| N16 (C32U) | $N_1$CUCCAGGUG$N_{11}$-$N_{12}$-$N_{13}$GUAACGGACUUA GAUCCAUUGUACG$N_{39}$ |
| N16 (C32G) | $N_1$CUCCAGGUG$N_{11}$-$N_{12}$-$N_{13}$GUAACGGACUUA GAUCCAGUGUACG$N_{39}$ |
| N16 (U33A) | $N_1$CUCCAGGUG$N_{11}$-$N_{12}$-$N_{13}$GUAACGGACUUA GAUCCACAGUACG$N_{39}$ |
| N16 (U33C) | $N_1$CUCCAGGUG$N_{11}$-$N_{12}$-$N_{13}$GUAACGGACUUA GAUCCACCGUACG$N_{39}$ |
| N16 (U33G) | $N_1$CUCCAGGUG$N_{11}$-$N_{12}$-$N_{13}$GUAACGGACUUA GAUCCACGGUACG$N_{39}$ |
| N16 (G34A) | $N_1$CUCCAGGUG$N_{11}$-$N_{12}$-$N_{13}$GUAACGGACUUA GAUCCACUAUACG$N_{39}$ |
| N16 (G34U) | $N_1$CUCCAGGUG$N_{11}$-$N_{12}$-$N_{13}$GUAACGGACUUA GAUCCACUUUACG$N_{39}$ |
| N16 (G34C) | $N_1$CUCCAGGUG$N_{11}$-$N_{12}$-$N_{13}$GUAACGGACUUA GAUCCACUCUACG$N_{39}$ |
| N16 (U35A) | $N_1$CUCCAGGUG$N_{11}$-$N_{12}$-$N_{13}$GUAACGGACUUA GAUCCACUGAACG$N_{39}$ |
| N16 (U35C) | $N_1$CUCCAGGUG$N_{11}$-$N_{12}$-$N_{13}$GUAACGGACUUA GAUCCACUGCACG$N_{39}$ |
| N16 (U35G) | $N_1$CUCCAGGUG$N_{11}$-$N_{12}$-$N_{13}$GUAACGGACUUA GAUCCACUGGACG$N_{39}$ |
| N16(A36U) | $N_1$CUCCAGGUG$N_{11}$-$N_{12}$-$N_{13}$GUAACGGACUUA GAUCCACUGUUCG$N_{39}$ |
| N16(A36C) | $N_1$CUCCAGGUG$N_{11}$-$N_{12}$-$N_{13}$GUAACGGACUUA GAUCCACUGUCCG$N_{39}$ |

(continued)

| Substitutions of N16 general formula structure | Aptamer structure general formula after substitution (underlined are bases after substitution) |
|---|---|
| N16(A36G) | $N_1$CUCCAGGUG$N_{11}$-$N_{12}$-$N_{13}$GUAACGGACUUA<br>GAUCCACUGU$\underline{G}$CG$N_{39}$ |
| N16(C37A) | $N_1$CUCCAGGUG$N_{11}$-$N_{12}$-$N_{13}$GUAACGGACUUA<br>GAUCCACUGUA$\underline{A}$G$N_{39}$ |
| N16(C37U) | $N_1$CUCCAGGUG$N_{11}$-$N_{12}$-$N_{13}$GUAACGGACUUA<br>GAUCCACUGUA$\underline{U}$G$N_{39}$ |
| N16(C37G) | $N_1$CUCCAGGUG$N_{11}$-$N_{12}$-$N_{13}$GUAACGGACUUA<br>GAUCCACUGUA$\underline{G}$G$N_{39}$ |
| N16(G38A) | $N_1$CUCCAGGUG$N_{11}$-$N_{12}$-$N_{13}$GUAACGGACUUA<br>GAUCCACUGUAC$\underline{A}$$N_{39}$ |
| N16(G38U) | $N_1$CUCCAGGUG$N_{11}$-$N_{12}$-$N_{13}$GUAACGGACUUA<br>GAUCCACUGUAC$\underline{U}$$N_{39}$ |
| N16(G38C) | $N_1$CUCCAGGUG$N_{11}$-$N_{12}$-$N_{13}$GUAACGGACUUA<br>GAUCCACUGUAC$\underline{C}$$N_{39}$ |
| N16 (C4G/U9A) | $N_1$CU$\underline{G}$CAGG$\underline{A}$G$N_{11}$-$N_{12}$-$N_{13}$GUAACGGACUUA<br>GAUCCACUGUACG$N_{39}$ |
| N16 (C4G/A16G) | $N_1$CU$\underline{G}$CAGGUG$N_{11}$-$N_{12}$-$N_{13}$GU$\underline{G}$ACGGACUUA<br>GAUCCACUGUACG$N_{39}$ |
| N16 (C4G/A17G) | $N_1$CU$\underline{G}$CAGGUG$N_{11}$-$N_{12}$-$N_{13}$GUA$\underline{G}$CGGACUUA<br>GAUCCACUGUACG$N_{39}$ |
| N16 (C4G/C22U) | $N_1$CU$\underline{G}$CAGGUG$N_{11}$-$N_{12}$-$N_{13}$GUAACGGA$\underline{U}$UUA<br>GAUCCACUGUACG$N_{39}$ |
| N16 (C4G/U24G) | $N_1$CU$\underline{G}$CAGGUG$N_{11}$-$N_{12}$-$N_{13}$GUAACGGACU$\underline{G}$A<br>GAUCCACUGUACG$N_{39}$ |
| N16 (C4G/U35G) | $N_1$CU$\underline{G}$CAGGUG$N_{11}$-$N_{12}$-$N_{13}$GUAACGGACUUA<br>GAUCCACUG$\underline{G}$ACG$N_{39}$ |
| N16 (U9A/A16G) | $N_1$CUCCAGG$\underline{A}$G$N_{11}$-$N_{12}$-$N_{13}$GU$\underline{G}$ACGGACUUA<br>GAUCCACUGUACG$N_{39}$ |
| N16 (U9A/A17G) | $N_1$CUCCAGG$\underline{A}$G$N_{11}$-$N_{12}$-$N_{13}$GUA$\underline{G}$CGGACUUA<br>GAUCCACUGUACG$N_{39}$ |

(continued)

| Substitutions of N16 general formula structure | Aptamer structure general formula after substitution (underlined are bases after substitution) |
|---|---|
| N16 (U9A/C22U) | $N_1$CUCCAGG$\underline{A}$G$N_{11}$-$N_{12}$-$N_{13}$GUAACGGA$\underline{U}$UUA GAUCCACUGUACG$N_{39}$ |
| N16 (U9A/U24G) | $N_1$CUCCAGG$\underline{A}$G$N_{11}$-$N_{12}$-$N_{13}$GUAACGGACU$\underline{G}$A GAUCCACUGUACG$N_{39}$ |
| N16 (U9A/U35G) | $N_1$CUCCAGG$\underline{A}$G$N_{11}$-$N_{12}$-$N_{13}$GUAACGGACUUA GAUCCACUG$\underline{G}$ACG$N_{39}$ |
| N16 (A16G/A17G) | $N_1$CUCCAGGUG$N_{11}$-$N_{12}$-$N_{13}$GU$\underline{GG}$CGGACUUA GAUCCACUGUACG$N_{39}$ |
| N16 (A16G/C22U) | $N_1$CUCCAGGUG$N_{11}$-$N_{12}$-$N_{13}$GU$\underline{G}$ACGGA$\underline{U}$UUA GAUCCACUGUACG$N_{39}$ |
| N16 (A16G/U24G) | $N_1$CUCCAGGUG$N_{11}$-$N_{12}$-$N_{13}$GU$\underline{G}$ACGGACU$\underline{G}$A GAUCCACUGUACG$N_{39}$ |
| N16 (A16G/U35G) | $N_1$CUCCAGGUG$N_{11}$-$N_{12}$-$N_{13}$GU$\underline{G}$ACGGACUUA GAUCCACUG$\underline{G}$ACG$N_{39}$ |
| N16 (A17G/C22U) | $N_1$CUCCAGGUG$N_{11}$-$N_{12}$-$N_{13}$GUA$\underline{G}$CGGA$\underline{U}$UUA GAUCCACUGUACG$N_{39}$ |
| N16 (A17G/U24G) | $N_1$CUCCAGGUG$N_{11}$-$N_{12}$-$N_{13}$GUA$\underline{G}$CGGACU$\underline{G}$A GAUCCACUGUACG$N_{39}$ |
| N16 (A17G/ U35G) | $N_1$CUCCAGGUG$N_{11}$-$N_{12}$-$N_{13}$GUA$\underline{G}$CGGACUUA GAUCCACUG$\underline{G}$ACG$N_{39}$ |
| N16 (C22U/U24G) | $N_1$CUCCAGGUG$N_{11}$-$N_{12}$-$N_{13}$GUAACGGA$\underline{U}$U$\underline{G}$A GAUCCACUGUACG$N_{39}$ |
| N16 (C22U/U35G) | $N_1$CUCCAGGUG$N_{11}$-$N_{12}$-$N_{13}$GUAACGGA$\underline{U}$UUA GAUCCACUGGACG$N_{39}$ |
| N16 (U24G/U35G) | $N_1$CUCCAGGUG$N_{11}$-$N_{12}$-$N_{13}$GUAACGGACU$\underline{G}$A GAUCCACUG$\underline{G}$ACG$N_{39}$ |
| N16 (C4G/U9A/A16G) | $N_1$CU$\underline{G}$CAGG$\underline{A}$G$N_{11}$-$N_{12}$-$N_{13}$GU$\underline{G}$ACGGACUUA GAUCCACUGUACG$N_{39}$ |
| N16 (C4G/U9A/A17G) | $N_1$CU$\underline{G}$CAGG$\underline{A}$G$N_{11}$-$N_{12}$-$N_{13}$GUA$\underline{G}$CGGACUUA GAUCCACUGUACG$N_{39}$ |

(continued)

| Substitutions of N16 general formula structure | Aptamer structure general formula after substitution (underlined are bases after substitution) |
|---|---|
| N16 (C4G/U9A/C22U) | $N_1CU\underline{G}CAGG\underline{A}GN_{11}-N_{12}-N_{13}GUAACGGA\underline{U}UUA$ $GAUCCACUGUACGN_{39}$ |
| N16 (C4G/U9A/U24G) | $N_1CU\underline{G}CAGG\underline{A}GN_{11}-N_{12}-N_{13}GUAACGGACU\underline{G}A$ $GAUCCACUGUACGN_{39}$ |
| N16 (C4G/U9A/U35G) | $N_1CU\underline{G}CAGG\underline{A}GN_{11}-N_{12}-N_{13}GUAACGGACUUA$ $GAUCCACUG\underline{G}ACGN_{39}$ |
| N16 (C4G/A16G/A17G) | $N_1CU\underline{G}CAGGUGN_{11}-N_{12}-N_{13}GU\underline{GG}CGGACUUA$ $GAUCCACUGUACGN_{39}$ |
| N16 (C4G/A16G/C22U) | $N_1CU\underline{G}CAGGUGN_{11}-N_{12}-N_{13}GU\underline{G}ACGGA\underline{U}UUA$ $GAUCCACUGUACGN_{39}$ |
| N16 (C4G/A16G/U24G) | $N_1CU\underline{G}CAGGUGN_{11}-N_{12}-N_{13}GU\underline{G}ACGGACU\underline{G}A$ $GAUCCACUGUACGN_{39}$ |
| N16 (C4G/A16G/U35G) | $N_1CU\underline{G}CAGGUGN_{11}-N_{12}-N_{13}GU\underline{G}ACGGACUUA$ $GAUCCACUGGACGN_{39}$ |
| N16 (C4G/A17G/C22U) | $N_1CU\underline{G}CAGGUGN_{11}-N_{12}-N_{13}GUA\underline{G}CGGA\underline{U}UUA$ $GAUCCACUGUACGN_{39}$ |
| N16 (C4G/A17G/U24G) | $N_1CU\underline{G}CAGGUGN_{11}-N_{12}-N_{13}GUA\underline{G}CGGACU\underline{G}A$ $GAUCCACUGUACGN_{39}$ |
| N16 (C4G/A17G/U35G) | $N_1CU\underline{G}CAGGUGN_{11}-N_{12}-N_{13}GUA\underline{G}CGGACUUA$ $GAUCCACUG\underline{G}ACGN_{39}$ |
| N16 (C4G/C22U/U24G) | $N_1CU\underline{G}CAGGUGN_{11}-N_{12}-N_{13}GUAACGGA\underline{U}U\underline{G}A$ $GAUCCACUGUACGN_{39}$ |
| N16 (C4G/C22U/U35G) | $N_1CU\underline{G}CAGGUGN_{11}-N_{12}-N_{13}GUAACGGA\underline{U}UUA$ $GAUCCACUG\underline{G}ACGN_{39}$ |
| N16 (C4G/U24G/U35G) | $N_1CU\underline{G}CAGGUGN_{11}-N_{12}-N_{13}GUAACGGACU\underline{G}A$ $GAUCCACUG\underline{G}ACGN_{39}$ |
| N16 (C4G/C22U/U24G/U9A) | $N_1CU\underline{G}CAGG\underline{A}GN_{11}-N_{12}-N_{13}GUAACGGA\underline{U}U\underline{G}A$ $GAUCCACUGUACGN_{39}$ |
| N16 (C4G/C22U/U24G/A16G) | $N_1CU\underline{G}CAGGUGN_{11}-N_{12}-N_{13}GU\underline{G}ACGGA\underline{U}U\underline{G}A$ $GAUCCACUGUACGN_{39}$ |

(continued)

| Substitutions of N16 general formula structure | Aptamer structure general formula after substitution (underlined are bases after substitution) |
|---|---|
| N16 (C4G/C22U/U24G/A17G) | $\mathrm{N_{1}CU\underline{G}CAGGUGN_{11}\text{-}N_{12}\text{-}N_{13}GUA\underline{G}CGGA\underline{U}U\underline{G}A}$<br>$\mathrm{GAUCCACUGUACGN_{39}}$ |
| N16 (C4G/C22U/U24G/U35G) | $\mathrm{N_{1}CU\underline{G}CAGGUGN_{11}\text{-}N_{12}\text{-}N_{13}GUAACGGA\underline{U}U\underline{G}A}$<br>$\mathrm{GAUCCACUG\underline{G}ACGN_{39}}$ |
| N16 (C4G/C22U/U24G/U9A/A16G) | $\mathrm{N_{1}CU\underline{G}CAGG\underline{A}GN_{11}\text{-}N_{12}\text{-}N_{13}GU\underline{G}ACGGA\underline{U}U\underline{G}A}$<br>$\mathrm{GAUCCACUGUACGN_{39}}$ |
| N16 (C4G/C22U/U24G/U9A/A17G) | $\mathrm{N_{1}CU\underline{G}CAGG\underline{A}GN_{11}\text{-}N_{12}\text{-}N_{13}GUA\underline{G}CGGA\underline{U}U\underline{G}A}$<br>$\mathrm{GAUCCACUGUACGN_{39}}$ |
| N16 (C4G/C22U/U24G/U9A/U35G) | $\mathrm{N_{1}CU\underline{G}CAGG\underline{A}GN_{11}\text{-}N_{12}\text{-}N_{13}GUAACGGA\underline{U}U\underline{G}A}$<br>$\mathrm{GAUCCACUG\underline{G}ACGN_{39}}$ |
| N16 (C4G/C22U/U24G/A16G/A17G) | $\mathrm{N_{1}CU\underline{G}CAGGUGN_{11}\text{-}N_{12}\text{-}N_{13}GU\underline{GG}CGGA\underline{U}U\underline{G}A}$<br>$\mathrm{GAUCCACUGUACGN_{39}}$ |
| N16 (C4G/C22U/U24G/A16G/U3 5G) | $\mathrm{N_{1}CU\underline{G}CAGGUGN_{11}\text{-}N_{12}\text{-}N_{13}GU\underline{G}ACGGA\underline{U}U\underline{G}A}$<br>$\mathrm{GAUCCACUG\underline{G}ACGN_{39}}$ |
| N16 (C4G/C22U/U24G/A17G/U35G) | $\mathrm{N_{1}CU\underline{G}CAGGUGN_{11}\text{-}N_{12}\text{-}N_{13}GUA\underline{G}CGGA\underline{U}U\underline{G}A}$<br>$\mathrm{GAUCCACUG\underline{G}ACGN_{39}}$ |

Aptamer molecules are single-stranded nucleic acid molecules that have a secondary structure (FIG. 1A) consisting of one or more base pairing regions (stem) and one or more unpaired regions (loop). The nucleic acid molecules of the present disclosure contain a secondary structure as predicted in FIG. 1A. The secondary structure comprises three stem structures, three loop structures and a stem-loop structure, wherein Stem 1 plays the role of stabilizing the entire nucleic acid aptamer molecule structure, and can be substituted by other nucleotide base pairs of any length and any composition that can form stem structures. The 5' end or 3' end of Stem 1 structure can be fused with any objective RNA molecule for extracellular or intracellular detection of objective RNA molecules. In a preferable embodiment of the present disclosure, the 5' end of the nucleic acid aptamer molecule is fused with a 5S RNA sequence (Genebank: NR_023377.1); in another preferable embodiment of the present disclosure, the 5' end of the nucleic acid aptamer molecule is fused with a ACTB RNA sequence (ACCESSION NM_001101).

The stem-loop structure in FIG. 1A plays the role of stabilizing the entire nucleic acid aptamer molecule structure, and can be replaced with other nucleotide base pairs of any length and any composition that can form stem-loop structures. The aptamer molecules of the present application may also contain other nucleotide sequences that can be inserted into the position of $N_{11}$-$N_{12}$-$N_{13}$, wherein the inserted nucleotide sequence replaces the stem-loop structure in FIG. 1A, and thus the structure (SEQ ID NO: 1) in FIG. 1B is obtained. The nucleotide sequence can specifically identify/bind to target molecules. In the absence of target molecules, the binding of aptamer molecules and fluorophore molecules is weak, as a result of which the fluorophore molecules show weak fluorescence light; in the presence of target molecules, the binding of target molecules and the aptamer will promote binding of the aptamer and fluorophore molecules, and thus can significantly enhance the fluorescence of fluorophore molecules under excitation light of appropriate wavelength. The target molecules can be small molecules, and signal molecules on the cell surface, etc. These nucleic acid aptamers bind with specific target molecules through non-covalent binding, which mainly depends on intermolecular ionic forces, dipole force, hydrogen bonds, Van der Waals forces, positron and negative electron interactions, stacking or the combination of the above forces. The stem-loop structure can be replaced with an RNA sequence that identifies the target molecules for extracellular or intracellular detection of the target molecules.

In a preferable embodiment of the present disclosure, the nucleic acid aptamer molecules are preferably SEQ

ID NO: 1, 2, 3, 4, 5, 6, 7, 8, 9, 10, 11, 12, 13 or 14, and can bind to fluorophore molecules so as to significantly enhance fluorescence of the mutation sequence under excitation light of appropriate wavelength.

The nucleic acid aptamer molecules of the present disclosure can also comprise a fragment of nucleotide sequence that increases its stability. In a preferable embodiment of the present disclosure, F30 scaffold RNA (SEQ ID NO: 2) was adopted, and its connection mode with the nucleic acid aptamer molecules is shown in FIG. 2A; in another preferable embodiment of the present invention, tRNA scaffold RNA (SEQ ID NO: 3) was adopted, and its connection mode with the nucleic acid aptamer molecules is shown in FIG. 2B.

The "nucleic acid aptamer molecules" in the present disclosure are RNA molecules, or DNA-RNA hybrid molecules with part of nucleotides being replaced with deoxyribonucleotides, wherein the nucleotides can be in a form of D and L enantiomers thereof and also contain derivatives thereof, including but not limited to 2'-F, 2'-amino, 2'-methoxyl, 5'-iodo, 5'-bromine-modified polynucleotides. Nucleic acids contain various modified nucleotides.

Identity

"Identity" describes the correlation between two nucleotide sequences in the present disclosure. The calculation of identity of two aptamer nucleotide sequences in the present disclosure does not include $N_1$, $N_{11}$, $N_{12}$, $N_{13}$, $N_{39}$ in Sequence (a). As for the present disclosure, identity of two aptamer nucleotide sequences is determined by using, for instance, Needle program, preferably Needleman-Wunsch Algprithm (Needleman and Wunsch, 1970, J.Mol.Biol.48: 443-453) executed in 3.0.0 version or higher, of EMBOSS software package (EMBOSS: The European Molecular Biology Open Software Suite, Rice etc., 2000, Trends in Genetics 16: 276-277). Optional parameters in use are gap penalty 10, gap extension penalty 0.5 and EBLOSUM62 substitution matrix (EMBOSS version of BLOSUM62). Output result labeled by Needle as "longest identity" (obtained by using the "-nobrief' option) serves as the percentage identity, and is calculated in a way as follows:

$$\text{(Identical residue}\times 100)/(\text{Alignment length-Total number of gaps in alignment}).$$

For instance, the sequences of N16 (C2A) and N16 (C2U) in Table 1 of the present disclosure are N16 (C2A): $N_1\underline{A}UCCAGGUGN_{11}$-$N_{12}$-$N_{13}GUAACGGACUUAGAUCCACUGUACGN_{39}$ and N16 (C2U): $N_1\underline{U}UCCAGGUGN_{11}$-$N_{12}$-$N_{13}GUAACGGACUUAGAUCCACUGUACGN_{39}$, and, according to the definition of the present disclosure, their identity alignment should not include nucleotide bases of $\underline{N_1}$, $\underline{N_{11}\text{-}N_{12}\text{-}N_{13}, N_{39}}$, so the alignment result of their sequence identity is 97% (the difference being one nucleotide).

Fluorophore molecules

The "fluorophore molecules" in the present disclosure are also called as "fluorophore" or "fluorescence molecules". "Fluorophore molecules" in the present disclosure are a kind of fluorophore molecules that can be conditionally activated, and show a relatively low quantum yield in the absence of nucleic acid aptamers. In specific embodiments, when a fluorophore is not bound to specific aptamers, its quantum yield is lower than 0.1, more preferably lower than 0.01, and optimally lower than 0.001; when the fluorophore is bound to specific aptamers, its quantum yield will be enhanced by more than two times, more preferably by more than 10 times, and optimally by more than 100 times. Fluorophore molecules are preferably water-soluble, non-toxic to cells and can easily penetrate membranes. Fluorophore of the present disclosure can preferably enter cytoplasm or pericytoplasm through membrane or cell wall by means of active transport or passive diffusion. In the embodiments of the present disclosure, the fluorophore can penetrate outer and inner membranes of Gram-negative bacteria, cell walls and membranes of plant cells, cell walls and membranes of fungi, membranes of animal cells, and GI and endothelial membranes of living animals.

The nucleic acid aptamer molecules of the present disclosure can specifically bind to a fluorophore, thereby significantly increase its fluorescence value under excitation of a specific wavelength. The fluorophore molecules are selected from the structure represent by Formula (I):

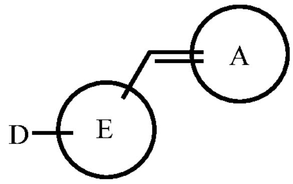

(I)

an electron donor part-D is -NX1-X2, X1 is selected from hydrogen, alkyl or modified alkyl, and X2 is selected from hydrogen, alkyl or modified alkyl; X1 and X2 are optionally interconnected, and form an aliphatic heterocycle with N atoms;

an electron donor part-D is -NX1-X2, X1 is selected from hydrogen, alkyl or modified alkyl, and X2 is selected from hydrogen, alkyl or modified alkyl; X1 and X2 are optionally interconnected, and form an aliphatic heterocycle with N atoms;

a conjugate system E is formed by at least one conjugate connection selected from double bond, triple bond, aromatic ring and aromatic heterocycle, wherein each hydrogen atom contained therein is optionally and independently substituted by a substituent group selected from halogen atom, hydroxyl group, amino group, primary amino group, secondary amino group, hydrophilic group, alkyl and modified alkyl, and the substituent group is optionally interconnected to form an aliphatic ring or an aliphatic heterocycle;

X1 and X2 are independently connected with the conjugate system E to form an aliphatic heterocycle;

an electron acceptor A optionally forms a ring structure represented by Formula (I-1-a) below:

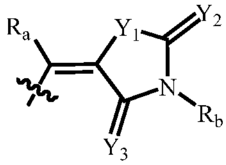

(I-1-a)

Ra is independently selected from hydrogen, halogen atom, nitro group, alkyl group, aryl group, heteroaryl group, hydrophilic group or modified alkyl; $R_b$ is independently selected from hydrogen, halogen atom, hydroxyl group, carboxyl group, amino group, nitro group, alkyl group, aryl group, heteroaryl group, hydrophilic group or modified alkyl, or is a group formed by conjugate connection of the double bond with at least one of the aromatic ring and aromatic heterocycle;

each $Y_1$ is independently selected from -O-, -S-, -(S=O)- and -($NR_i$)-, wherein $R_i$ is selected from hydrogen, amino group, alkyl or modified alkyl;

each $Y_2$ is independently selected from =O, =S, =S=O and =$NR_i$, wherein $R_i$ is selected from hydrogen, alkyl or modified alkyl;

each $Y_3$ is independently selected from =O, =S, =S=O and =$NR_i$, wherein $R_i$ is selected from hydrogen, alkyl or modified alkyl;

or, each $Y_3$ independently is =C ($R_e$)(CN); wherein $R_e$ is selected from hydrogen, ester group, amide group, sulfonic group, sulfamine, and sulfonate ester group;

wherein:

the "alkyl" is $C_1$-$C_{30}$ straight or branched alkyl; preferably, the "alkyl" is $C_1$-$C_{10}$ straight or branched alkyl; preferably, the "alkyl" is $C_1$-$C_7$ straight or branched alkyl; preferably, the "alkyl" is $C_1$-$C_5$ straight or branched alkyl; preferably, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;

the "modified alkyl group" is a group obtained by replacement of any carbon atom of an alkyl group with at least one group selected from a halogen atom, -O-, -OH, -CO-, -CS-, $-NO_2$, -CN, -S-, $-SO_2$-, -(S=O)-,

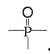,

phenyl group, phenylene group, primary amino group, secondary amino group, tertiary amino group, quaternary ammonium group, saturated or unsaturated monocyclic or bicyclic cyclic-hydrocarbon group, biaryl heterocyclic group, and a bridged aliphatic heterocyclic group, the modified alkyl group having 1 to 30 carbon atoms, and the carbon-carbon single bond is optionally and independently replaced with a carbon-carbon double bond or a carbon-carbon triple bond;

the replacement of carbon atoms refers to that carbon atoms or the carbon atoms and hydrogen atoms thereon together are replaced by corresponding groups;

the "aliphatic ring" is a saturated or unsaturated 4- to 10-membered monocyclic or polycyclic aliphatic ring;

the "aliphatic heterocycle" is a saturated or unsaturated 4- to 10-membered monocyclic or polycyclic aliphatic heterocycle containing at least one heteroatom selected from N, O, S, or Si; if contained on the aliphatic heterocycle, S atoms are in the form of -S-, -SO-, or $-SO_2-$; the aliphatic heterocycle is optionally substituted with halogen atom, nitro group, alkyl group, aromatic group, hydrophilic group, and modified alkyl group;

the "aromatic group or aromatic ring" is a 5- to 10-membered monocyclic or fused bicyclic aromatic group;

the "heteroaryl or aromatic heterocyclic ring" is a 5- to 10-membered monocyclic or fused bicyclic heteroaromatic group containing at least one heteroatom selected from N, O, S or Si on the ring;

the "halogen atom" is respectively and independently selected from F, Cl, Br, I;

the "hydrophilic group" is hydroxyl group, sulfonic acid group, carboxyl group, phosphite group, primary amino group, secondary amino group, or tertiary amino group;

the "bridged aliphatic heterocycle" is a 5- to 20-membered bridged aliphatic heterocycle containing at least one heteroatom selected from N, O, or S on the ring;

the "ester group" is R(C=O)OR' group;

the "phosphite group" is $RP(=O)(OH)_2$ group;

the "sulfonic group" is $RSO_3H$ group;

the "sulfonate ester group" is $RSO_3R'$ group;

the "sulfamine" is $RSO_2NR'R''$ group;

the "primary amino group" is $RNH_2$ group;

the "secondary amino group" is RNHR' group;

the "tertiary amino group" is RNR'R" group;

the "quaternary ammonium group" is R R'R" R‴$N^+$ group;

each R, R', R", R‴ respectively and independently is single bond, alkyl group, alkylene group, modified alkyl group, or modified alkylene group, and the modified alkyl group or modified alkylene group is a group obtained by replacement of any carbon atom of $C_1$-$C_{10}$ (preferably $C_1$-$C_6$) alkyl or alkylene group with a group selected from -O-, -OH, -CO-, -CS-, -(S=O)-;

optionally, the modified alkyl group or modified alkylene group respectively and independently is a group containing at least one group selected from -OH, -O-, ethylene glycol unit ($-(CH_2CH_2O)n-$), $C_1$-$C_8$ alkyl group, $C_1$-$C_8$ alkoxy group, $C_1$-$C_8$ acyloxy group, $C_1$-$C_8$ haloalkyl group, monosaccharide group, disaccharide group, polysaccharide group, -O-CO-, -NH-CO-, $-(-NH-CHR''''-CO-)_n-$, $-SO_2-O-$, -SO-, $-SO_2-NH-$, -S-S-, -CH=CH-, halogen atom, cyano group, nitro group, o-nitrophenyl group, benzoylmethyl group, and phosphate easter group, wherein n is 1 to 100, preferably 1 to 50, more preferably 1 to 30, more preferably 1 to 10; R″″ is H or residue of $\alpha$ amino acid; the "phosphate easter group" is defined as above;

the "monosaccharide unit" is a saccharide unit that can no longer be simply hydrolyzed into smaller sugar molecules;

the "disaccharide unit" is a saccharide unit formed by dehydration of two monosaccharides;

the "polysaccharide unit" is a saccharide unit formed by dehydration of 10 or more monosaccharides;

optionally, the $C_1$-$C_8$ alkyl group is methyl, ethyl, propyl, or isopropyl, the $C_1$-$C_8$ alkoxy group is methoxy, ethoxy, propoxy, or isopropoxy, the $C_1$-$C_8$ acyloxy is acetoxy, ethyl, propyl, or isopropyl, and the $C_1$-$C_8$ haloalkyl is trifluoromethyl, chloromethyl, or bromomethyl;

optionally, the aliphatic heterocycle is selected from azetidine, pyrrolidine, piperidine, tetrahydrofuran, tetrahydropyran, morpholine, and thiomorpholine;

optionally, the conjugate system E is a structure selected from following Formulae (I-2-1) to (I-2-39):

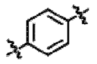 (I-2-1), 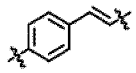 (I-2-2), 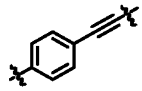 (I-2-3), 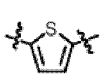 (I-2-4),

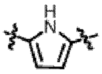 (I-2-5), 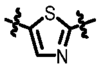 (I-2-6), 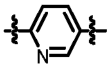 (I-2-7), 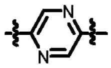 (I-2-8),

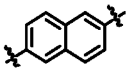 (I-2-9), 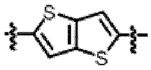 (I-2-10), 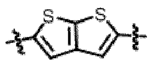 (I-2-11), 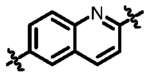 (I-2-12),

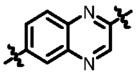 (I-2-13), 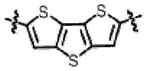 (I-2-14), 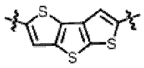 (I-2-15),

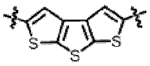 (I-2-16), 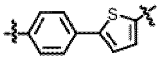 (I-2-17), 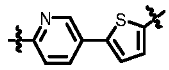 (I-2-18),

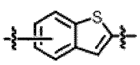 (I-2-19), 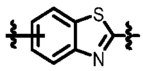 (I-2-20), 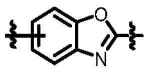 (I-2-21),

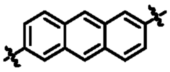 (I-2-22), 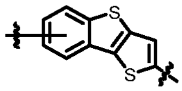 (I-2-23), 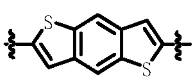 (I-2-24),

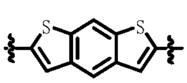 (I-2-25), 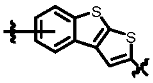 (I-2-26), 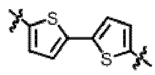 (I-2-27),

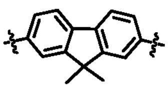 (I-2-28), 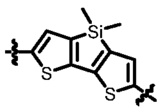 (I-2-29), 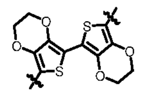 (I-2-30),

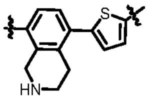 (I-2-31), 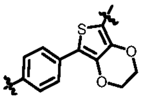 (I-2-32), 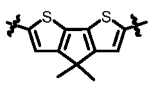 (I-2-33),

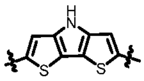 (I-2-34), 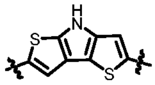 (I-2-35), 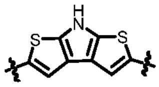 (I-2-36),

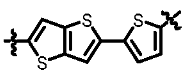 (I-2-37), 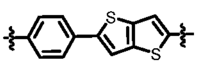 (I-2-38), 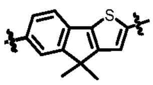 (I-2-39);

optionally, the conjugate system E and -N$X_1$-$X_2$ form an aliphatic heterocycle represented by Formulae (I-3-1) to (I-3-5):

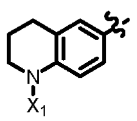 (I-3-1), 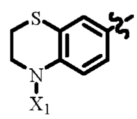 (I-3-2), 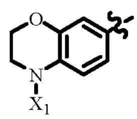 (I-3-3),

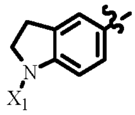 (I-3-4), 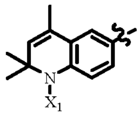 (I-3-5)

or, the conjugate system E and $-NX_1-X_2$ form a structure represented by Formula (I-3-6):

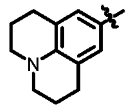 (I-3-6);

and

optionally, the electron acceptor part is one selected from Formulae (I-4-1) to (I-4-42) below:

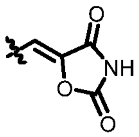 (I-4-1) 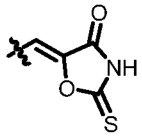 (I-4-2) 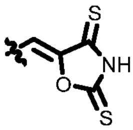 (I-4-3) 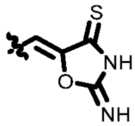 (I-4-4) 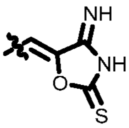 (I-4-5) 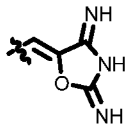 (I-4-6)

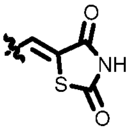 (I-4-7) 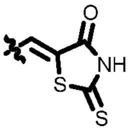 (I-4-8) 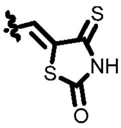 (I-4-9) 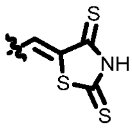 (I-4-10) 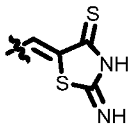 (I-4-11) 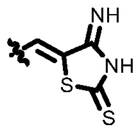 (I-4-12)

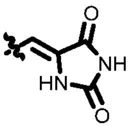 (I-4-13) 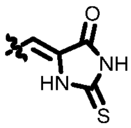 (I-4-14) 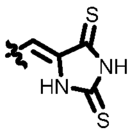 (I-4-15) 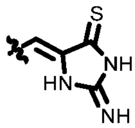 (I-4-16) 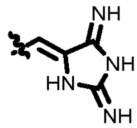 (I-4-17) 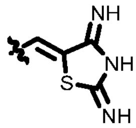 (I-4-18)

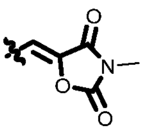 (I-4-19) 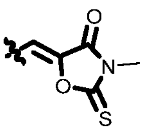 (I-4-20) 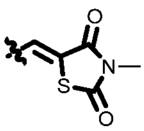 (I-4-21) 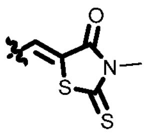 (I-4-22) 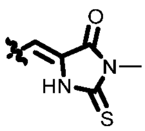 (I-4-23) 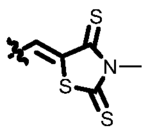 (I-4-24)

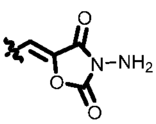 (I-4-25) 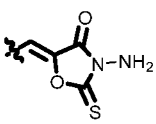 (I-4-26) 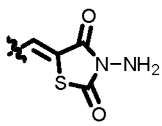 (I-4-27) 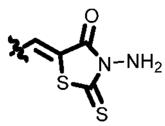 (I-4-28) 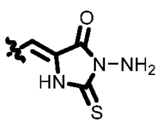 (I-4-29) 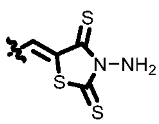 (I-4-30)

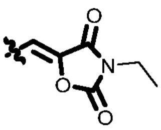 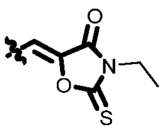 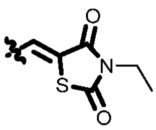 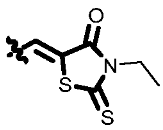 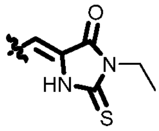 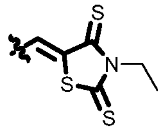

(I-4-31) (I-4-32) (I-4-33) (I-4-34) (I-4-35) (I-4-36)

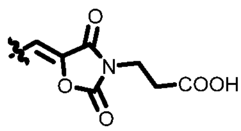 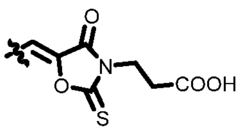 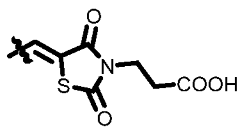 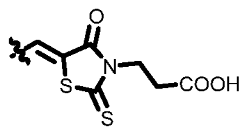

(I-4-37) (I-4-38) (I-4-39) (I-4-40)

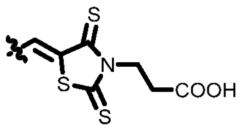 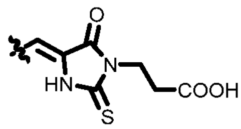

(I-4-41) (I-4-42).

Optionally, the above-mentioned fluorescent probe, wherein the fluorescent probe formula (I) is selected from compounds represented by the formulae below:

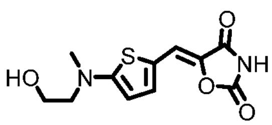 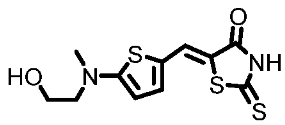 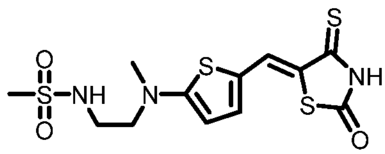

(I-5-1) (I-5-2) (I-5-3)

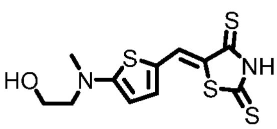 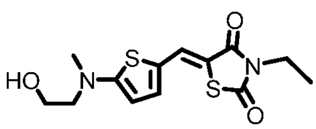 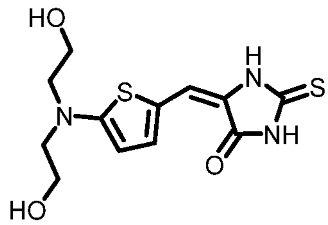

(I-5-4) (I-5-5) (I-5-6)

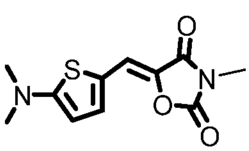 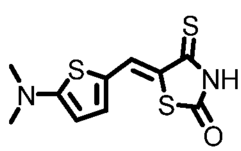 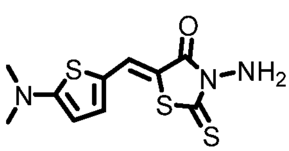

(I-5-7) (I-5-8) (I-5-9)

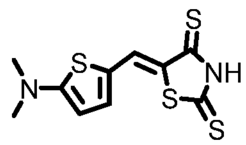
(I-5-10)

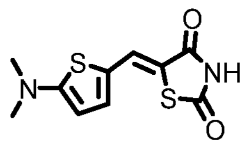
(I-5-11)

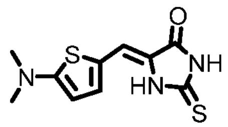
(I-5-12)

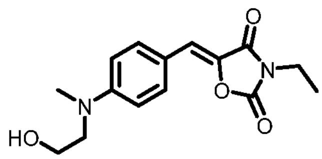
(I-5-13)

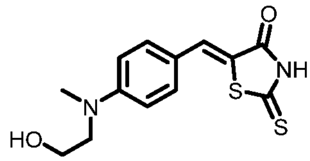
(I-5-14)

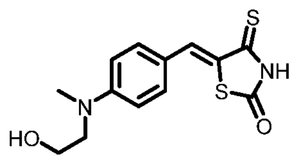
(I-5-15)

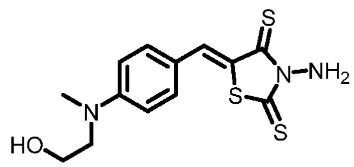
(I-5-16)

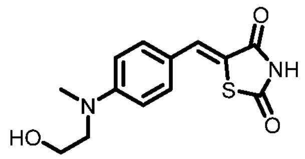
(I-5-17)

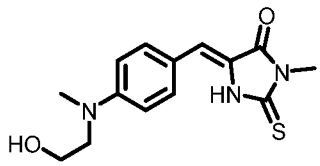
(I-5-18)

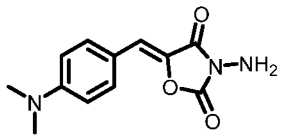
(I-5-19)

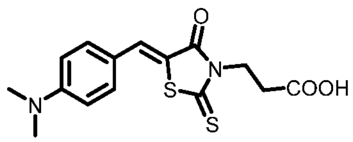
(I-5-20)

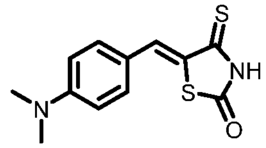
(I-5-21)

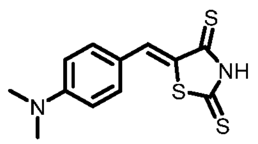
(I-5-22)

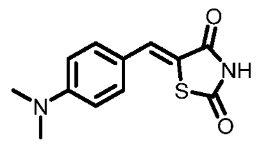
(I-5-23)

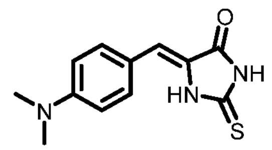
(I-5-24)

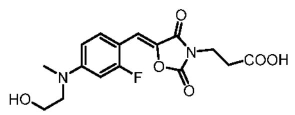
(I-5-25)

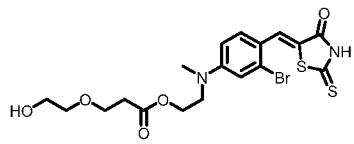
(I-5-26)

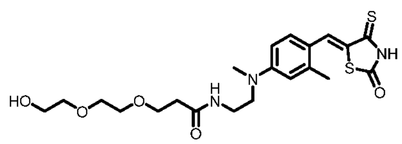
(I-5-27)

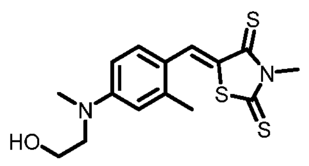
(I-5-28)

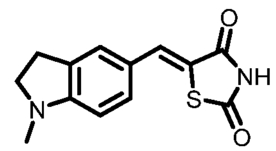
(I-5-29)

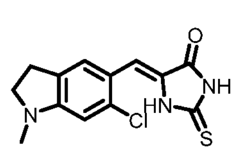
(I-5-30)

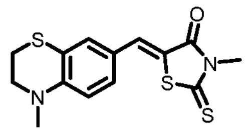
(I-5-31)

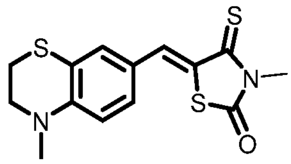
(I-5-32)

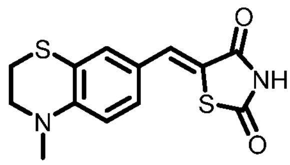
(I-5-33)

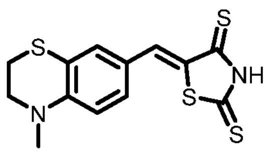
(I-5-34)

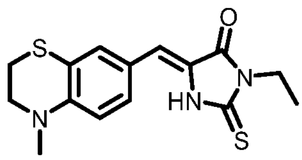
(I-5-35)

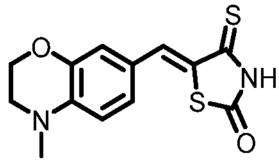
(I-5-36)

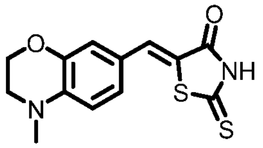
(I-5-37)

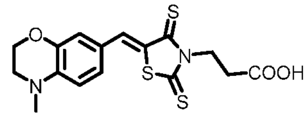
(I-5-38)

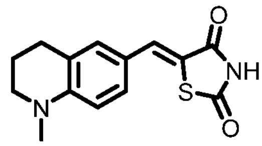
(I-5-39)

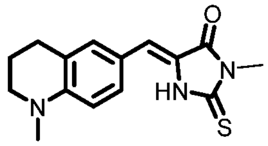
(I-5-40)

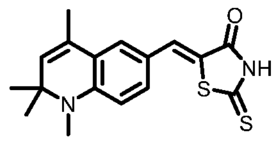
(I-5-41)

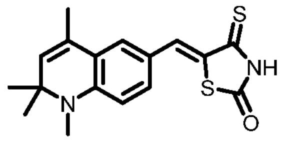
(I-5-42)

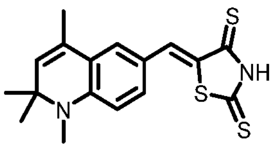
(I-5-43)

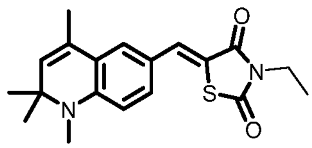
(I-5-44)

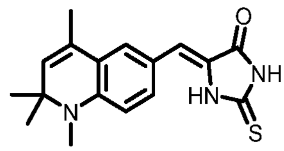
(I-5-45)

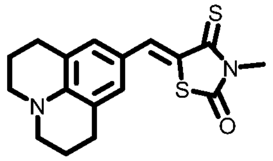
(I-5-46)

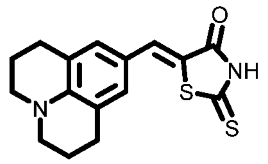
(I-5-47)

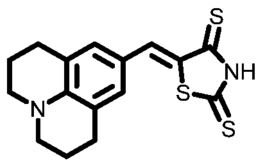
(I-5-48)

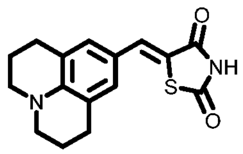
(I-5-49)

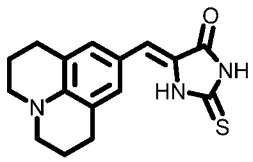
(I-5-50)

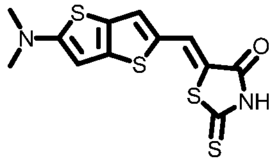
(I-5-51)

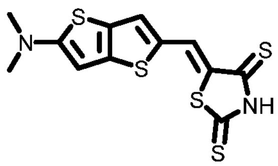
(I-5-52)

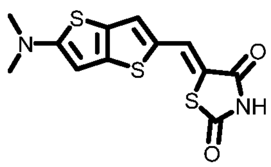
(I-5-53)

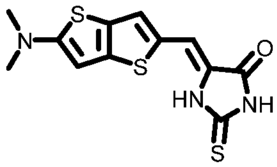
(I-5-54)

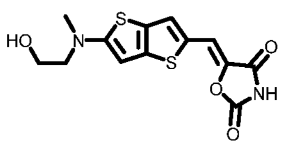
(I-5-55)

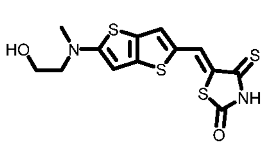
(I-5-56)

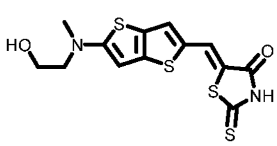
(I-5-57)

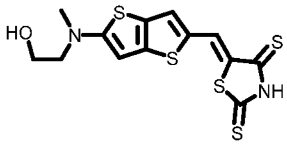
(I-5-58)

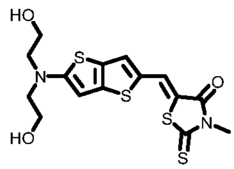
(I-5-59)

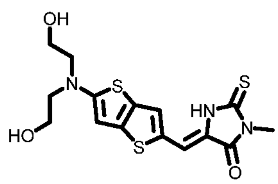
(I-5-60)

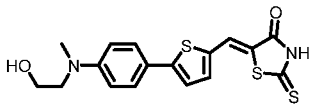
(I-5-61)

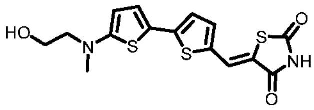
(I-5-62)

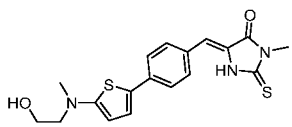
(I-5-63)

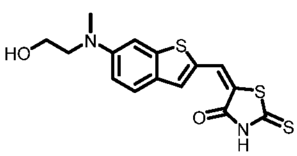
(I-5-64)

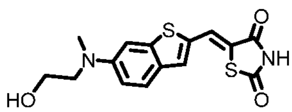
(I-5-65)

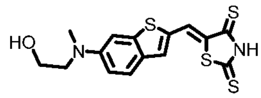
(I-5-66)

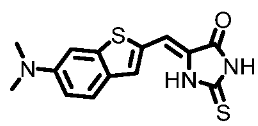
(I-5-67)

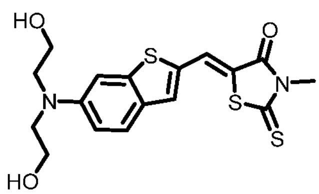
(I-5-68)

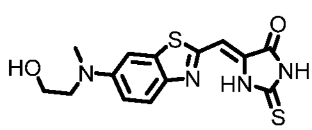
(I-5-69)

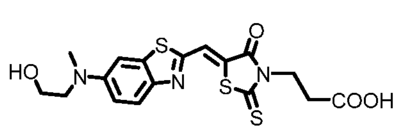
(I-5-70)

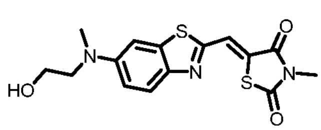
(I-5-71)

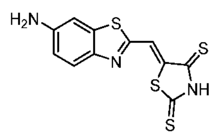
(I-5-72)

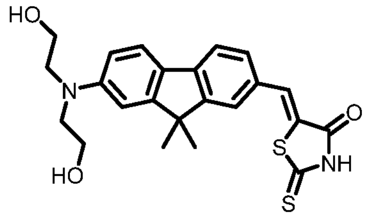
(I-5-73)

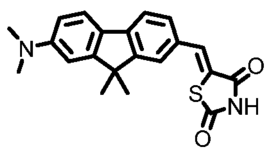
(I-5-74)

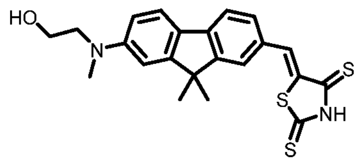
(I-5-75)

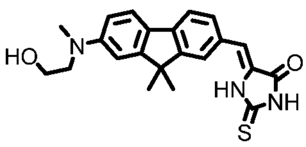
(I-5-76)

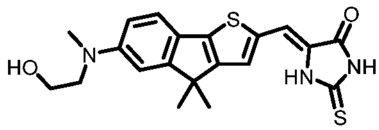
(I-5-77)

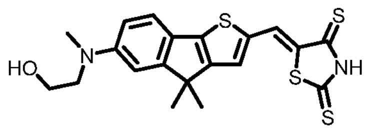
(I-5-78)

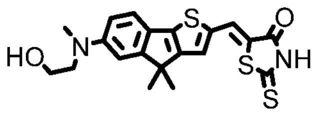
(I-5-79)

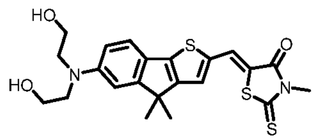
(I-5-80)

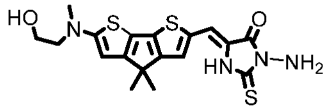
(I-5-81)

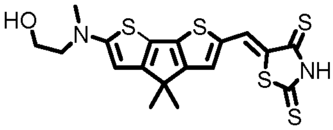
(I-5-82)

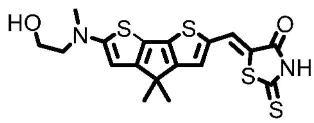
(I-5-83)

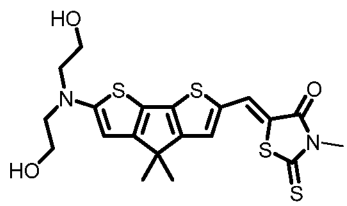
(I-5-84).

In a preferable embodiment of the present disclosure, the fluorophore molecule contains I-5-1, I-5-2, I-5-3, I-5-4, I-5-5, I-5-6, I-5-7, I-5-8, I-5-9, I-5-10, I-5-11, I-5-12, I-5-13, I-5-14, I-5-15, I-5-16, I-5-17, I-5-18, I-5-19, I-5-20, I-5-21, I-5-22, I-5-23, I-5-24, I-5-25, I-5-26, I-5-27, I-5-28, I-5-29, I-5-30, I-5-31, I-5-32, I-5-33, I-5-34, I-5-35, I-5-36, I-5-37, I-5-38, I-5-39, I-5-40, I-5-41, I-5-42, I-5-43, I-5-44, I-5-45, I-5-46, I-5-47, I-5-48, I-5-49, I-5-50, I-5-51, I-5-52, I-5-53, I-5-54, I-5-55, I-5-56, I-5-57, I-5-58, I-5-59, I-5-60, I-5-61, I-5-62, I-5-63, I-5-64, I-5-65, I-5-66, I-5-67, I-5-68, I-5-69, I-5-70, I-5-71, I-5-72, I-5-73, I-5-74, I-5-75, I-5-76, I-5-77, I-5-78, I-5-79, I-5-80, I-5-81, I-5-82, I-5-83 and I-5-84. Expressions such as "improving fluorescence signals", "fluorescence increase", "enhancing fluorescence intensity", "improving fluorescence intensity" in the present disclosure refer to the increase of the quantum yield of the fluorophore or the migration (relative to emission peaks of fluorophore itself in ethanol or aqueous solution) of the maximum emission peak of fluorescence signals under the excitation light of appropriate wavelength, or an increase of molar extinction coefficient, or two or more of the above. In a preferable embodiment of the present disclosure, the quantum yield was increased by at least two times; in another preferable embodiment of the present disclosure, the quantum yield was increased by at least 5 to 10 times; in another more preferable embodiment of the present disclosure, the quantum yield was increased by at least 20 to 50 times; in another more preferable embodiment of the present disclosure, the quantum yield was increased by at least 100 to 200 times; in another more preferable embodiment of the present disclosure, the quantum yield was increased by at least 500 to 1,000 times; in another more preferable embodiment of the present disclosure, the quantum yield was increased by at least 1,000 to 10,000 times; in another more preferable embodiment of the present disclosure, the quantum yield is increased by more than 10,000 times; the light source used for exciting the fluorophore to produce fluorescence signals can be any appropriate lighting device, such as LED lamp, incandescent lamp, fluorescent lamp and laser; excitation light can be either emitted directly from these devices or obtained indirectly by means of other fluorophores, such as donor fluorophores of FERT, or donor luminophors of BRET.

Target molecules

The target molecules of the present disclosure can be any biomaterial or small molecules, including but not limited to: proteins, nucleic acid (RNA or DNA), lipid molecules, carbohydrates, hormones, cytokines, chemokines, and metabolite metal ions and so on. Target molecules can be molecules associated with diseases or pathogen infection.

In the structure shown in FIG. 1B, the inserted nucleotide sequence replaced the stem-loop structures of $N_{11}$, $N_{12}$, $N_{13}$ in FIG. 1A by means of the aptamer molecules in the present disclosure, wherein the nucleotide sequence can specifically identify/bind to target molecules. In the absence of target molecules, aptamer molecules do not or weakly bind to fluorophore molecules, and thus cannot significantly improve the fluorescence of fluorophore molecules under excitation light of appropriate wavelength; in the presence of target molecules, the binding of target molecules and the nucleotide sequence will promote binding of the aptamer molecules and fluorophore molecules, and thus can significantly improve the fluorescence of fluorophore molecules under excitation light of appropriate wavelength, thereby realizing detection, imaging and quantitative analysis of target molecules.

Target molecules can also be whole cells or molecules expressed on the entire cell surface. Typical cells include but are not limited to cancer cells, bacterial cells, fungal cells and normal animal cells. The target molecules can also be virus particles. At present, many aptamers of the afore-mentioned target molecules have been identified, and can be integrated into the polyvalent nucleic acid aptamers of the present disclosure. RNA aptamers that have been reported to bind to target molecules include but are not limited to: T4 RNA polymerase aptamer, HIV reverse transcriptase aptamer, and phage R17 capsid protein aptamer.

Objective nucleic acid molecules

"Objective nucleic acid molecules", also called as "target nucleic acid molecules", refer to the nucleic acid molecules to be detected, which can be either intracellular or extracellular; objective nucleic acid molecules include objective RNA molecules and objective DNA molecules. Objective nucleic acid molecules are connected with the nucleic acid aptamer molecules, and are bound to the nucleic acid aptamer molecules via fluorophore molecules so as to significantly improve the fluorescence value of fluorophore molecules under excitation light of appropriate wavelength, thereby detecting the content and distribution of objective nucleic acid molecules.

"Objective RNA molecules" in the present disclosure include any RNA molecule, including but not limited to pre-mRNA, mRNA of coding cells per se or exogenous expression products thereof, pre-rRNA, rRNA, tRNA, hnRNA, snRNA, miRNA, siRNA, shRNA, sgRNA, crRNA, and long non-coding RNA, wherein phage capsid protein MCP identifies the binding sequence MS2RNA, phage capsid protein PCP identifies the binding sequence PP7RNA, λ phage transcription termination protein N identifies the binding sequence boxB RNA or the like. Target RNA can be fused at 5' end or 3' end or the position of $N_{25}$-$N_{26}$-$N_{27}$ of the RNA aptamer molecules of the present disclosure.

"sgRNA" in the present disclosure refers to single guide RNA (single guide RNA, sgRNA) formed by modifying tracrRNA and crRNA in the CRISPR/Cas9 system, wherein the sequence of about 20 nt at the 5' end of the system targets DNA site via base pair complementation, and promotes the Cas9 protein to induce DNA double-strand break at this site.

Concatemers of nucleic acid aptamer

The nucleic acid aptamer molecules of the present disclosure may further include concatemers that can bind multiple fluorophore molecules. The concatemers are connected by spacer sequences of appropriate length, and the number of N16-1 structures in series may be 2, 3, 4, 5, 6, 7, 8, 9, 10 or greater. The concatemers may be in many forms. In a preferable embodiment of the present disclosure, the tandem form is "Tandem 1", as shown in FIG. 6A, and preferable nucleotide sequences are SEQ ID NO: 6 and 7, wherein 2N16-4 indicates Concatemer 1 with 2 N16-4 structures; in another preferable embodiment of the present disclosure, the tandem form is "Tandem 2", as shown in FIG. 6B, and preferable nucleotide sequences are SEQ ID NO: 8 and 9, wherein 2xN16-5 indicates Concatemer 2 with 2 N16-5 structures; in another preferable embodiment of the present disclosure, the tandem form is "Tandem 3", as shown in FIG. 6C, and preferable nucleotide sequences are SEQ ID NO: 10 and 11, wherein 2x2N16-4 indicates Concatemer 3 with 2 2N16-4 structures; in any form, the spacer sequences between the concatemers can be changed.

The monomer aptamers of the present disclosure refer to aptamers containing only one N16-1 structure, that is, the aptamers containing three stem structures, three loop structures and one stem-loop structure (FIG. 1B).

The polymer aptamers refer to aptamers containing one or more N16-1 structures, including but not limited to the aptamer composed of several tandem forms as shown in FIG. 6.

Aptamer-fluorophore complex

The aptamer-fluorophore complex of the present disclosure contains one nucleic acid aptamer molecule and one or more fluorophore molecules. In an embodiment of the present disclosure, the molecule complex containing one nucleic acid molecule and one fluorophore molecule is F30-N16-1-I-5-14.

In another embodiment of the present disclosure, nucleic acid molecules of the concatemer and a plurality of fluorophore molecules forms a complex, for instance, a complex F30-4N16-4-4×(I-5-14) formed in the way of "Tandem 1" by F30-4N16-4 containing 4 aptamer units and 4 fluorophore molecules. The molecule complex may exist in vitro in the form of two separate solutions, or in the same solution, or in cells.

Nucleic acid aptamer function

The aptamer function of the present disclosure means to significantly enhance fluorescence intensity of fluorophore molecules under excitation light of appropriate wavelength, nucleic acid aptamers can be prepared using the commonly used Experimental method (I) in the embodiments, and the fluorescence value can be detected according to the commonly used Experimental method (II) in the examples. In a preferable embodiment of the present disclosure, the fluorescence intensity is increased by at least two times; in another preferable embodiment of the present disclosure, the fluorescence intensity is increased by at least 5 to 10 times; in another more preferable embodiment of the present disclosure, the fluorescence intensity is increased by at least 20 to 50 times; in another more preferable embodiment of the present disclosure, the fluorescence intensity is increased by at least 100 to 200 times; and in another more preferable embodiment of the present disclosure, the fluorescence intensity is increased by at least 500 to 1,000 times.

Secondary structure of nucleic acid aptamers

In the present disclosure, the secondary structure of nucleic acid aptamers is obtained by simulation and prediction using mFold online analysis software (http://www.unafold.org/RNA_form.php). The stem structure in the secondary structure refers to a local double-strand structure formed by complementary pairing of hydrogen bonds in some regions of the single strand of nucleic acid aptamer molecules. In general, the formation of the double-strand structure does not require complementary pairing of all nucleotides in this region; in general, the stem structure will be formed when complementary pairing occurs between at least 50% of the nucleotides in a fragment of sequences $N_1$ and $N_{39}$, as well as $N_{11}$ and $N_{13}$ and the other fragment. If $N_1$ and $N_{39}$ are single nucleotides, the stem structure can be formed with complete complement of $N_1$ and $N_{39}$ (as shown in FIG. 1A).

DNA molecules expressing nucleic acid aptamers

The DNA molecules contain a DNA sequence which can encode the nucleic acid aptamer molecules of the present disclosure. The DNA molecules contain a nucleotide sequence $R_1$CTCCAGGTG$R_{11}$-$R_{12}$-$R_{13}$GTAACGGACTTAGATCCACTGTACG$R_{39}$, as well as a nucleotide sequence with identity of at least 71%, wherein $R_1$ encodes $N_1$ in the General Formula N16 structure, $R_{11}$ encodes Nn in the General Formula N16 structure, $R_{12}$ encodes $N_{12}$ in the General Formula N16 structure, $R_{13}$ encodes $N_{13}$ in the General Formula N16 structure, and $R_{39}$ encodes $N_{39}$ in the General Formula N16 structure. The DNA molecules may also contain a promoter which controls DNA transcription, wherein the promoter is in operable connection to the DNA sequence encoding the nucleic acid aptamer. In an embodiment of the present disclosure, the DNA molecule contains an U6 promoter; in another embodiment of the present disclosure, the DNA molecules contain a CMV promoter. The DNA molecules may further contain a DNA sequence which encodes any objective nucleic acid molecule. In an embodiment of the present disclosure, the DNA molecules encoding the objective RNA contained a DNA sequence (the sequence for embedding RNA is respectively SEQ ID No: 13) encoding f-actin ACTB.

Promoters

"Promoters" in the present disclosure include promoters of eukaryotic and prokaryotic cells. Promoter sequences of eukaryotic cells are totally different from those of prokaryotic cells. Generally, eukaryotic promoters cannot be identified by RNA polymerases in prokaryotic cells or mediate RNA transcription. Similarly, prokaryotic promoters cannot be identified by RNA polymerases in eukaryotic cells or mediate RNA transcription either. The strength of different promoters varies greatly (strength refers to the ability to mediate transcription). According to actual disclosure, strong promoters can be used for achieving high level transcription. For instance, high level expression is better for labeling, and, for evaluation of transcription behavior, lower-level transcription will allow cells to process transcription in a timely manner. One or more suitable promoters can be selected for different host cells. For instance, being used in Escherichia coli

cells, T7 phage promoter, lac promoter, trp promoter, recA promoter, ribosome RNA promoter, PR and PL promoters in λ phage, and other promoters, but not limited to: lacUV5 promoter, ompF promoter, bla promoter, lpp promoter etc. Moreover, a hybrid trp-lacUV5 promoter (tac promoter) or other Escherichia coli cells obtained through recombinant or synthetic DNA technology can all be used for transcribing the RNA aptamers of the present disclosure. Some of the operator sequences in bacteria per se can combine with promoter sequences to form inducible promoters, and specific inducers need to be added at this moment to induce transcription of DNA molecules. For instance, the expression of lac operator needs to be induced by the addition of lactose or lactose analogues (IPTG), other operators including trp, pro or the like.

As mentioned above, the regulating sequence of 5' end of DNA molecule decoding sequence is promoters. Suitable promoters need to be selected according to the promoter intensity either to obtain RNA aptamers via in vitro transcription or to express aptamers in cultured cells or tissues. Since the expression of aptamers in vivo can be genetically manipulated, another type of promoters is inducible promoters that induce DNA transcription in response to a specific environment, such as in a specific tissue, at a specific time, and in a specific developmental stage. These different promoters can be identified by RNA polymerase I, II or III.

Promotion of transcription in eukaryotic cells also needs suitable promoters, including but not limited to β-globulin promoter, CAG promoter, GAPDH promoter, β-actin promoter, actin promoter, Cstf2t promoter, SV40 promoter, PGK promoter, MMTV promoter, adenovirus Ela promoter, CMV promoter and so on. Termination of transcription in eukaryotic cells depends on the specific cleavage site in RNA sequence. Similarly, since the transcription genes of RNA polymerase are different, transcriptional terminators also vary significantly. However, those skilled in the art can realize screening of suitable 3' transcriptional terminator sub-regions by means of routine experimental skills.

Expression system

The "expression system" of the present disclosure, also called as "expression vector", contains and is integrated with DNA molecules expressing nucleic acid aptamer. The expression system of the present disclosure can be a plasmid or a virus particle.

Recombinant virus of "expression vector" can be obtained by transfection of plasmids into viral-infected cells. Suitable vectors include but are not limited to virus vectors such as λ vector system gt11, gt WES.tB, Charon 4, and plasmid vectors include pBR322, pBR325, pACYC177, pACYC184, pUC8, pUC9, pUC18, pUC19, pLG399, pR290, pKC37, pKC101, pBluescript II SK+/- or KS+/- (see Stratagene cloning system), pET28 series, pACYCDuet1, pCDFDuet1, pRSET series, pBAD series, pQE, pIH821, pGEX, pIIIEx426 RPR and so on.

A large number of host expression systems can be used for expressing the DNA molecules of the present disclosure. Mostly, the vector system has to be compatible to the host cells in use, wherein the host vector system comprises but is not limited to: transformed phage DNA, or plasmid DNA, or bacteria with cosmid DNA; yeast containing yeast vector; mammalian cells infected with a virus (e.g. adenovirus, adeno-associated virus, retrovirus); insect cells infected with a virus (e.g. baculovirus); and plant cells infected with bacteria or transformed by means of particle bombardment. Expression elements in the vectors are significantly different in strength and characteristics. Any one or more suitable transcription elements can be selected according to the host-vector system in use.

Once the constructed DNA molecules are cloned into the vector system, it will be easy to transfer them into host cells. Based on different vector or host cell systems, the method comprises but is not limited to transformation, transduction, conjugation, fixation, electrical transfer or the like.

An embodiment of the present disclosure provides expression plasmids pET28a-T7-F30-N16-1 and pLKO.1-F30-N16-1 containing DNA molecules for encoding F30-N16-1 RNA. Another embodiment of the present disclosure provides expression plasmid pLKO.1-F30-5x(F30-2xN16-1) containing DNA molecules for encoding F30-2xN16-1 RNA. Another embodiment of the present disclosure provides expression plasmids pCDNA3.1/hygro(+)-EGFP-F30-5x(F30-2xN16-1) and pCDNA3.1/hygro(+)-ACTB-F30-5x(F30-2xN16-1) containing DNA molecules for encoding EGFP-F30-5x (F30-2xN16-1) and ACTB-F30-5x(F30-2xN16-1). Another embodiment of the present disclosure provides expression plasmid pLKO.1-N16-1-MS2 containing DNA molecules for encoding N16-1-MS2.

The present disclosure further provides expression vectors integrated with DNA molecules for encoding nucleic acid aptamers, but with vacant encoding DNA sequences of objective RNA molecules, wherein the vacancy of encoding DNA sequences of objective RNA molecules allows the users to choose DNA sequences of objective RNA molecules to be detected, for instance, corresponding encoding DNA sequence of GAPDH mRNA inserts the DNA sequence into the expression vector of the present disclosure by means of standard recombination DNA technology, and guides the obtained expression vector into the host cells of (transfection, transform, infection and so on), thereby detecting the content and distribution of objective RNA.

Host cells

"Host cells" in the present disclosure include but are not limited to bacteria, yeast, mammalian cells, insect cells, plant cells, zebra fish cells, fruit fly cells, and nematode cells. Host cells preferably are cultured cells in vitro or whole in vivo living tissue. Mammalian cells contained in the host cells of the present disclosure include but are not limited to 297T, COS-7, BHK, CHO, HEK293, HeLa, H1299, stem cells of fertilized eggs, inducible totipotent stem cell, and primary cells isolated directly from mammalian tissues and so on; escherichia coli cells contained therein include but are not limited to BL21 (DE3), BL21 (DE3, Star), TOP10, Mach1, and DH5$\alpha$; and yeast cells contained therein include but are not limited to BY4741, BY4742, and AH109.

Detection array

The detection array of the present disclosure comprises one or more nucleic acid aptamer molecules of the present disclosure, wherein the nucleic acid aptamer molecules are anchored at discrete locations on the array surface composed of solid supports, including but not limited to glass, metals, and ceramic and so on. The nucleic acid aptamer molecules of the present disclosure can be anchored to the array surface by, but not limited to, the following methods: (1) labeling the 5' end or 3' end of the nucleic acid aptamer molecule with biotin, coating the array surface with streptavidin, and anchoring the nucleic acid aptamer molecule by specific binding of biotin and streptavidin; (2) identifying the binding sequence MS2 by using the phage capsid protein MCP, identifying the biding sequence PP7 by using the phage capsid protein PCP or identifying the binding sequence boxB by using the $\lambda$ phase transcription terminating protein N, fusing the RNA sequence at the 5', 3' or stem-loop structure of the nucleic acid aptamer molecules, coating the array surface with protein MCP, PP7 or $\lambda_N$ protein identified and bound thereby, and anchoring the nucleic acid aptamer molecules through the specific effects of MS2 with MCP protein, PP7 with PCP protein or boxB RNA with $\lambda_N$ protein; (3) fusing a fragment of RNA or DNA sequence at the 5' end or 3' end of the nucleic acid aptamer molecules, anchoring an RNA sequence in complementary pairing with the RNA sequence segment or an DNA sequence in complementary pairing with the DNA sequence segment on the array surface, and anchoring the nucleic acid aptamer molecules on the array surface by means of the molecular hybridization principle. The detection array can be used for detecting the presence or absence of the target molecule as well as the concentration level, as a result, the nucleic acid aptamer molecules be bound with the fluorophore molecules and significantly improve the fluorescence intensity under excitation light of appropriate wavelength only with the presence of target molecules; and, within a certain range, the higher the concentration of the target molecules, the higher the fluorescence intensity.

Kit

Kit of the present disclosure comprises the nucleic acid aptamer molecules and/or the fluorophore molecules of the present disclosure, and corresponding instructions; or comprises an expression system for expressing the nucleic acid aptamer molecules and/or the fluorophore molecules, and corresponding instructions; or comprises host cells expressing the aptamer molecular expression system and/or the fluorophore molecules, and corresponding instructions. The nucleic acid aptamer molecules and the fluorophore molecules in the kits respectively exist in individual solutions, or exist in the same solution.

**Embodiments**

The present disclosure will be further elaborated in the following examples, which are merely used for giving examples, rather than limiting the scope of the present disclosure. The examples mainly adopt conventional cloning methods of molecular biology in genetic engineering, which are well known to ordinary technicians in this field, for instance, relevant chapters from *Lab Ref:* A Handbook of Recipes, Reagents, and Other Reference Tools for Use at the Bench by Jane Roskams et al, and Molecular Cloning-A Laboratory Manual (Third Edition, August 2002, Science Press, Beijing) written by Sambrook.J, D. W. Russell and translated by Peitang HUANG et al. Based on the following examples, it is easy for one skilled in the art to successfully implement the present disclosure after slight amendment and change made according to actual situations.

In the examples, the pCDNA3.1/hygro(+) plasmid vector was purchased from Invitrogen Company, pLKO.1-puro plasmid vector was purchased from Sigma Company, pET28a plasmid vector was purchased from Novagen Company, pYES2.1 TOPO TA plasmid vector was purchased from Invitrogen. PCR primers used in the examples were synthesized, purified and identified correct via mass spectrometry by Shanghai Generay Biotech Co., Ltd. Taq DNA polymerase used in examples was purchased from Yeasen Biotechnology (Shanghai) Co., Ltd., PrimeSTAR DNA polymerase was purchased from TaKaRa Company, and corresponding polymerase buffers and dNTP were included during purchasing of these three kinds of polymerases. EcoRI, BamHI, BglII, HindIII, NdeI, XhoI, SacI, XbaI, SpeI and

other restriction endonuclease, T4 ligase, T4 phosphorylase (T4 PNK), and T7 RNA polymerase were purchased from Fermentas Company, and corresponding polymerase buffers and so on were included during purchasing. Kanamycin was purchased from Ameresco Company; and Amp was purchased from Ameresco Company. Expression plasmids constructed in the examples all went through the sequence determination accomplished by JIE LI BIOLOGY

DNA purification kits used in the examples were purchased from BBI Company (Canada), ordinary plasmid micro extraction kits were purchased from Tiangen Biotech (Beijing) Co., Ltd, Hieff Clone™ One Step cloning kits used in the examples were purchased from Yeasen Biotechnology (Shanghai) Co., Ltd, and Eastep Super total RNA extraction kit was purchased from Promega Company. BL21 (DE3, Star) bacterial strains were purchased from Invitrogen Company. 293T/17 cells and COS-7 cells were purchased from Cell Bank of Committee of Typical Culture Collection, Chinese Academy of Sciences. 384-well and 96-well fluorescence detection blackboards were purchased from Grenier Company. DFHBI-1T and DFHO were purchased from Lucerna Company. All the chemical reagents used in the examples were purchased from chemical reagent companies, such as Titan Technology, Bidepharm, and Leyan, etc., and were directly used without further purification. Drying solvents used in the experiments, such as methanol and methylene chloride, were directly purchased from Titan Technology and used without further treatment.

Main instruments used in the examples are as follows: Synergy Neo2 Multiscan Spectrum (Bio-Tek Company of America), X-15R high speed freezing centrifuge (Beckman Company of America), Microfuge22R tabletop high speed freezing centrifuge (Beckman Company of America), PCR amplifier (Biometra Company of German), in vivo imaging system (Kodak Company of America), photometer (Wako Company of Japan), nucleic acid electrophoresis apparatus (Shenneng Bocai Company), Bruker Avance 600 (400 MHz)-type NMR spectrometer, Micromass GCTTM-type mass spectrometer, Micromass LCTTM-type mass spectrometer, Leica SP8 laser scanning confocal microscope, and Zeiss Elyra PS.1 super-resolution imaging microscope.

Meanings of abbreviations are as follows: "h" stands for hour, "min" stands for minute, "s" stands for second, "d" stands for day, "μL" stands for micro-liter, "ml" stands for milliliter, "L" stands for liter, "bp" stands for base pair, "mM" stands for millimole, and "μM" stands for micromole.

**Commonly-used experimental methods and materials in the examples**

**(I) Preparation of nucleic acid aptamer molecules:**

A primer containing T7 promoter was used for amplification of corresponding cDNA of RNA to be detected, and T7 RNA polymerase (purchased from Fermentas) was used for obtaining RNA by means of transcription with recovered double-stranded cDNA as a template. 10 μL of 3 M NaAc and 115 μL of DEPC water were added into 20 μL of transcription system, and were mixed well before 150 μL of phenol chloroform-isopropyl alcohol mixture (phenol:chloroform:isopropyl alcohol=25:24:1) was added; then the mixture was mixed well by means of oscillation, and the supernatant was taken after centrifugation at 10,000 rpm for 5 min. An equal volume of chloroform solution was added and was mix well by means of oscillation, and then the supernatant was taken after centrifugation at 10,000 rpm for 5 min; the process was repeated once. 2.5 times the volume of anhydrous ethanol was added into the supernatant, and the mixture was placed in a refrigerator at -20°C for 30 min and was subject to centrifugation at 12,000 rpm at 4°C for 5 min; then the supernatant was removed, and the precipitation was cleaned twice with 75% pre-cooled anhydrous ethanol. After volatilization of the ethanol, the precipitation was re-suspended with an appropriate amount of screening buffer, and then was treated at 75°C for 5 min and was placed at room temperature for more than 10 min for subsequent experiments.

**(II) Function detection of the nucleic acid aptamer**

Nucleic acid aptamer molecules of N16 or N16 mutants were prepared by using the commonly-used experimental method (I), 2.5 μM of nucleic acid aptamer molecules and 0.5 μM of fluorophore molecules were incubated in a detection buffer (40 mM of HEPES, pH 7.4, 125 mM of KCl, 5 mM of $MgCl_2$, 5% DMSO), and the maximum excitation peak and the maximum emission peak of the fluorescence of the nucleic acid aptamer-fluorophore molecular complex were detected with Synergy Neo2 multifunctional enzyme-labeler. Synergy Neo2 multifunctional enzyme-labeler was used again to detect fluorescence intensity of the nucleic acid aptamer-fluorophore molecular complex under its maximum excitation and emission conditions, the control sample (1 μM of fluorophore molecules without nucleic acid aptamer) was also measured under the same conditions, and the ratio of fluorescence intensity was calculated. For example, as for fluorescence of the complex formed by 2.5 μM of N16-1 nucleic acid aptamer and 0.5 μM of I-5-14 fluorophore molecules, the maximum excitation peak is 512 nm and the maximum emission peak is 574 nm. It was detected by the Synergy Neo2 multifunctional enzyme-labeler that the fluorescence intensity of the complex at an excitation condition of 512±10 nm and an emission condition of 574 nm±10 nm was 49,088, while the fluorescence intensity of the control (0.5 μM of I-5-14 fluorophore molecules) under the same detection conditions was 399, so the activation multiple of

N16-1 nucleic acid aptamer to I-5-14 fluorophore molecules was 123.

**(III) Construction of recombinant plasmid based on homologous recombination**

1. Preparation of linearized vector: an appropriate cloning site was selected and the vector was linearized, and the linearized vector could be prepared by means of enzyme digestion or reverse PCR amplification.
2. Preparation of an inserted fragment by means of PCR amplification: homologous sequences of 15-25 bp (excluding the enzyme cut site) at the ends of a linearized vector was introduced into the 5' end of positive and reverse PCR primers of the inserted fragment, so that the 5' and 3' ends of the PCR products of the inserted fragment respectively had exactly the same sequences corresponding to the two ends of the linearized vector.
3. Concentration measurement of the linearized vector and the inserted fragment: amplified products of the linearized vector and the inserted fragment were subjected to several equal volume dilution gradients, 1 μL of original product and 1 μL of diluted product were respectively taken for agar-gel electrophoresis, and the band brightness was compared with DNA molecular weight standard (DNA Marker) so as to determine approximate concentration thereof.
4. Recombination reaction

The optimal usage amount of vector in the recombination reaction system was 0.03 pmol; and the molar ratio of the optimal vector to the inserted fragment is 1:2 to 1:3, namely, the optimal usage amount of inserted fragment is 0.06 to 0.09 pmol.

| Components | Recombination Reaction |
|---|---|
| $ddH_2O$ | 20 μL at most |
| 2×Hieff Clone Enzyme Premix (purchased from Yeasen Company) | 10 μL |
| Linearized vector | X μL |
| Inserted fragment | Y μL |

Respectively, X and Y are usage amounts of linearized vectors and inserted fragments calculated according to the formula. After the system is prepared, all components were mixed well and were subject to reaction at 50°C for 20 min. When the inserted fragments were greater than 5 kb, the incubation time could be prolonged to 25 min. When the reaction was completed, it was recommended that the reaction tube is placed on ice for 5 min to be cooled. The reaction products could be converted directly or stored at -20°C for thawing and conversion when needed.

**(IV) Cell culture and transfection:**

293T/17 cells in this example were cultured in a $CO_2$ incubator with a high-glucose medium (RPMI) containing 10% fetal bovine serum (FBS) as well as streptomycin and penicillin; and COS-7 cells in this Example were all cultured in a $CO_2$ incubator with a high-glucose medium (DMEM) containing 10% fetal bovine serum (FBS) as well as streptomycin and penicillin, and the cells were subject to subculturing when the growth reached confluent degree of 80-90%. Transfection was performed by using FuGENE®HD (purchased from Promega) according to the instructions.

**(V) Fluorescent imaging:**

Main imaging experiments in the examples were carried out by using a confocal laser microscope Leica SP8 equipped with white laser, and an HCXPL APO 63x1.47 oil mirror and a HyD detector were used.

**Example 1.** Secondary structure of N16 nucleic acid aptamer molecules

The secondary structure of N16 nucleic acid aptamer was analyzed with mFold online RNA structure analysis software. N16 consists of 3 stem structures, 3 ring structures, and 1 stem-ring structure (FIG. 1A). For a sequence of one stem and stem-ring, a secondary structure (FIG. 1B) of N16-1 was predicted (SEQ ID NO: 1).

**Example 2.** Fluorescence activation effects of different N16-1 mutants for I-5-14 fluorophore molecules.

In order to detect fluorescence activation effects of different N16-1 mutants for I-5-14 fluorophore molecules, point mutations, as shown in Table 1, were performed for sequences in N16-1, and N16-1 mutant RNA containing different base mutations were prepared by using the commonly-used experimental method (I), wherein: 0.5 μM of I-5-14 was incubated respectively with 2.5 μM of different N16-1 mutant RNAs, and fluorescence activation multiples thereof for I-5-14 fluorophore molecules were measured by using the commonly-used experimental methods (II). According to the results, most N16-1 mutants with a single base mutation retained a relatively strong fluorescence activation effect (no smaller than 2 times) on I-5-14 (Table 2). Some N16-1 mutants with 2 to 5 base mutations still retained a relatively strong fluorescence activation effect (greater than 20 times) for I-5-14 (Table 3). In a word, many single-base and multi-base mutants of N16-1 might still retain the aptamer function of activating the I-5-14 fluorophore molecules.

**Table 2 Activation effects of N16-1 mutants containing a single base mutation for 1-5-14**

| Mutants | Activation Multiple | Mutants | Activation Multiple | Mutants | Activation Multiple |
|---|---|---|---|---|---|
| N16-1 | 123 | A16C | 80 | A27G | 41 |
| C2A | 2 | A16G | 90 | U28A | 46 |
| C2U | 7 | A17U | 2 | U28C | 37 |
| C2G | 3 | A17C | 2 | U28G | 49 |
| U3A | 53 | A17G | 95 | C29A | 2 |
| U3C | 82 | C18A | 28 | C29U | 52 |
| U3G | 86 | C18U | 6 | C29G | 14 |
| C4A | 96 | C18G | 85 | C30A | 2 |
| C4U | 44 | G19A | 2 | C30U | 4 |
| C4G | 113 | G19U | 2 | C30G | 2 |
| C5A | 69 | G19C | 2 | A31U | 6 |
| C5U | 43 | G20A | 2 | A31C | 2 |
| C5G | 84 | G20U | 2 | A31G | 14 |
| A6U | 75 | G20C | 2 | C32A | 41 |
| A6C | 4 | A21U | 2 | C32U | 22 |
| A6G | 59 | A21C | 18 | C32G | 64 |
| G7A | 2 | A21G | 23 | U33A | 7 |
| G7U | 2 | C22A | 41 | U33C) | 2 |
| G7C | 2 | C22U | 159 | U33G) | 7 |
| G8A | 25 | C22G | 79 | G34A) | 57 |
| G8U | 2 | U23A | 18 | G34U) | 52 |
| G8C | 2 | U23C | 87 | G34C) | 73 |
| U9A | 91 | U23G | 7 | U35A | 79 |
| U9C | 66 | U24A | 54 | U35C | 43 |
| U9G | 58 | U24C | 143 | U35G | 90 |
| G10A | 16 | U24G | 151 | A36U | 47 |
| G10U | 2 | A25U | 52 | A36C | 57 |
| G10C | 2 | A25C | 54 | A36G | 15 |
| G14A | 2 | A25G | 71 | C37A | 60 |
| G14U | 2 | G26A | 93 | C37U | 63 |

(continued)

| Mutants | Activation Multiple | Mutants | Activation Multiple | Mutants | Activation Multiple |
|---|---|---|---|---|---|
| G14C | 2 | G26U | 92 | C37G | 68 |
| U15A | 2 | G26C | 44 | G38A | 2 |
| U15C | 15 | A27U | 12 | G38U | 2 |
| U15G | 2 | A27C | 76 | G38C | 2 |
| A16U | 81 | | | | |
| Note: N16-1 in Table 2 is a nucleic acid aptamer with a sequence SEQ ID NO: 1; and other aptamers are point mutations performed at a nucleotide position in N16-1 sequence corresponding to N16-1 in FIG. 1B. | | | | | |

**Table 3 Activation effects of N16-1 mutant containing multi-base mutation for 1-5-14**

| Mutants | Activation Multiple | Mutants | Activation Multiple |
|---|---|---|---|
| N16-1 | 123 | C4G/U9A/A17G | 45 |
| C4G/U9A | 58 | C4G/U9A/C22U | 57 |
| C4G/A16G | 64 | C4G/U9A/U24G | 63 |
| C4G/A17G | 25 | C4G/U9A/U35G | 78 |
| C4G/C22U | 26 | C4G/A16G/A17G | 91 |
| C4G/U24G | 24 | C4G/A16G/C22U | 72 |
| C4G/U35G | 35 | C4G/A16G/U24G | 86 |
| U9A/A16G | 37 | C4G/A16G/U35G | 45 |
| U9A/A17G | 64 | C4G/A17G/C22U | 57 |
| U9A/C22U | 71 | C4G/A17G/U24G | 63 |
| U9A/U24G | 69 | C4G/A17G/U35G | 37 |
| U9A/U35G | 78 | C4G/C22U/U24G | 67 |
| A16G/A17G | 95 | C4G/C22U/U35G | 42 |
| A16G/C22U | 21 | C4G/U24G/U35G | 58 |
| A16G/U24G | 28 | C4G/C22U/U24G/U9A | 91 |
| A16G/U35G | 29 | C4G/C22U/U24G/A16G | 74 |
| A17G/C22U | 35 | C4G/C22U/U24G/A17G | 57 |
| A17G/U24G | 42 | C4G/C22U/U24G/U35G | 62 |
| A17G/U35G | 47 | C4G/C22U/U24G/U9A/A16G | 28 |
| C22U/U24G | 51 | C4G/C22U/U24G/U9A/A17G | 35 |
| C22U/U35G | 56 | C4G/C22U/U24G/U9A/U35G | 41 |
| U24G/U35G | 48 | C4G/C22U/U24G/A16G/A17G | 49 |
| C4G/U9A/A16G | 39 | C4G/C22U/U24G/A16G/U35G | 54 |
| | | C4G/C22U/U24G/A17G/U35G | 61 |

**Example 3.** Secondary structure of N16-1 nucleic acid aptamer molecules

The secondary structure (FIG. 1B) of N16-1 (SEQ ID NO: 1) nucleic acid aptamer was analyzed by using mFold online RNA structure analysis software. The secondary structure (FIG. 2A) of F30-N16-1 (SEQ ID NO: 2) nucleic acid

aptamer was obtained by F30 scaffold RNA connection. The secondary structure (FIG. 2B) of tRNA-N16-1 (SEQ ID NO: 3) nucleic acid aptamer was obtained by tRNA scaffold RNA connection.

**Example 4.** Identification of spectral properties of N16-1-I-5-14 complex

In order to detect whether the N16-1-I-5-14 complex can be activated by ultraviolet light, N16-1 (SEQ ID NO: 2) RNA was prepared by using the commonly-used experimental method (I). 2 μM of I-5-14 was incubated with 10 μM of N16-1. According to the results, none of the pure I-5-14 fluorophore solution, the mixed solution of I-5-14 fluorophore molecules added with negative control RNA, or the mixed solution of N16-1 and I-5-14 fluorophore molecules containing RNase A had obvious fluorescence under ultraviolet irradiation. However, the mixed solution of N16-1 and the I-5-14 fluorophore molecules emitted bright yellow fluorescence under ultraviolet irradiation (FIG. 3A).

In order to detect the spectral properties of N16-1-I-5-14 complex, N16-1 (SEQ ID NO: 1) RNA was prepared by using the commonly-used experimental method (I). 1 μM of I-5-14 was incubated with 5 μM of N16-1. According to the results, the maximum excitation light of N16-1-I-5-14 complex was 512 nm, and the maximum emission light thereof was 574 nm (FIG. 3B).

In order to detect the difference between the light absorption of N16-1-I-5-14 complex and that of I-5-14 fluorophore molecules per se, 2 μM of I-5-14 incubated with 10 μM of N16-1, or 5 μM of pure I-5-14 was used for detecting the light absorption of I-5-14 and that of N16-1-I-5-14 complex respectively. According to the results, the maximum light absorption of I-5-14 was 477 nm, while the maximum light absorption of N16-1-I-5-14 complex had a large red shift relative to I-5-14 itself, and the maximum light absorption thereof was 502 nm (FIG. 3C).

In order to detect the binding constant of N16-1 and I-5-14, 10 nM of N16-1 was incubated with I-5-14 of different concentrations, so that fluorescence values thereof could be detected. According to the results, the binding constant of N16-1 and I-5-14 was 15.69 nM (FIG. 3D).

**Example 5.** Stability identification of N16-1-I-5-14 complex

In order to detect the stability of N16-1-I-5-14 complex at different pH, N16-1-I-5-14 complex was placed in different pH environments for 60 min, and the fluorescence value thereof was detected. According to the results, N16-1-I-5-14 complex maintained rather strong fluorescence signals when pH ranged from 5 to 9 (FIG. 4A), indicating that N16-1-I-5-14 complex had good pH stability.

In order to detect the dependence of the N16-1-I-5-14 complex on $Mg^{2+}$ ions, 1 μM of N16-1 and 5 μM of I-5-14 were incubated in buffer solutions with different magnesium ion contents, respectively. According to the results, the concentration of magnesium ions had a weak influence on the fluorescence of N16-1-I-5-14 complex (FIG. 4B), indicating that N16-1-I-5-14 complex had good magnesium ion stability.

In order to detect the temperature stability of N16-1, 10 μM of I-5-14 was incubated with 1 μM of N16-1 in a detection buffer solution containing 5 mM of $Mg^{2+}$, and then was placed at different temperatures for 5 min to detect the fluorescence value thereof. According to the results, the Tm value of N16-1-I-5-14 was 61.2°C (FIG. 4C), indicating that the N16-1-I-5-14 complex had good temperature stability.

In order to detect the dependence of N16-1-I-5-14 complex on $K^+$ ions, 10 μM of I-5-14 and 1 μM of N16-1 were incubated in a buffer solution containing 100 mM of KCl or 100 mM of LiCl, and were treated at 70°C for 5 min, and then were placed at room temperature for more than 15 min, and then fluorescence values under different conditions were detected. As reported in previous documents, the stability of the G-quadruplet structure was highly dependent on the presence of $K^+$ ions. According to the results, the fluorescence of N16-1-I-5-14 complex was independent of the presence of $K^+$ ions (FIG. 4D), suggesting that there was no G-quadruplet structure in the N16-1 structure.

**Example 6.** Property identification of I-5-14 analogues

F30-N16-1 RNA aptamer molecules were prepared by using the commonly-used experimental method (I), and were used for detecting basic properties, including fluorescence spectrum and fluorescence activation multiple, of the binding of I-5-14 analogues with N16. The results were shown in Table 4, indicating that F30-N16-1 could activate the fluorescence of I-5-14 analogues to different extents.

**Table 4:** Determination of physicochemical properties of binding of F30-N16-1 RNA aptamer molecules with different fluorescent molecules

| | Maximum excitation peak (nm) | Maximum emission peak (nm) | Activation Multiple |
|---|---|---|---|
| F30-N16-1-I-5-1 | 460 | 500 | 14 |

(continued)

| | Maximum excitation peak (nm) | Maximum emission peak (nm) | Activation Multiple |
|---|---|---|---|
| F30-N16-1-I-5-2 | 546 | 572 | 68 |
| F30-N16-1-I-5-3 | 550 | 580 | 14 |
| F30-N16-1-I-5-4 | 625 | 638 | 47 |
| F30-N16-1-I-5-5 | 487 | 518 | 11 |
| F30-N16-1-I-5-6 | 536 | 550 | 23 |
| F30-N16-1-I-5-7 | 465 | 504 | 24 |
| F30-N16-1-I-5-8 | 554 | 582 | 15 |
| F30-N16-1-I-5-9 | 552 | 578 | 17 |
| F30-N16-1-I-5-10 | 626 | 642 | 11 |
| F30-N16-1-I-5-11 | 493 | 524 | 9 |
| F30-N16-1-I-5-12 | 535 | 555 | 11 |
| F30-N16-1-I-5-13 | 393 | 492 | 8 |
| F30-N16-1-I-5-14 | 513 | 574 | 123 |
| F30-N16-1-I-5-15 | 567 | 598 | 31 |
| F30-N16-1-I-5-16 | 615 | 648 | 11 |
| F30-N16-1-I-5-17 | 445 | 506 | 105 |
| F30-N16-1-I-5-18 | 473 | 545 | 57 |
| F30-N16-1-I-5-19 | 397 | 499 | 18 |
| F30-N16-1-I-5-20 | 493 | 565 | 22 |
| F30-N16-1-I-5-21 | 569 | 605 | 24 |
| F30-N16-1-I-5-22 | 613 | 657 | 126 |
| F30-N16-1-I-5-23 | 433 | 515 | 156 |
| F30-N16-1-I-5-24 | 473 | 543 | 7 |
| F30-N16-1-I-5-25 | 403 | 502 | 6 |
| F30-N16-1-I-5-26 | 497 | 567 | 21 |
| F30-N16-1-I-5-27 | 565 | 603 | 11 |
| F30-N16-1-I-5-28 | 615 | 654 | 11 |
| F30-N16-1-I-5-29 | 453 | 525 | 14 |
| F30-N16-1-I-5-30 | 497 | 562 | 11 |
| F30-N16-1-I-5-31 | 503 | 610 | 125 |
| F30-N16-1-I-5-32 | 519 | 625 | 15 |
| F30-N16-1-I-5-33 | 442 | 553 | 154 |
| F30-N16-1-I-5-34 | 547 | 659 | 11 |
| F30-N16-1-I-5-35 | 487 | 585 | 10 |
| F30-N16-1-I-5-36 | 517 | 625 | 9 |
| F30-N16-1-I-5-37 | 497 | 603 | 256 |
| F30-N16-1-I-5-38 | 545 | 647 | 7 |
| F30-N16-1-I-5-39 | 429 | 509 | 14 |
| F30-N16-1-I-5-40 | 473 | 548 | 8 |
| F30-N16-1-I-5-41 | 517 | 612 | 85 |
| F30-N16-1-I-5-42 | 527 | 627 | 11 |
| F30-N16-1-I-5-43 | 572 | 665 | 17 |
| F30-N16-1-I-5-44 | 459 | 549 | 21 |
| F30-N16-1-I-5-45 | 497 | 595 | 11 |
| F30-N16-1-I-5-46 | 542 | 633 | 7 |
| F30-N16-1-I-5-47 | 529 | 607 | 245 |
| F30-N16-1-I-5-48 | 579 | 675 | 6 |
| F30-N16-1-I-5-49 | 469 | 539 | 1025 |

(continued)

| | Maximum excitation peak (nm) | Maximum emission peak (nm) | Activation Multiple |
|---|---|---|---|
| F30-N16-1-I-5-50 | 512 | 597 | 7 |
| F30-N16-1-I-5-51 | 582 | 654 | 1125 |
| F30-N16-1-I-5-52 | 686 | 725 | 7 |
| F30-N16-1-I-5-53 | 518 | 593 | 1281 |
| F30-N16-1-I-5-54 | 562 | 633 | 9 |
| F30-N16-1-I-5-55 | 513 | 627 | 14 |
| F30-N16-1-I-5-56 | 568 | 627 | 12 |
| F30-N16-1-I-5-57 | 579 | 647 | 1279 |
| F30-N16-1-I-5-58 | 692 | 725 | 1422 |
| F30-N16-1-I-5-59 | 581 | 655 | 12 |
| F30-N16-1-I-5-60 | 552 | 626 | 8 |
| F30-N16-1-I-5-61 | 483 | 677 | 6 |
| F30-N16-1-I-5-62 | 529 | 689 | 256 |
| F30-N16-1-I-5-63 | 521 | 673 | 6 |
| F30-N16-1-I-5-64 | 522 | 665 | 12 |
| F30-N16-1-I-5-65 | 462 | 605 | 35 |
| F30-N16-1-I-5-66 | 666 | 748 | 81 |
| F30-N16-1-I-5-67 | 516 | 652 | 7 |
| F30-N16-1-I-5-68 | 536 | 663 | 9 |
| F30-N16-1-I-5-69 | 531 | 661 | 11 |
| F30-N16-1-I-5-70 | 556 | 686 | 12 |
| F30-N16-1-I-5-71 | 471 | 627 | 6 |
| F30-N16-1-I-5-72 | 636 | 721 | 14 |
| F30-N16-1-I-5-73 | 681 | 731 | 12 |
| F30-N16-1-I-5-74 | 635 | 691 | 14 |
| F30-N16-1-I-5-75 | 741 | 791 | 7 |
| F30-N16-1-I-5-76 | 724 | 774 | 9 |
| F30-N16-1-I-5-77 | 736 | 783 | 5 |
| F30-N16-1-I-5-78 | 582 | 681 | 8 |
| F30-N16-1-I-5-79 | 682 | 733 | 11 |
| F30-N16-1-I-5-80 | 686 | 743 | 7 |
| F30-N16-1-I-5-81 | 635 | 713 | 7 |
| F30-N16-1-I-5-82 | 748 | 801 | 3 |
| F30-N16-1-I-5-83 | 685 | 733 | 4 |
| F30-N16-1-I-5-84 | 685 | 737 | 6 |

**Example 7.** Activation effect of base-modified N16-1 for I-5-14

In order to detect the activation effect of base-modified N16-1 on I-5-14, base-modified N16-2 (SEQ ID NO: 4, the underlined base in the sequence: GGGCUCCAGGUGCTGCTTCGGCAGGUAACGGACUUAGAUCCACUGUACGCCCC is a deoxyribose nucleotide base) and N16-3 (SEQ ID NO: 5, underlined bases in the sequence: GGGCUCCAGGUGCUGCUUCGGCAGGUAACGGACUUAGAUCCACUGUACGCCC are 2'-F modified bases) (synthesized by Shanghai GenePharma Co., Ltd) were synthesized, and they contained stem-loop structure bases replaced with deoxyribonucleotides (bases in the dash area in FIG. 5A), and some of the bases were modified by 2'-F (bases in the dash area in FIG. 5B), respectively. Fluorescence activation effects of these base-modified N16-1 for I-5-14 fluorophore molecules were detected by using the commonly-used experimental method (II). According to the results, base-modified N16-2 and N16-3 could still significantly activate the fluorescence of the I-5-14 fluorophore molecules (FIG. 5C).

**Example 8.** N16-1 concatemer

In order to detect the activation effect of N16-1 concatemer for I-5-14 fluorescence, N16-1 was connected in series in the following three different ways:

(1) In the way of "Tandem 1" (FIG. 6A), the "head" and "tail" on N16-1 structure are connected in the way of "head-tail" connection, so as to obtain nN16-1 (where n is N16-1 that can be arbitrarily copied). In this example, coding cDNAs of F30-2N16-4 and F30-4N16-4 were respectively synthesized by whole genes (sequences of coding RNA aptamers are SEQ ID NO: 6 and SEQ ID NO: 7), and nucleic acid aptamer RNA was prepared by using the commonly-used experimental method (I) after PCR amplification; 0.1 μM of RNA aptamer was incubated with 10 μM of I-5-14, and fluorescence intensity was detected by using the commonly-used experimental method (II). According to the results, the nN16-4-I-5-14 fluorescence increased with n; the nN16-4-I-5-14 fluorescence did not increase by equal multiples, but was still much higher than the N16-1-I-5-14 fluorescence (FIG. 6D), indicating that the fluorescence intensity of N16-1-I-5-14 complex can be enhanced by means of "Tandem 1".
(2) In the way of "Tandem 2" (FIG. 6B), N16-1 is connected in series as a structural unit, so as to obtain nxN16-1 (where n is N16-1 that can be arbitrarily copied). In this example, coding cDNAs of 2xN16-5 and 4xN16-5 were respectively synthesized by whole genes (sequences of coding RNA aptamers are SEQ ID NO: 8 and SEQ ID NO: 9), and nucleic acid aptamer RNA was prepared by using the commonly-used experimental method (I); 0.1 μM of RNA aptamer was incubated with 10 μM of I-5-14, and fluorescence intensity was detected by using the commonly-used experimental method (II). According to the results, the nxN16-1-I-5-14 fluorescence increased with n (FIG. 6E), indicating that the fluorescence intensity of N16-1-I-5-14 complex can be enhanced by means of "Tandem 2".
(3) In the way of "Tandem 3" (FIG. 6C), the afore-mentioned "Tandem 1" and "Tandem 2" are combined, and nN16-1 obtained by means of "Tandem 1" is connected in series as a structural unit in the way of "Tandem 2", so as to obtain n1x n2N16-1 (where n1 and n2 are N16-1 that can be arbitrarily copied). In this example, coding cDNAs of 2x2N16-4 and 4x2N16-4 were respectively synthesized by whole genes (sequences for coding RNA aptamers are SEQ ID NO: 10 and SEQ ID NO: 11), and nucleic acid aptamer RNA was prepared by using the commonly-used experimental method (I); 0.1 μM of RNA aptamer was incubated with 20 μM of I-5-14, and fluorescence intensity was detected by using the commonly-used experimental method (II). According to the results, fluorescence intensity of the N16-1 concatenate-1-5-14 complex obtained by means of "Tandem 3" was significantly higher than that of N16-1-I-5-14 (FIG. 6C), indicating that the fluorescence intensity of N16-1-I-5-14 complex can be enhanced by means of "Tandem 3".

**Example 9.** Labeling of RNA in bacteria by using F30-N16-1-I-5-14 complex

In order to detect the effect of F30-N16-1-1-5-14 in bacteria, a bacterial expression plasmid expressing F30-N16-1 (SEQ ID NO: 2) was constructed first. F30-N16-1 in Example 2 was amplified with primers, pET28a was amplified with primers so as to remove the promoter and polyclonal site region, and then the F30-N16-1 DNA fragment obtained by means of amplification was connected to pET28a linearized vector by using the experimental method (III), and the obtained recombinant plasmid was named as pET28a-T7-F30-N16-1.

Primers used for amplification of F30-N16-1 fragment are as follows:

Upstream primer (P1):
5’-TTGCCATGTGTATGTGGGTTCGCCCACATACTCTGATGATCC-3’
Upstream primer (P2):

5’-CCACATACTCTGATGATCCGGGCTCCAGGTGCTGCTTCGGCAGGTAACGGACTTAGATC-3’

Downstream primer (P3):
5’-TTGCCATGAATGATCCGGGCGTACAGTGGATCTAAGTCCGTTACCTGC-3’

Primers used for amplification of pET28a vector so as to linearize it are as follows:

Upstream primer (P4):
5’-GCCCGGATCATTCATGGCAATAGCATAACCCCTTGGGGCC-3’

Downstream primer (P5):

5'-GAACCCACATACACATGGCAACCCTATAGTGAGTCGTATTAATTTC-3'

BL21 (DE3, Star) escherichia coli strain was transformed with recombinant plasmid pET28a-T7-F30-N16-1, and monoclone was picked and cultured at 37°C; when $OD_{600}$ is about 0.2, 1 mM of IPTG was added to induce the expression of F30-N16-1, and bacteria were collected 4 h later; resuspension was performed with a PBS solution containing 2 μM of I-5-14. BL21 (DE3, Star) escherichia coli with transformed pET28a empty vector served as control. According to the results, the bacteria could show bright yellow fluorescence only with F30-N16-1 expressed and in the presence of I-5-14 (FIG. 7), indicating that the N16-1-I-5-14 complex can be used for fluorescent labeling of RNA in bacteria.

**Example 10.** Labeling of RNA in mammalian cells by using circular-N16-1 and I-5-14, and analogues thereof

In order to detect the expression of circular-N16-1 and I-5-14 in mammalian cells, a mammalian cell expression plasmid expressing circular N16-1 (SEQ ID No: 12) was constructed. Gene fragment of circular N16-1 was synthesized by whole gene, and served as a template for amplification with primers P6 and P7. Primers used for amplifying circular-N16-1 fragment were as follows:

Upstream primer (P6): 5'-GGGCCGCACTCGCCGGTCCC-3'
Downstream primer (P7): 5'-GCGTGGACTGTACCCTCCAC-3'

Primers used for amplification of pLKO. 1 puro vector so as to linearize it are as follows:

Upstream primer (P8):
5'-GTGGAGGGTACAGTCCACGCTTTTTTGAATTCTCGACCTCG-3'
Downstream primer (P9):
5'-GGGACCGGCGAGTGCGGCCCTCTAGAGTTTCGTCCTTTCCAC-3'

These fragments were inserted into pLKO.1 puro vector by using the experimental method (III), an expression vector obtained thereby was named as pLKO.1-circular-N16-1, and the plasmid expressed circular-N16-1.

293T/17 cells were transfected with pLKO.1-circular-N16-1 plasmid, 1 μm of I-5-14 was added 36 h later so as to label circular-N16-1, cells not expressing the corresponding aptamer served as control, and the labeling effect was detected by using experimental method (V). According to the results, the circular-N16-1-I-5-14 complex showed very bright yellow fluorescence (FIG. 8).

**Example 11.** Labeling of RNA in mammalian cells by using F30-N16-1 and I-5-14, and analogues thereof

In order to detect labeling of RNA in mammalian cells by using F30-N16-1 and I-5-14, a mammalian cell expression plasmid expressing F30-N16-1 (SEQ ID NO: 2) was constructed. F30-N16-1 in Example 9 was amplified with primers P1, P2, and P3, respectively.

Primers used for amplification of pLKO.1 puro vector so as to linearize it are as follows:

Upstream primer (P10):
5'-GCCCGGATCATTCATGGCAATTTTTTTGAATTCTCGACCTCG-3'
Downstream primer (P11):
5'-CGAACCCACATACACATGGCAATCTAGAGTTTCGTCCTTTCCAC-3'

These fragments were inserted into pLKO.1 puro vector by using the experimental method (III), an expression vector obtained thereby was named as pLKO.1-F30-N16-1, and the plasmid expressed F30-N16-1.

293T/17 cells were transfected with pLKO.1-F30-N16-1 plasmid, 1 μm of I-5-14 was added 36 h later so as to label F30-N16-1, cells not expressing the corresponding aptamer served as control, and the labeling effect was detected by using experimental method (V). According to the results, the F30-N16-1-I-5-14 complex showed very bright yellow fluorescence (FIG. 9).

**Example 12.** Labeling of mRNA in living cells with N16-1-I-5-14 as a label

In order to verify labeling of f-actin ACTB mRNA in living cells with N16-1 and I-5-14 as labels, mammalian cell expression plasmids were constructed. According to a published document (Chen et al. Nature biotechnology 2019. 37: 1287-1293), pCDNA3.1/hygro(+)-ACTB and pCDNA3.1/hygro(+)-ACTB-N16-1 were constructed, and these plasmids

expressed ACTB and ACTB-N16-1, respectively (SEQ ID NO: 13).

Firstly, expression plasmids of chimeric RNA fusing N16-1 with different RNAs were constructed. Since the N16-1 fragment is short, the N16-1 gene fragment obtained by primer bridging could be inserted into pCDNA3.1/hygro(+) vector by means of primer homologous recombination, thereby obtaining pCDNA3.1/hygro(+)-N16-1 recombinant plasmid.

Primers used for amplifying pCDNA3.1/hygro(+)-N16-1 fragment are as follows:

Upstream primer (P12):

5’-CGGCAGGTAACGGACTTAGATCCACTGTACGCCCGCGGCTCTAGAGGGCCCGTTTAAAC-3’

Downstream primer (P13):

5’-TAAGTCCGTTACCTGCCGAAGCAGCACCTGGAGCCCGCGGCCTCGAGGGATCCGAGCTC-3’

ACTB gene fragments (ACTB RNA sequence: ACCESSION NM_001101) were synthesized by means of whole gene and were amplified with primers, and were inserted into the primer-linearized pCDNA3.1/hygro(+)-N16-1 vector by using experimental method (III), thereby obtaining pCDNA3.1/hygro(+)-ACTB-N16-1 recombinant plasmid and encoding ACTB-N16-1 chimeric RNA.

Primers used for amplifying ACTB fragments are as follows:

Upstream primer (P14): 5’-ATGGATGATGATATCGCCGC-3’
Downstream primer (P15): 5’-CTAGAAGCATTTGCGGTGGAC-3’

Primers used for amplification of pCDNA3.1/hygro(+)-ACTB-N16-1 vector so as to linearize it are as follows:

Upstream primer (P16):
5’-GTCCACCGCAAATGCTTCTAGCTCGAGGCCGCGGGCTCCAGG-3’
Downstream primer (P17):
5’-GCGGCGATATCATCATCCATGGATCCGAGCTCGGTACCAAG-3’

The pCDNA3.1/hygro(+)-ACTB and pCDNA3.1/hygro(+)-ACTB-N16-1 plasmids were transfected into COS-7 cells, 0.5 μM of I-5-14 was added for labeling 24 h later, and the labeling effect was detected by means of the experimental method (V). According to the results, N16-1-I-5-14 could be used for labeling ACTB mRNA (FIG. 10).

**Example 13.** N16-1 serving as a label for RNA extraction and purification

In order to detect the application of N16-1 to RNA extraction and purification, the pCDNA3.1/hygro(+)-ACTB-N16-1 recombinant plasmids in Example 13 were transfected into COS-7 cells, cells were collected 24 h later, and total RNA of cells was extracted by using Eastep Super total RNA extraction kit (Promega). The extracted total RNA dissolved in a buffer solution having a pH value of 7.4 and containing 40 mM of HEPES, 125 mM of KCl and 5 mM of $MgCl_2$, and was incubated at 70°C for 10 min, and then was placed at room temperature for more than 30 min.

500 uL of Activated Thiol Sepharose 4B (GE Healthcare) was taken and was washed twice with 500 μL of PBS, and PBS containing 10 mM of TCEP (Sigma) was added for incubation at room temperature for 1 h. 500 μL of PBS was used for cleaning twice, I-5-14 fluorophore molecules (Mal-1-5-14) containing maleic amide were added for reaction at room temperature for 30 min, and then 500 μL of PBS was used for cleaning for three times. Total RNA treated in the above way was incubated with treated microbeads at room temperature, and was subject to centrifugation at 4,000 rpm for 2 min after 30 min; then the supernatant was discarded, and agarose microbeads were cleaned with a buffer having a pH value of 7.4 and containing 40 mM of HEPES, 125 mM of KCl and 5 mM of MgCh for 6 times, wherein the supernatant was removed each time by means of centrifugation. The microbeads were resuspended with DEPC water and were treated at 70°C for 10 min, and then were subject to centrifugation at 4,000 rpm for 2 min before the supernatant was collected. 1/10 volume of NaAc and 2.5 times volume of anhydrous ethanol were added into the collected supernatant, and then the mixture was placed in the refrigerator at -80°C for 20 min, and was subject to centrifugation at 14,000 rpm for 10 min at 4°C; the precipitation was remained while the supernatant was discarded, was cleaned with

pre-cooled 70% ethanol solution, and was subject to centrifugation at 14,000 rpm for 10 min at 4°C; then the precipitation was remained while the supernatant was discarded; the whole process was repeated once. The precipitation was placed at room temperature for 5 min, and was resuspended with a small volume of DEPC water after volatilization of the ethanol.

With crushed supernatant of blank cells as control, the fluorescence of the crushed supernatant of cells incubated with fluorophore I-5-14, and that of the eluent after final high temperature elution incubated with fluorophore I-5-14 were respectively detected. According to the results, the fluorescence of the eluent incubated with I-5-14 was significantly higher than that of the crushed supernatant before sample loading (FIG. 11), which proved that ACTB-N16-1 RNA was well enriched, indicating that N16-1 could be used as a label for RNA separation and purification.

**Example 14.** Synthesis of I-5-14 analogues

Embodiments of the present disclosure will be described in detail below. It should be noted that the embodiments described here merely serve as exemplary description, rather than limits, of the present disclosure.

Compound 1

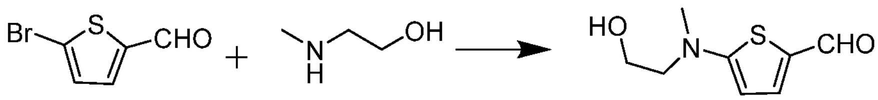

Compound 1

5-bromothiophene-2 methanal (0.5 g, 2.6 mmol) and 2-methylaminoethanol (0.78 g, 10.4 mmol) dissolved into 10 ml of water, and were heated to 100°C by means of oil bath under Ar protection and were subject to reaction overnight; after the reaction, they were cooled to room temperature, extracted with dichloromethane for three times, washed with saturated salt solution, and dried with anhydrous sodium sulfate; the organic phase was dried by means of spinning, and the residue was subjected to column chromatography, as a result, 0.4 g of Compound 1 was obtained, and the yield was 84%. 1H NMR (400 MHz, DMSO-d6) δ 9.40 (s, 1H), 7.65 (d, J=4.5 Hz, 1H), 6.12 (d, J=4.5 Hz, 1H), 4.86 (t, J=5.4 Hz, 1H), 3.62 (q, J=5.5 Hz, 2H), 3.47 (t, J=5.6 Hz, 2H), 3.09 (s, 3H).

(I-5-1)

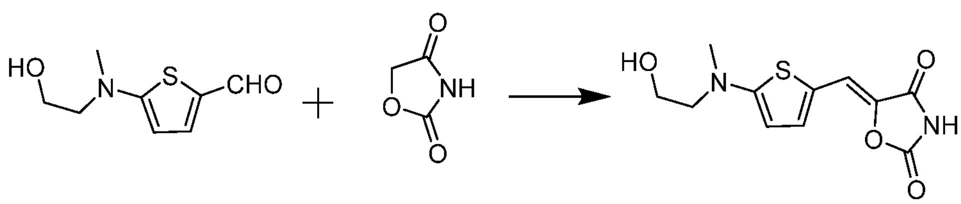

Compound 1 (I-5-1)

Compound 1 (0.2 g, 1.08 mmol) and 2,4-oxazolidinedione (0.13 g, 1.30 mmol) dissolved into 50 ml of absolute ethyl alcohol, were added with a catalytic dose of piperidine, and were heated to 90°C by means of oil bath under Ar protection and were subject to reaction for 3 h; after the reaction, they were cooled to room temperature, and a large amount of solid precipitated in the system; the solid was filtered, and the filter cake was washed twice with cold alcohol and then was subject to vacuum drying, thereby obtaining 0.2 g of a yellow compound (I-5-1), and the yield was 71.4%. 1HNMR (400 MHz, DMSO-d6) δ 12.09 (s, 1H), 7.81 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.09 (s, 3H).

(I-5-2)

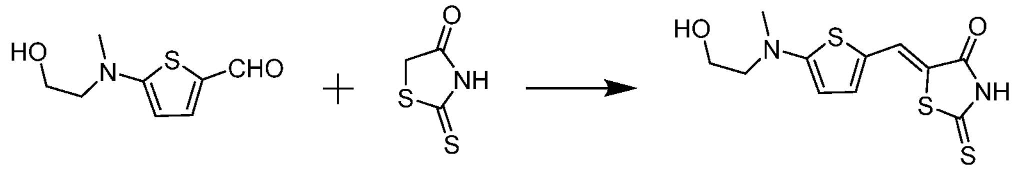

Compound 1 (I-5-2)

(I-5-2) was synthesized with reference to the synthetic method of (I-5-1), and the yield was 75.2%. 1H NMR (400 MHz, DMSO-d6) δ 13.09 (s, 1H), 7.71 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.34 (s, 3H).

Compound 2

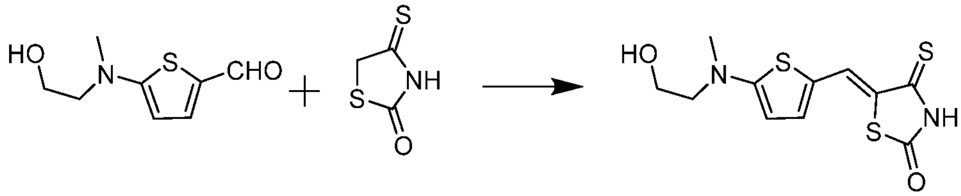

Compound 1 Compound 2

Compound 2 was synthesized with reference to the synthetic method of (I-5-1), and the yield was 71.6%. 1H NMR (400 MHz, DMSO-d6) δ 14.09 (s, 1H), 7.01 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.09 (s, 3H).

Compound 3

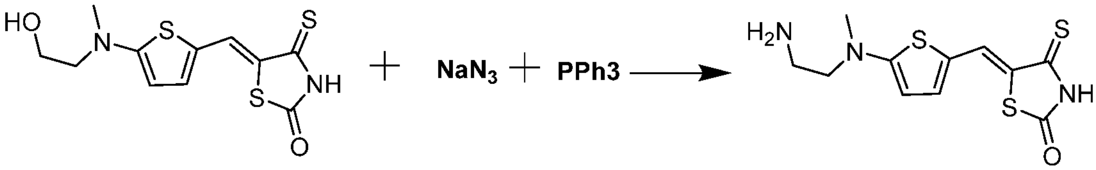


Compound 2 Compound 3

Compound 2 (0.5 g, 1.66 mmol) dissolved in acetonitrile, and then sodium azide (1.08 g, 16.6 mmol) was added; then the mixture was heated to 60°C by means of oil bath and was subject to reaction for 24 h; after reaction of the raw material Compound 2 was over, extraction was performed with dichloromethane and water, organic phase was combined, and a yellow solid was obtained by means of spinning and drying; then the yellow solid dissolved with tetrahydrofuran and water (10:1), and triphenylphosphine (0.52 g, 1.98 mmol) was added for reduction; the reaction lasted for 4 h; after the reaction was completed, tetrahydrofuran was dried by means of spinning and was extracted with dichloromethane and water; organic phase was combined, crude product was dried by means of spinning, and 0.25 g of Compound 3 was obtained via silica gel column chromatography with a yield of 50.3%. 1H NMR (400 MHz, DMSO-d6) δ 14.09 (s, 1H), 7.01 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.09 (s, 3H).

(I-5-3)

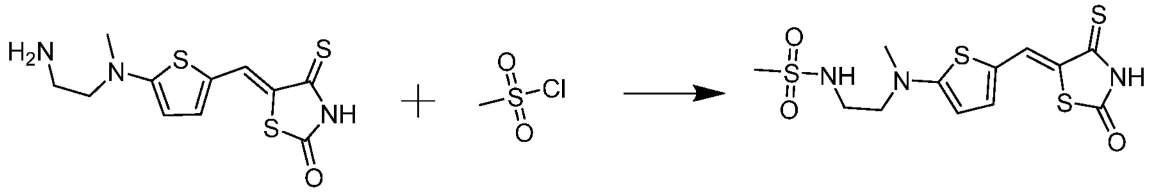

Compound 3 (I-5-3)

Compound 3 (0.25 g, 0.84 mmol) dissolved in dichloromethane, and methylsulfonyl chloride (0.114 g, 1.0 mmol) dissolved by dichloromethane was added under nitrogen protection for a reaction of 30 min at room temperature; after complete reaction of Compound 3, extraction was performed with dichloromethane and water, organic phase was combined and was dried by means of spinning, thereby obtaining the crude product; (I-5-3), which is a yellow solid, was obtained via silica gel column chromatography with a yield of 59.4%. 1H NMR (400 MHz, DMSO-d6) δ 14.09 (s, 1H), 7.01 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.09 (s, 3H), 2.93 (s, 3H).

(I-5-4)

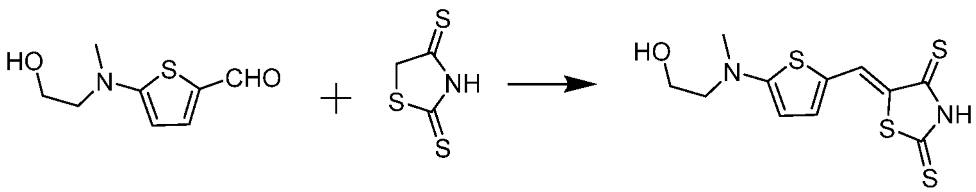

Compound 1 (I-5-4)

(I-5-4) was synthesized with reference to the synthetic method of (I-5-1), and the yield was 73.2%. 1H NMR (400 MHz, DMSO-d6) δ 16.09 (s, 1H), 7.91 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 2H), 3.46 (t, J=5.6 Hz, 2H), 3.09 (s, 3H).

(I-5-5)

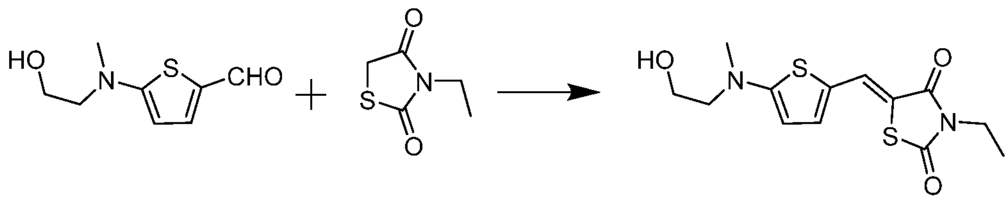

Compound 1 (I-5-5)

(I-5-5) was synthesized with reference to the synthetic method of (I-5-1), and the yield was 74.3%. 1H NMR (400 MHz, DMSO-d6) δ 12.10 (s, 1H), 7.71 (s, 1H), 7.43 (d, J=4.4 Hz, 1H), 6.15 (d, J=4.4 Hz, 1H), 4.87 (t, J=5.3 Hz, 1H), 4.24 (q,2H), 3.62 (q, J=5.5 Hz, 2H), 3.46 (t, J=5.6 Hz, 2H), 3.09 (s, 3H), 1.20 (t, 3H).

Compound 4

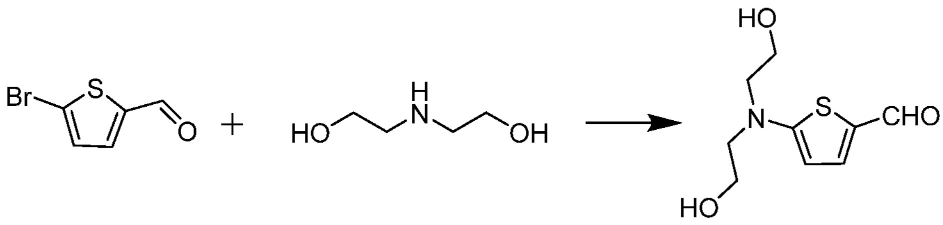

Compound 4

5-bromothiophene-2 methanal (0.5 g, 2.6 mmol) dissolved into 10 ml of diethanolamine, and was heated to 100°C by means of oil bath under Ar protection and was subject to reaction overnight; after the reaction, it was cooled to room temperature, extracted with dichloromethane for three times, washed with saturated salt solution, and dried with anhydrous sodium sulfate; the organic phase was dried by means of spinning, and the residue was subjected to column chromatography, as a result, 0.32 g of Compound 4 was obtained, and the yield was 57.1%. 1H NMR (400 MHz, DMSO-d6) δ 9.40 (s, 1H), 7.65 (d, J=4.5 Hz, 1H), 6.12 (d, J=4.5 Hz, 1H), 4.86 (t, J=5.4 Hz, 1H), 3.62 (q, J=5.5 Hz, 4H), 3.47 (t, J=5.6 Hz, 4H), 3.09 (s, 3H).

(I-5-6)

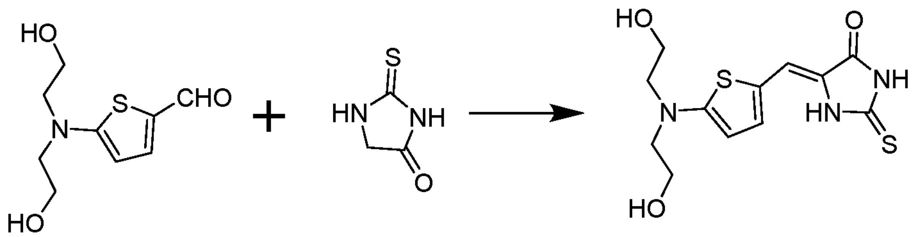

Compound 4 (I-5-6)

(I-5-6) was synthesized with reference to the synthetic method of (I-5-1), and the yield was 70.4%. 1H NMR (400 MHz, DMSO-d6) δ 12.10 (s, 1H), 11.20 (s, 1H),7.51 (s, 1H), 7.43 (d, J=4.4 Hz, 1H), 6.15 (d, J=4.4 Hz, 1H), 4.87 (t, J=5.3 Hz, 1H), 3.62 (q, J=5.5 Hz, 4H), 3.46 (t, J=5.6 Hz, 4H), 3.09 (s, 3H).

Compound 5

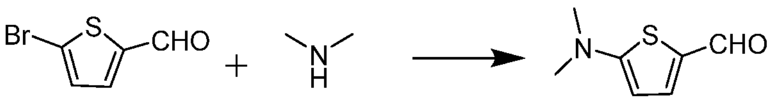

Compound 5

Compound 5 was synthesized with reference to the synthetic method of Compound 1, and the yield was 92.5%. 1H NMR (400 MHz, DMSO-d6) δ 10.40 (s, 1H), 7.65 (d, J=4.5 Hz, 1H), 6.12 (d, J=4.5 Hz, 1H), 3.09 (s, 6H).

(I-5-7)

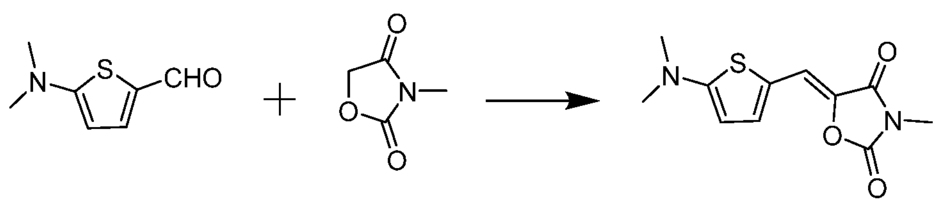

Compound 5 (I-5-7)

(I-5-7) was synthesized with reference to the synthetic method of (I-5-1), and the yield was 89.4%. 1HNMR (400 MHz, DMSO-d6) δ 12.09 (s, 1H), 7.81 (s, 1H), 7.43 (d, J=4.4 Hz, 1H), 6.15 (d, J=4.4 Hz, 1H), 3.34 (s, 3H),3.09 (s, 6H).

(I-5-8)

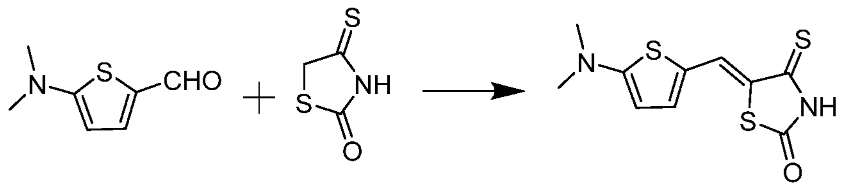

Compound 5 (I-5-8)

(I-5-8) was synthesized with reference to the synthetic method of (I-5-1), and the yield was 90.2%. 1HNMR (400 MHz, DMSO-d6) δ 14.08 (s, 1H), 7.51 (s, 1H), 7.43 (d, J=4.4 Hz, 1H), 6.15 (d, J=4.4 Hz, 1H), 3.09 (s, 6H).

(I-5-9)

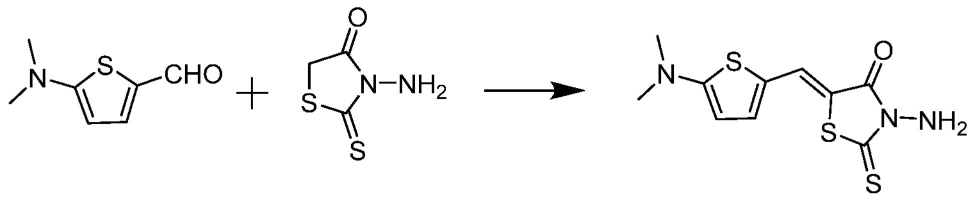

Compound 5 (I-5-9)

(I-5-9) was synthesized with reference to the synthetic method of (I-5-1), and the yield was 79.5%. 1H NMR (400 MHz, DMSO-d6) δ 13.01 (s, 1H), 7.71 (s, 1H), 7.43 (d, J=4.4 Hz, 1H), 6.15 (d, J=4.4 Hz, 1H), 3.09 (s, 6H).

(I-5-10)

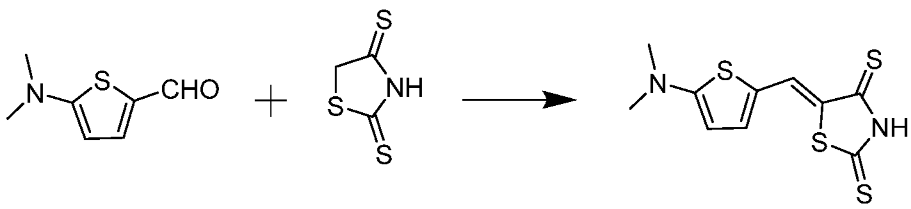

Compound 5 (I-5-10)

(I-5-10) was synthesized with reference to the synthetic method of (I-5-1), and the yield was 80.5%. 1H NMR (400 MHz, DMSO-d6) δ 15.01 (s, 1H), 7.91 (s, 1H), 7.44 (d, J=4.4 Hz, 1H), 6.17 (d, J=4.4 Hz, 1H), 3.09 (s, 6H).

(I-5-11)

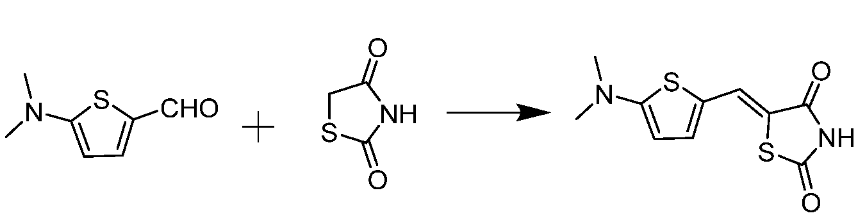

Compound 5 (I-5-11)

(I-5-11) was synthesized with reference to the synthetic method of (I-5-1), and the yield was 82.7%. 1H NMR

(400 MHz, DMSO-d6) δ 12.05 (s, 1H), 7.56 (s, 1H), 7.44 (d, J=4.4 Hz, 1H), 6.17 (d, J=4.4 Hz, 1H), 3.09 (s, 6H).

(I-5-12)

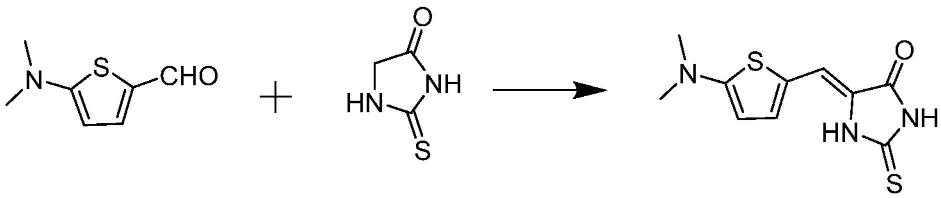

Compound 5 (I-5-12)

(I-5-12) was synthesized with reference to the synthetic method of (I-5-1), and the yield was 85.2%. 1H NMR (400 MHz, DMSO-d6) δ 13.65 (s, 1H), 7.66 (s, 1H), 7.44 (d, J=4.4 Hz, 1H), 6.17 (d, J=4.4 Hz, 1H), 3.09 (s, 6H).

(I-5-13)

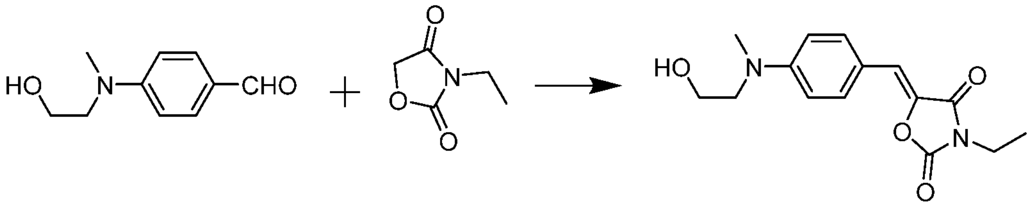

(I-5-13)

(I-5-13) was synthesized with reference to the synthetic method of (I-5-1), and the yield was 89.1%. 1H NMR (400 MHz, DMSO-d6) δ 12.14 (s, 1H), 7.59 (d, J=8.6 Hz, 2H), 6.77 (d, J=8.7 Hz, 2H), 6.61 (s, 1H), 4.74 (d, J=13.1 Hz, 1H), 4.24 (q, 2H), 3.56 (d, J=5.9 Hz, 2H), 3.47 (t, J=6.0 Hz, 2H), 3.01 (s, 3H), 1.20 (t, 3H).

(I-5-14)

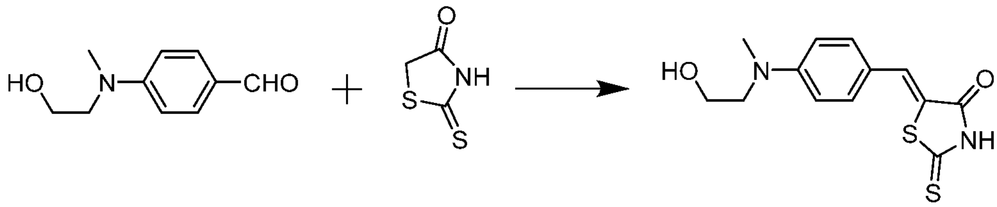

(I-5-14)

(I-5-14) was synthesized with reference to the synthetic method of (I-5-1), and the yield was 73.6%. 1H NMR (400 MHz, DMSO-d6) 13.14 (s, 1H), δ 7.67 (d, J=8.6 Hz, 2H), 6.90 (s, 1H), 6.80 (d, J=8.7 Hz, 2H), 4.75 (s, 1H), 3.57 (d, J=5.9 Hz, 2H), 3.51 (d, J=5.7 Hz, 2H), 3.04 (s, 3H).

(I-5-15)

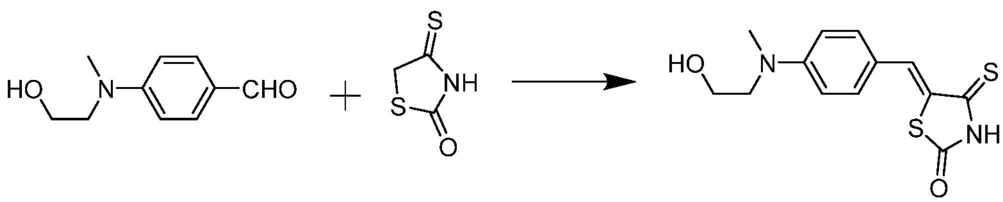

(I-5-15)

(I-5-15) was synthesized with reference to the synthetic method of (I-5-1), and the yield was 85.6%. 1H NMR

(400 MHz, DMSO-d6) 13.84 (s, 1H), δ 7.67 (d, J=8.6 Hz, 2H), 6.82 (s, 1H), 6.80 (d, J=8.7 Hz, 2H), 4.75 (s, 1H), 3.57 (d, J=5.9 Hz, 2H), 3.51 (d, J=5.7 Hz, 2H), 3.04 (s, 3H).

(I-5-16)

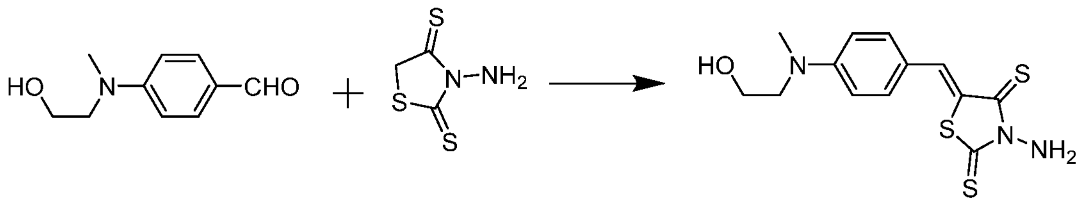

(I-5-16)

(I-5-16) was synthesized with reference to the synthetic method of (I-5-1), and the yield was 86.7%. 1H NMR (400 MHz, DMSO-d6) 19.94 (s, 1H), δ 7.67 (d, J=8.6 Hz, 2H), 6.96 (s, 1H), 6.80 (d, J=8.7 Hz, 2H), 4.75 (s, 1H), 3.57 (d, J=5.9 Hz, 2H), 3.51 (d, J=5.7 Hz, 2H), 3.04 (s, 3H).

(I-5-17)

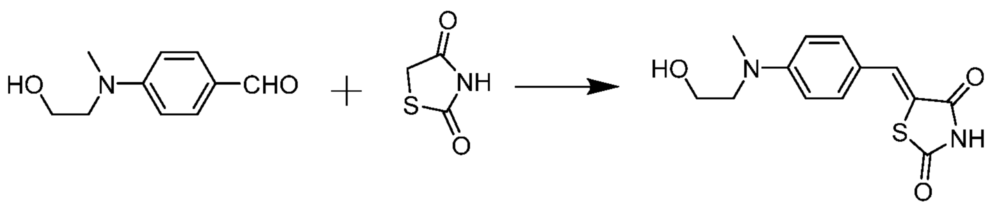

(I-5-17)

(I-5-17) was synthesized with reference to the synthetic method of (I-5-1), and the yield was 83.8%. 1H NMR (400 MHz, DMSO-d6) 12.11 (s, 1H), δ 7.67 (d, J=8.6 Hz, 2H), 7.23 (s, 1H), 6.80 (d, J=8.7 Hz, 2H), 4.75 (s, 1H), 3.57 (d, J=5.9 Hz, 2H), 3.51 (d, J=5.7 Hz, 2H), 3.04 (s, 3H).

(I-5-18)

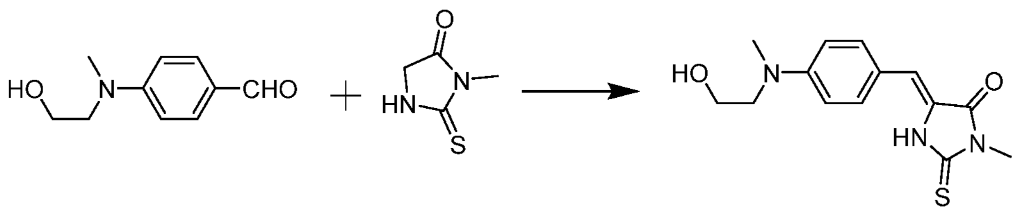

(I-5-18)

(I-5-18) was synthesized with reference to the synthetic method of (I-5-1), and the yield was 84.8%. 1H NMR (400 MHz, DMSO-d6) 12.12 (s, 1H), δ 7.67 (d, J=8.6 Hz, 2H), 6.93 (s, 1H), 6.80 (d, J=8.7 Hz, 2H), 4.75 (s, 1H), 3.57 (d, J=5.9 Hz, 2H), 3.51 (d, J=5.7 Hz, 2H), 3.04 (s, 3H).

(I-5-19)

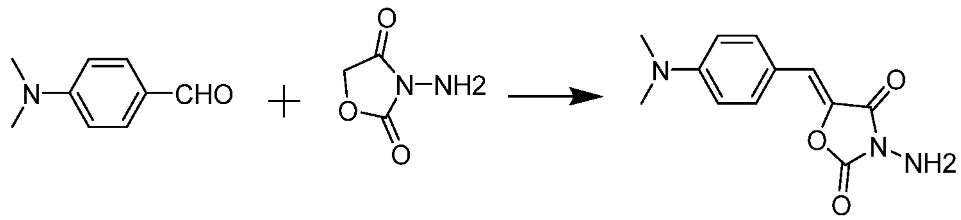

(I-5-19)

(I-5-19) was synthesized with reference to the synthetic method of (I-5-1), and the yield was 81.2%. 1H NMR (400 MHz, DMSO-d6) δ12.57 (s, 1H), 7.63 (s, 1H), 7.48-7.35 (m, 2H), 6.91-6.79 (m, 2H), 3.04 (s, 6H).

(I-5-20)

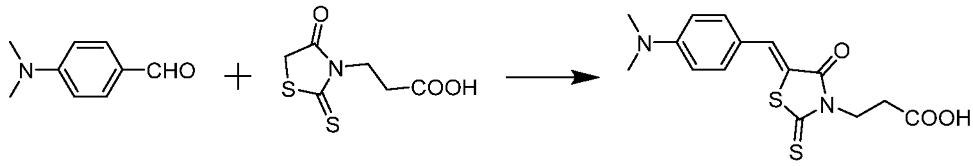

(I-5-20)

(I-5-20) was synthesized with reference to the synthetic method of (I-5-1), and the yield was 83.4%. 1H NMR (400 MHz, DMSO-d6) δ13.87 (s, 1H), 7.49 (s, 1H), 7.45-7.35 (m, 2H), 6.91-6.59 (m, 2H), 4.24 (t, J=5.28, 2H), 3.04 (s, 6H), 2.63 (t, J =5.28, 2H).

(I-5-21)

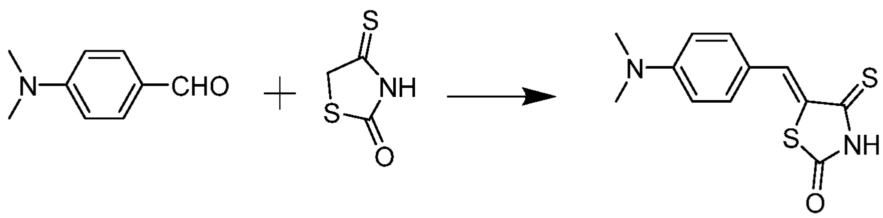

(I-5-21)

(I-5-21) was synthesized with reference to the synthetic method of (I-5-1), and the yield was 74.2%. 1H NMR (400 MHz, DMSO-d6) δ14.07 (s, 1H), 7.89 (s, 1H), 7.55-7.25 (m, 2H), 6.71-6.39 (m, 2H), 3.06 (s, 6H).

(I-5-22)

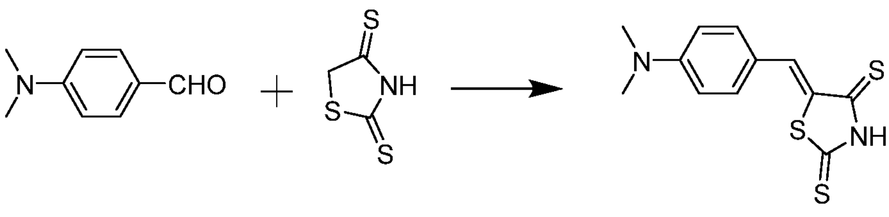

(I-5-22)

(I-5-22) was synthesized with reference to the synthetic method of (I-5-1), and the yield was 90.8%. 1H NMR (400 MHz, DMSO-d6) δ16.27 (s, 1H), 7.90 (s, 1H), 7.65-7.45 (m, 2H), 6.81-6.41 (m, 2H), 3.05 (s, 6H).

(I-5-23)

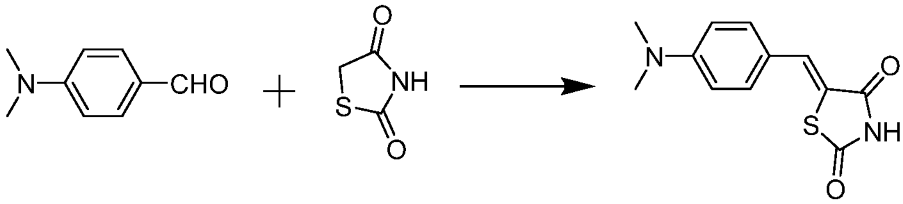

(I-5-23)

(I-5-23) was synthesized with reference to the synthetic method of (I-5-1), and the yield was 88.4%. 1H NMR (400 MHz, DMSO-d6) δ11.57 (s, 1H), 7.74 (s, 1H), 7.65-7.45 (m, 2H), 6.81-6.41 (m, 2H), 3.05 (s, 6H).

(I-5-24)

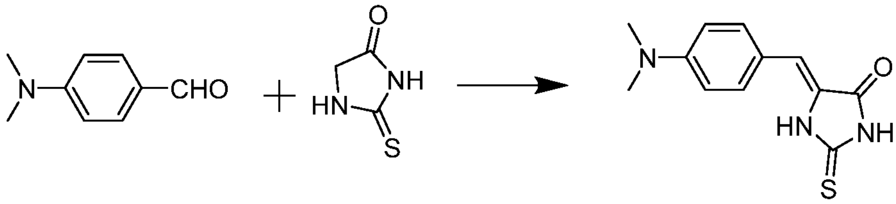

(I-5-24)

(I-5-24) was synthesized with reference to the synthetic method of (I-5-1), and the yield was 83.1%. 1H NMR (400 MHz, DMSO-d6) δ12.87 (s, 1H), 7.94 (s, 1H), 7.65-7.32 (m, 2H), 6.78-6.41 (m, 2H), 3.04 (s, 6H).

Compound 6

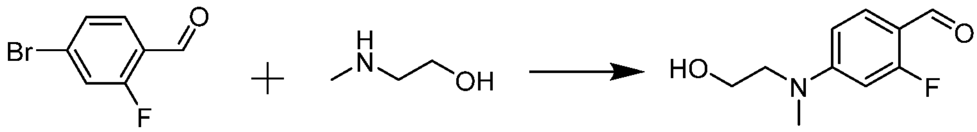

Compound 6

4-bromo-2-fluorobenzaldehyde (0.5 g, 2.5 mmol) dissolved into 15 mL of N-methyl-N-hydroxyethylamine, and then copper powder (6.4 mg, 0.01 mmol), copper iodide (19 mg, 0.01 mmol) and tripotassium phosphate (0.63 g, 2.96 mmol) were added; they were heated to 80°C by means of oil bath under Ar protection and were subject to reaction overnight; after the reaction, they were cooled to room temperature; the system was poured into 50 mL of water, and was extracted with dichloromethane for three times; the organic phase was combined and the solvent was dried by means of spinning and evaporation, as a result, 0.35 g of yellow product Compound 6 was obtained after column chromatography separation, and the yield was 77.8%. 1H NMR (400 MHz, DMSO-d6) 10.21 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H).

(I-5-25)

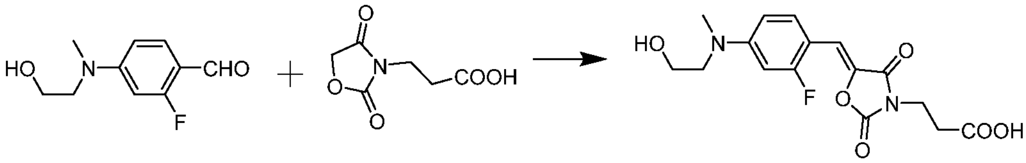

Compound 6 (I-5-25)

(I-5-25) was synthesized with reference to the synthetic method of (I-5-1), and the yield was 85.2%. 1H NMR (400 MHz, DMSO-d6) δ11.07 (s, 1H), 7.72 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H).

Compound 7

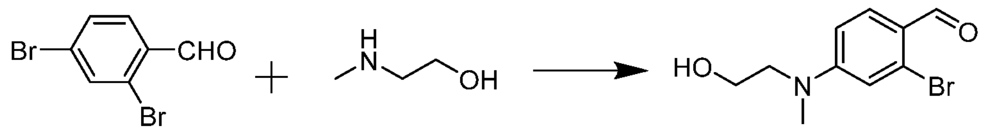

Compound 7

Compound 7 was synthesized with reference to the synthetic method of Compound 6, and the yield was 79.2%. 1H NMR (400 MHz, DMSO-d6) δ 10.21 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H).

Compound 8

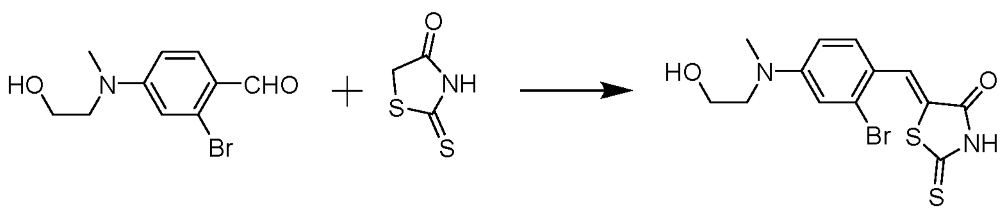

Compound 7 Compound 8

Compound 8 was synthesized with reference to the synthetic method of (I-5-1), and the yield was 88.8%. 1H NMR (400 MHz, DMSO-d6) δ12.27 (s, 1H), 7.62 (s, 1H), δ 7.57 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H).

(I-5-26)

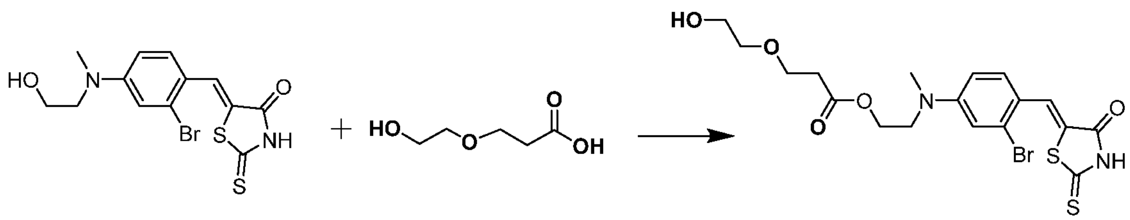

Compound 8 (I-5-26)

Compound 8 (0.5 g, 1.34 mmol) dissolved in 20 ml of DMF, and 3-(2-hydroxyethoxy) propionic acid (0.22 g, 1.64 mmol) and pybop (1.39 g, 2.67 mmol) were added; the reaction was conducted at room temperature and stirring was performed overnight, after complete reaction of the crude material Compound 8, dichloromethane was used for extraction for three times, organic phase was combined, and then the crude product was obtained after spinning and drying; 0.52 g of (I-5-26), which is a yellow solid, was obtained via silica gel column chromatography, and the yield was 78.8%. 1H NMR (400 MHz, DMSO-d6) δ12.27 (s, 1H), 7.62 (s, 1H), δ 7.57 (d, J=8.6 Hz, 2H), 6.93 (s, 1H), 4.55 (s, 1H), 3.73 (m, 6H), 3.57 (d, J=5.9 Hz, 2H), 3.51 (d, J=5.7 Hz, 2H), 3.02 (s, 3H), 2.42 (m, 2H).

Compound 9

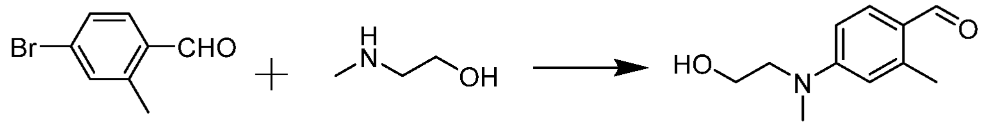

Compound 9

Compound 9 was synthesized with reference to the synthetic method of Compound 6, and the yield was 43.2%. 1H NMR (400 MHz, DMSO-d6) δ13.16 (s, 1H), 7.73 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H), 2.43 (s, 3H).

Compound 10

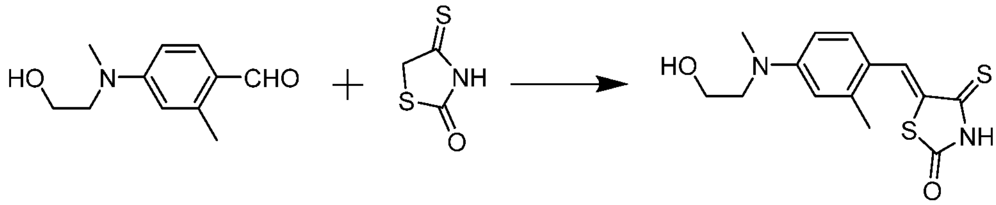

Compound 9 Compound 10

Compound 10 was synthesized with reference to the synthetic method of (I-5-1), and the yield was 80.1%. 1H NMR (400 MHz, DMSO-d6) δ13.16 (s, 1H), 7.73 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H), 2.43 (s, 3H).

Compound 11

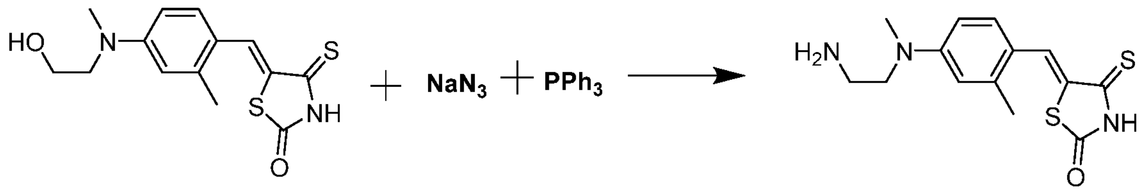


Compound 10 Compound 11

Compound 11 was synthesized with reference to the synthetic method of Compound 3, and the yield was 56.3%. 1H NMR (400 MHz, DMSO-d6) δ13.16 (s, 1H), 7.73 (s, 1H), δ 7.67 (d, J=8.6 Hz, 2H), 6.93 (s, 1H), 4.55 (s, 1H), 3.57 (d, J=5.9 Hz, 2H), 3.51 (d, J=5.7 Hz, 2H), 3.02 (s, 3H), 2.43 (s, 3H).

(I-5-27)

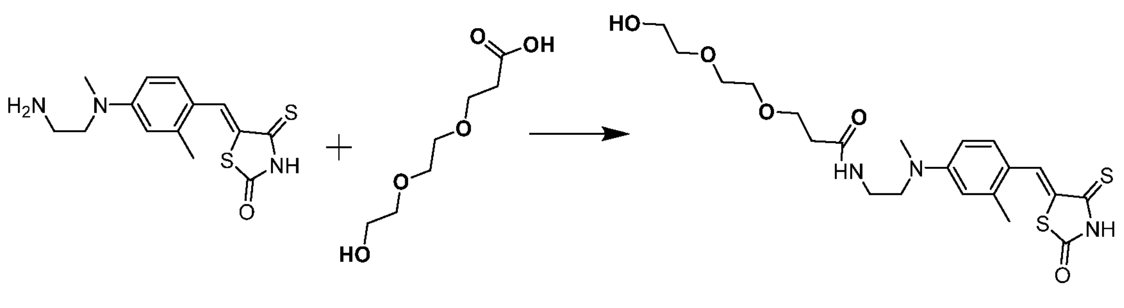

Compound 11 (I-5-27)

(I-5-27) was synthesized with reference to the synthetic method of (I-5-26), and 3the yield was 38.2%. 1H NMR

(400 MHz, DMSO-d6) δ13.16 (s, 1H), 7.73 (s, 1H), δ 7.67 (d, J=8.6 Hz, 2H), 6.93 (s, 1H), 4.55 (s, 1H), 3.70 (m, 2H), 3.57 (d, J=5.9 Hz, 2H), 3.51 (d, J=5.7 Hz, 2H), 3.48 (m, 6H), 3.02 (s, 3H), 2.43 (s, 3H), 2.35 (m, 2H).

(I-5-28)

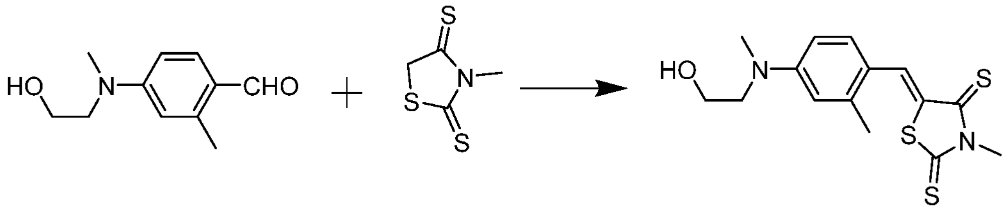

Compound 9 (I-5-28)

(I-5-28) was synthesized with reference to the synthetic method of (I-5-1), and the yield was 98.1%. 1H NMR (400 MHz, DMSO-d6) δ15.86 (s, 1H), 7.83 (s, 1H), δ 7.79 (d, J=8.6 Hz, 2H), 7.03 (s, 1H), 4.55 (s, 1H), 3.57 (d, J=5.9 Hz, 2H), 3.51 (d, J=5.7 Hz, 2H), 3.02 (s, 3H), 2.43 (s, 3H).

(I-5-29)

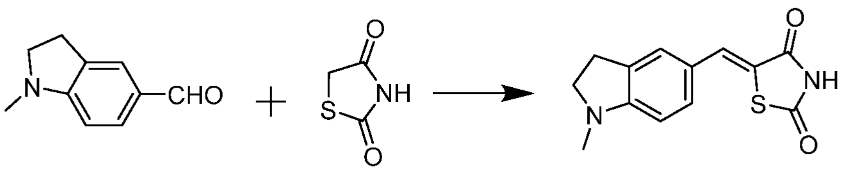

(I-5-29)

(I-5-29) was synthesized with reference to the synthetic method of (I-5-1), and the yield was 89.3%. 1H NMR (400 MHz, DMSO-d6) δ11.26 (s, 1H), 7.63 (s, 1H), δ 7.59 (d, J=8.6 Hz, 1H), 7.03 (d, J=8.6 Hz, 1H), 6.68 (s, 1H), 3.61 (t, J=5.3 Hz, 2H), 2.98 (t, J=5.3 Hz, 2H), 2.75 (s, 3H).

(I-5-30)

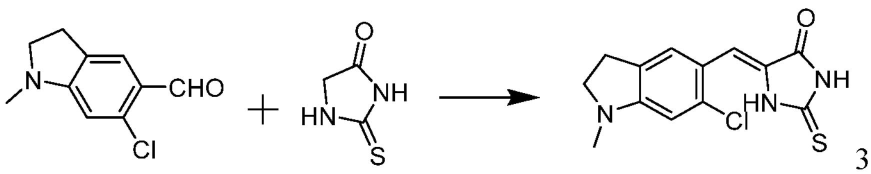

(I-5-30)

(I-5-30) was synthesized with reference to the synthetic method of (I-5-1), and the yield was 78.5%. 1H NMR (400 MHz, DMSO-d6) δ11.26 (s, 1H), 7.63 (s, 1H), δ 7.59 (s, 1H), 6.68 (s, 1H), 3.61 (t, J=5.3 Hz, 2H), 2.98 (t, J=5.3 Hz, 2H), 2.75 (s, 3H).

Compound 12

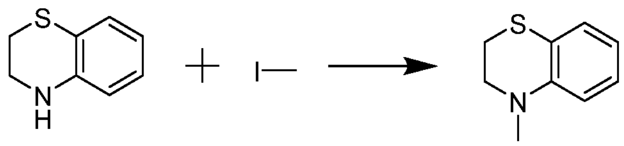

Compound 12

3,4-dihydro-2H-benzo[b][1,4]thiazine (0.30 g, 2 mmol) dissolved in 20 mL of DMF, and cesium carbonate (0.78 g, 2.4 mmol) and methyl iodide (0.31 g, 2.2 mmol) were added, they were heated to 65°C by means of oil bath under Ar protection and were subject to reaction for 4 h; after the reaction, they were cooled to room temperature; the system was poured into 50 mL of water, and was extracted with dichloromethane for three times; the organic phase was combined and the solvent was dried by means of spinning and evaporation, as a result, 0.29 g of product was obtained after column chromatography separation, and the yield was 90.6%. 1H NMR (400 MHz, DMSO-d6) δ 6.98-6.94 (m, 1H), 6.94-6.88 (m, 1H), 6.67 (dd, J=8.1, 1.2 Hz, 1H), 6.57 (td, J=7.5, 1.2 Hz, 1H), 3.57-3.42 (m, 2H), 3.13-3.00 (m, 2H), 2.87 (s, 3H).

Compound 13

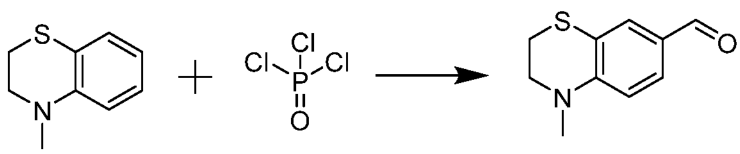

Compound 12 Compound 13

A three-mouth flask was added with 10 ml of DMF and was cooled for 5 min by means of ice bath; 0.2 ml of phosphorus oxychloride was added dropwise, and was stirred for 1 h under the condition of ice bath; Compound 4 dissolved into DMF, then the solution was added dropwise into the system and was stirred for 0.5 h under Ar protection and ice bath; the system slowly recovered to room temperature, and was stirred for another 5 h; after the reaction, saturated sodium carbonate solution was added for regulation till pH=10.0, and the stirring was performed overnight at room temperature; the organic phase was separated the next day, and the aqueous phase was extracted three times with 50 ml of dichloromethane for three times; the organic phase was combined and was washed twice with saturated salt solution; the organic phase was dried with anhydrous sodium sulfate, the solvent was dried by means of spinning and evaporation, and the residue was separated with column chromatography separation, as a result, 0.25 g of yellow solid was obtained, and the yield was75.3%. 1H NMR (400 MHz, DMSO-d6) δ10.11 (s, 1H), 6.98-6.94 (m, 1H), 6.94-6.88 (m, 1H), 6.57 (td, J=7.5, 1.2 Hz, 1H), 3.57-3.42 (m, 2H), 3.13-3.00 (m, 2H), 2.87 (s, 3H).

(I-5-31)

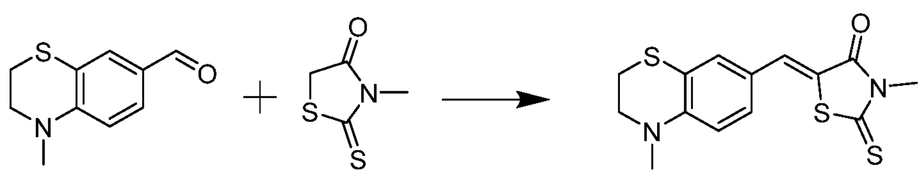

Compound 13 (I-5-31)

(1-5-31) was synthesized with reference to the synthetic method of (1-5-1), and the yield was 89.8%. 1H NMR (400 MHz, DMSO-d6) δ 13.61 (s, 1H), 7.46 (s, 1H), 7.28-7.15 (m, 2H), 6.82 (d, J=8.7 Hz, 1H), 3.76-3.57 (m, 5H), 3.13-3.05 (m, 2H), 3.03 (s, 3H).

(I-5-32)

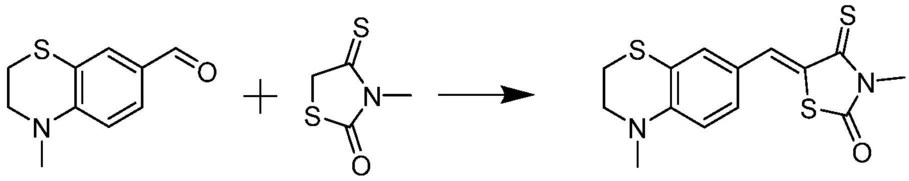

Compound 13 (I-5-32)

(I-5-32) was synthesized with reference to the synthetic method of (I-5-1), and the yield was 69.5%. 1H NMR (400 MHz, DMSO-d6) δ 14.21 (s, 1H), 7.58 (s, 1H), 7.38-7.15 (m, 2H), 6.82 (d, J=8.7 Hz, 1H), 3.76-3.57 (m, 2H), 3.13-3.05 (m, 2H), 3.53 (s, 3H), 3.03 (s, 3H).

(I-5-33)

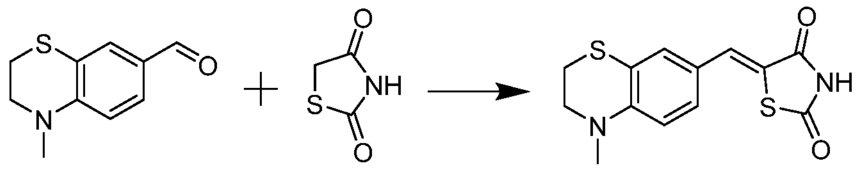

Compound 13 (I-5-33)

(I-5-33) was synthesized with reference to the synthetic method of (I-5-1), and the yield was 85.8%. 1HNMR (400 MHz, DMSO-d6) δ 11.75 (s, 1H), 7.42 (s, 1H), 7.38-7.15 (m, 2H), 6.82 (d, J=8.7 Hz, 1H), 3.76-3.57 (m, 2H), 3.13-3.05 (m, 2H), 3.03 (s, 3H).

(I-5-34)

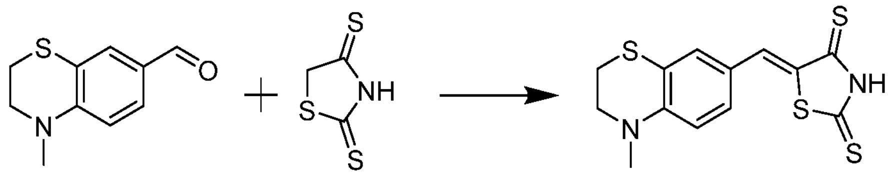

Compound 13 (I-5-34)

(I-5-34) was synthesized with reference to the synthetic method of (I-5-1), and the yield was 99.2%. 1H NMR (400 MHz, DMSO-d6) δ 16.05 (s, 1H), 7.51 (s, 1H), 7.38-7.15 (m, 2H), 6.82 (d, J=8.7 Hz, 1H), 3.76-3.57 (m, 2H), 3.13-3.05 (m, 2H), 3.03 (s, 3H).

(I-5-35)

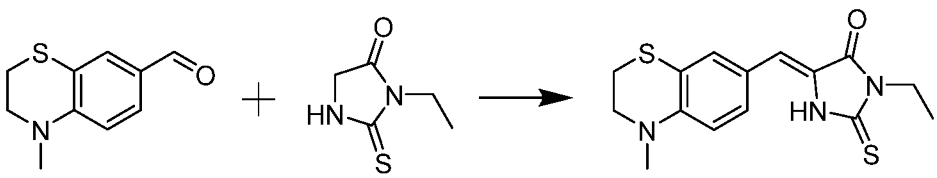

Compound 13 (I-5-35)

(I-5-35) was synthesized with reference to the synthetic method of (I-5-1), and the yield was 75.3%. 1H NMR

(400 MHz, DMSO-d6) δ 12.17 (s, 1H), 7.46 (s, 1H), 7.38-7.15 (m, 2H), 6.82 (d, J=8.7 Hz, 1H), 4.07 (q, J=5.5Hz, 3H), 3.76-3.57 (m, 2H), 3.13-3.05 (m, 2H), 3.03 (s, 3H), 1.20 (t, J=5.5Hz, 3H).

Compound 14

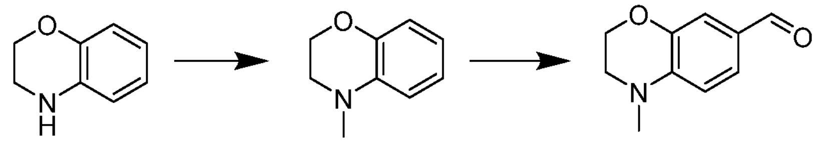

Compound 14

Compound 14 was synthesized with reference to the synthetic method of Compound 13, and the yield was 58.5%. 1H NMR (400 MHz, DMSO-d6) δ10.26(s, 1H), 6.98-6.94 (m, 1H), 6.94-6.88 (m, 1H), 6.57 (td, J=7.5, 1.2 Hz, 1H), 3.38-3.32 (m, 2H), 3.05-2.80 (m, 2H), 2.69 (s, 3H).

(I-5-36)

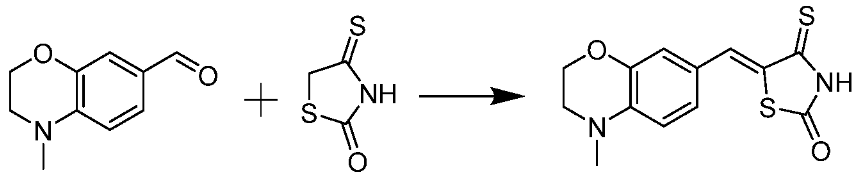

Compound 14 (I-5-36)

(I-5-36) was synthesized with reference to the synthetic method of (I-5-1), and the yield was 69.3%. 1H NMR (400 MHz, DMSO-d6) δ 14.52 (s, 1H), 7.44 (s, 1H), 7.38-7.15 (m, 2H), 6.82 (d, J=8.7 Hz, 1H), 3.56-3.47 (m, 2H), 3.23-3.05 (m, 2H), 3.03 (s, 3H).

(I-5-37)

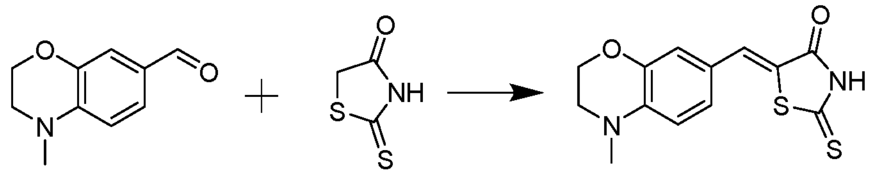

Compound 14 (I-5-37)

(I-5-37) was synthesized with reference to the synthetic method of (I-5-1), and the yield was 89.9%. 1H NMR (400 MHz, DMSO-d6) δ 13.42 (s, 1H), 7.46 (s, 1H), 7.38-7.15 (m, 2H), 6.82 (d, J=8.7 Hz, 1H), 3.56-3.47 (m, 2H), 3.23-3.05 (m, 2H), 3.03 (s, 3H).

(I-5-38)

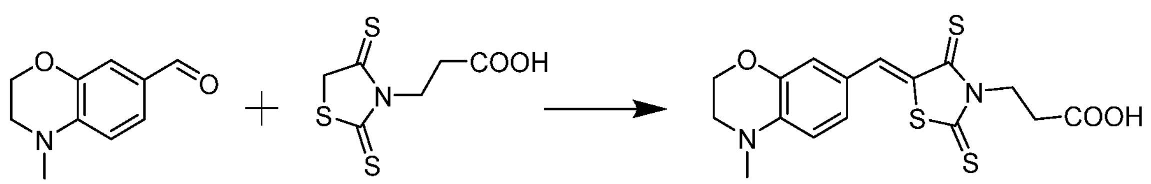

Compound 14 (I-5-38)

(I-5-38) was synthesized with reference to the synthetic method of (I-5-1), and the yield was 80.1%. 1H NMR (400 MHz, DMSO-d6) δ 16.72 (s, 1H), 7.56 (s, 1H), 7.48-7.15 (m, 2H), 6.82 (d, J=8.7 Hz, 1H), 4.40 (t, J=5.1Hz, 2H), 3.56-3.47 (m, 2H), 3.23-3.05 (m, 2H), 3.03 (s, 3H), 2.61 (t, J=5.1 Hz, 2H).

(I-5-39)

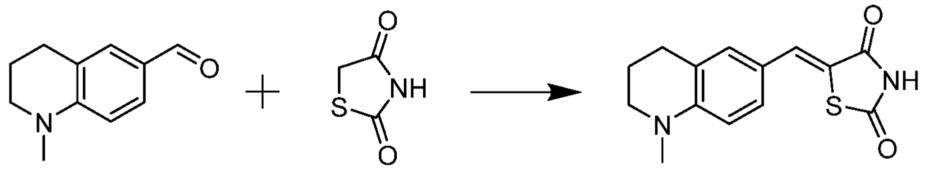

(I-5-39)

(I-5-39) was synthesized with reference to the synthetic method of (I-5-1), and the yield was 85.8%. 1HNMR (400 MHz, DMSO-d6) δ 11.54 (s, 1H), 7.26 (s, 1H), 7.24-7.15 (m, 2H), 6.82 (d, J=8.7 Hz, 1H), 3.56-3.47 (m, 2H), 3.23-3.05 (m, 2H), 3.03 (s, 3H), 2.79 (m, 2H).

(I-5-40)

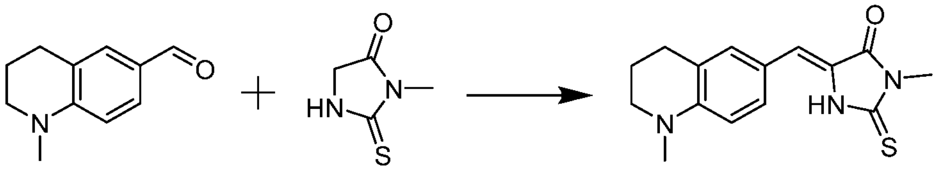

(I-5-40)

(I-5-40) was synthesized with reference to the synthetic method of (I-5-1), and the yield was 76.1%. 1H NMR (400 MHz, DMSO-d6) δ 12.78 (s, 1H), 7.26 (s, 1H), 7.24-7.15 (m, 2H), 6.82 (d, J=8.7 Hz, 1H), 3.59 (s, 3H), 3.56-3.47 (m, 2H), 3.23-3.05 (m, 2H), 3.03 (s, 3H), 2.79 (m, 2H).

Compound 15

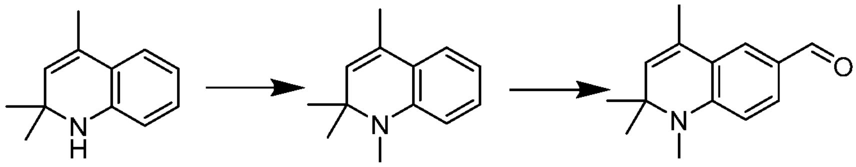

Compound 15

Compound 15 was synthesized with reference to the synthetic method of Compound 13, and the yield was 69.7%. 1H NMR (400 MHz, DMSO-d6) δ 10.21 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 2.87 (s, 3H), 1.96 (d, J=1.4 Hz, 3H), 1.34 (s, 6H).

(I-5-41)

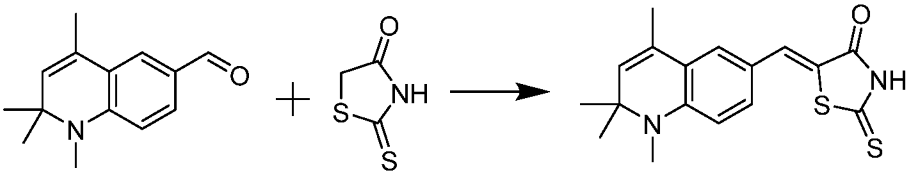

Compound 15 (I-5-41)

(I-5-41) was synthesized with reference to the synthetic method of (I-5-1), and the yield was 95.1%. 1HNMR (400 MHz, DMSO-d6) δ 13.57 (s, 1H), 7.51 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 2.87 (s, 3H), 1.96 (d, J=1.4 Hz, 3H), 1.34 (s, 6H).

(I-5-42)

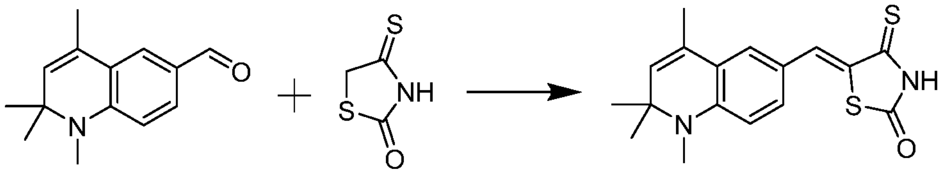

Compound 15

(I-5-42) was synthesized with reference to the synthetic method of (I-5-1), and the yield was 74.6%. 1H NMR (400 MHz, DMSO-d6) δ 13.86 (s, 1H), 7.41 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 2.87 (s, 3H), 1.96 (d, J=1.4 Hz, 3H), 1.34 (s, 6H).

(I-5-43)

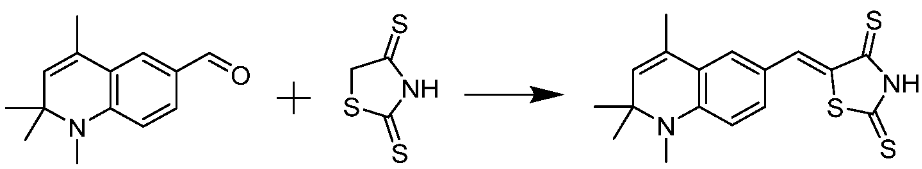

Compound 15

(I-5-43) was synthesized with reference to the synthetic method of (I-5-1), and the yield was 93.2%. 1H NMR (400 MHz, DMSO-d6) δ 15.72 (s, 1H), 7.65 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 2.87 (s, 3H), 1.96 (d, J=1.4 Hz, 3H), 1.34 (s, 6H).

(I-5-44)

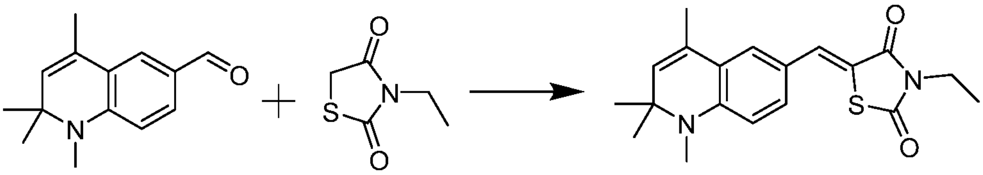

Compound 15

(I-5-44) was synthesized with reference to the synthetic method of (I-5-1), and the yield was 75.9%. 1H NMR (400 MHz, DMSO-d6) δ 11.02 (s, 1H), 7.35 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 4.07 (q, J=5.4Hz, 2H), 2.87 (s, 3H), 1.96 (d, J=1.4 Hz, 3H), 1.3 4 (s, 6H) 1.20 (t, J=5.4Hz, 2H).

(I-5-45)

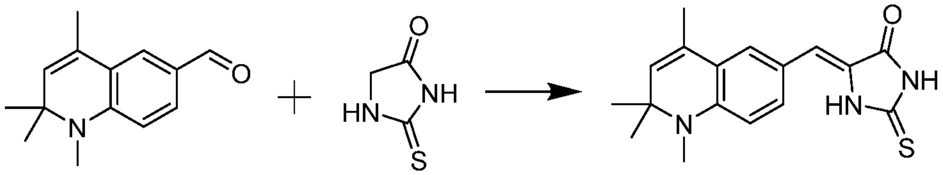

Compound 15 (I-5-45)

(I-5-45) was synthesized with reference to the synthetic method of (I-5-1), and the yield was 78.9%. 1H NMR (400 MHz, DMSO-d6) δ 15.72 (s, 1H), 7.48 (s, 1H), 7.33 (dd, J=8.8, 2.2 Hz, 1H), 7.15 (d, J=2.2 Hz, 1H), 6.63 (d, J=8.8 Hz, 1H), 5.47 (d, J=1.5 Hz, 1H), 2.87 (s, 3H), 1.96 (d, J=1.4 Hz, 3H), 1.34 (s, 6H).

Compound 16

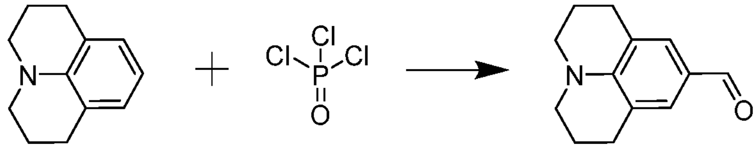

Compound 16

Compound 16 was synthesized with reference to the synthetic method of Compound 13, and the yield was 89.4%. 1H NMR (400 MHz, DMSO-d6) δ 10.25 (s, 1H), 6.95 (s, 2H), 3.25 (dd, J=6.6, 4.9 Hz, 4H), 2.68 (t, J=6.3 Hz, 4H), 2.05-1.57 (m, 4H).

(I-5-46)

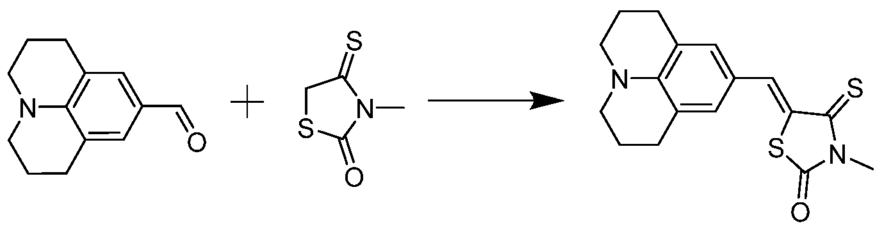

Compound 16 (I-5-46)

(I-5-46) was synthesized with reference to the synthetic method of (I-5-1), and the yield was 72.7%. 1H NMR (400 MHz, DMSO-d6) δ 12.74 (s, 1H), 7.61 (s, 1H), 6.95 (s, 2H), 3.59 (s, 3H), 3.25 (dd, J=6.6, 4.9 Hz, 4H), 2.68 (t, J=6.3 Hz, 4H), 2.05-1.57 (m, 4H).

(I-5-47)

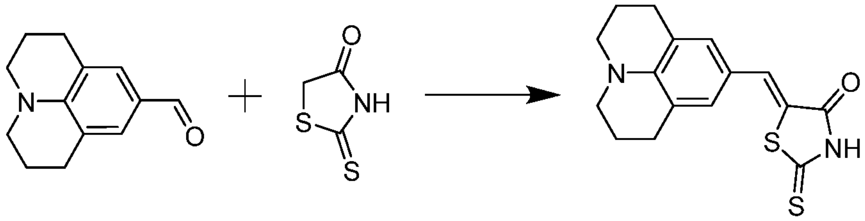

Compound 16 (I-5-47)

(I-5-47) was synthesized with reference to the synthetic method of (I-5-1), and the yield was 86.7%. 1H NMR (400 MHz, DMSO-d6) δ 12.58 (s, 1H), 7.46 (s, 1H), 6.95 (s, 2H), 3.25 (dd, J=6.6, 4.9 Hz, 4H), 2.68 (t, J=6.3 Hz, 4H), 2.05-1.57 (m, 4H).

(I-5-48)

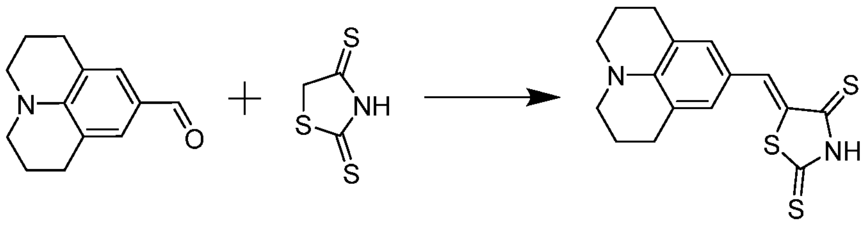

Compound 16 (I-5-48)

(I-5-48) was synthesized with reference to the synthetic method of (I-5-1), and the yield was 92.3%. 1H NMR (400 MHz, DMSO-d6) δ 15.79 (s, 1H), 7.38 (s, 1H), 6.95 (s, 2H), 3.25 (dd, J=6.6, 4.9 Hz, 4H), 2.68 (t, J=6.3 Hz, 4H), 2.05-1.57 (m, 4H).

(I-5-49)

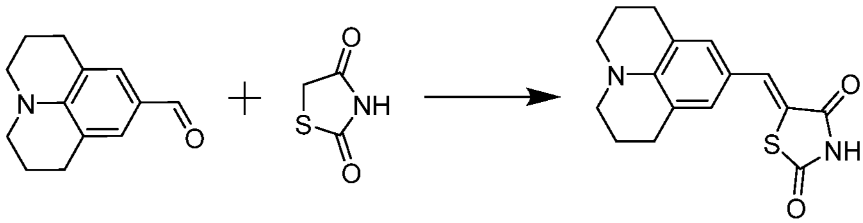

Compound 16 (I-5-49)

(I-5-49) was synthesized with reference to the synthetic method of (I-5-1), and the yield was 78.3%. 1H NMR (400 MHz, DMSO-d6) δ 11.23 (s, 1H), 7.35 (s, 1H), 6.95 (s, 2H), 3.25 (dd, J=6.6, 4.9 Hz, 4H), 2.68 (t, J=6.3 Hz, 4H), 2.05-1.57 (m, 4H).

(I-5-50)

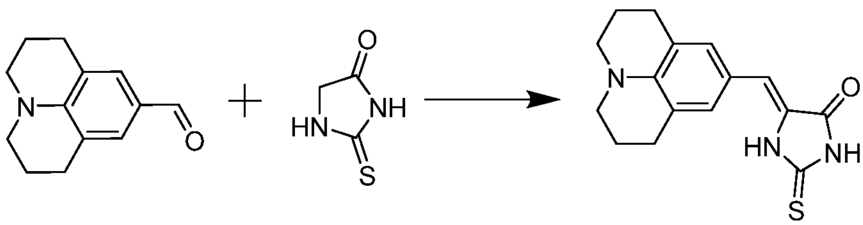

Compound 16 (I-5-50)

(I-5-50) was synthesized with reference to the synthetic method of (I-5-1), and the yield was 71.2%. 1H NMR (400 MHz, DMSO-d6) δ 12.21 (s, 1H), 7.31 (s, 1H), 6.95 (s, 2H), 3.25 (dd, J=6.6, 4.9 Hz, 4H), 2.68 (t, J=6.3 Hz, 4H), 2.05-1.57 (m, 4H).

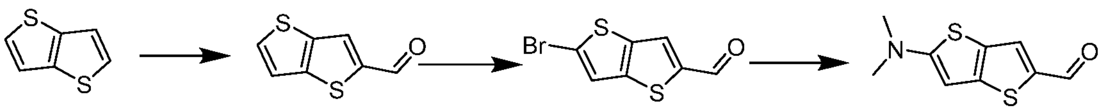

Compound 17 Compound 18 Compound 19

To a three-mouth flask of 250 ml, thieno[3,2-b]thiophene (10 g, 71.31 mmol) was added and 120 ml of anhydrous tetrahydrofuran was added for dissolution, and then the three-mouth flask was placed at -78°C to be cooled; 49 ml of 1.6M n-butyllithium was added dropwise to the system; the temperature of -78°C was kept for 2h, then the refrigeration was turned off, and the temperature slowly recovered to room temperature; the stirring was performed overnight, and 40 ml of water was added under ice bath after the reaction so as to quench the reaction; the system was poured into 200 ml of dichloromethane and was extracted, the aqueous phase was extracted with dichloromethane for three times, the organic phase was dried with anhydrous sodium sulfate, the solvent was dried by means of spinning, and the residue was separated with column chromatography separation, as a result, 10.7 g of compound was obtained, and the yield was 90%. 1H NMR (400 MHz, Chloroform-d) δ 9.97 (s, 1H), 7.94 (s, 1H), 7.70 (d, J=5.3 Hz, 1H), 7.33 (d, J=5.3 Hz, 1H).

Compound 18

Compound 9 (10 g, 59.5 mmol) dissolved in 120 ml of mixed solution of dry DMF and acetic acid (1:1), NBS (11.66 g, 65.5 mmol) was added, and was heated to 120°Cby means of oil bath under Ar protection overnight for refluxing; after the reaction, extraction was performed for three times with ethyl acetate and water, the organic phase was combined and dried, and then the solvent was dried by means of spinning, as a result, 11.2 g of product was obtained after the residual was subject to column chromatography, and the yield was 78%. 1H NMR (400 MHz, Chloroform-d) δ 10.01 (s, 1H), 7.70 (d, J=5.3 Hz, 1H), 7.33 (d, J=5.3 Hz, 1H).

Compound 19

Compound 19 was synthesized with reference to the synthetic method of Compound 1, and the yield was 85%. 1H NMR (400 MHz, DMSO-d6) δ 10.15 (s, 1H), 7.87 (s, 1H), 6.39 (s, 1H), 3.09 (s, 6H).

(I-5-51)

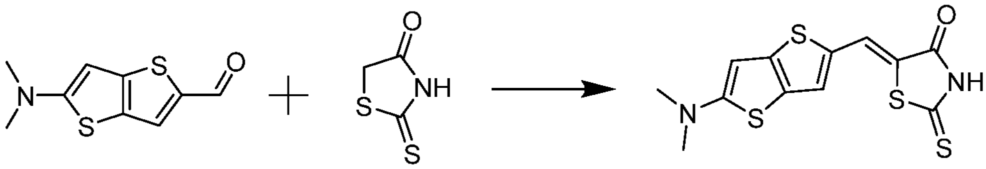

Compound 19 (I-5-51)

(I-5-51) was synthesized with reference to the synthetic method of (I-5-1), and the yield was 90.2%. 1H NMR (400 MHz, DMSO-d6) δ 12.25 (s, 1H), 8.017 (s, 1H), 87 (s, 1H), 6.39 (s, 1H), 3.09 (s, 6H).

(I-5-52)

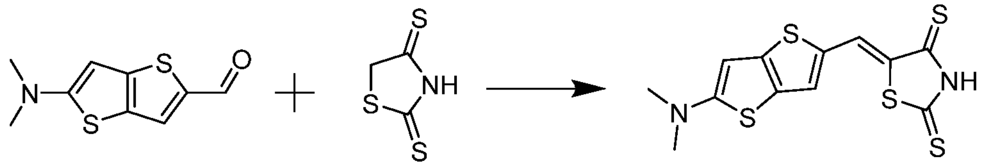

Compound 19 (I-5-52)

(I-5-52) was synthesized with reference to the synthetic method of (I-5-1), and the yield was 96.1%. 1H NMR (400 MHz, DMSO-d6) δ 15.35 (s, 1H), 7.91 (s, 1H), 7.87 (s, 1H), 6.59 (s, 1H), 3.09 (s, 6H).

(I-5-53)

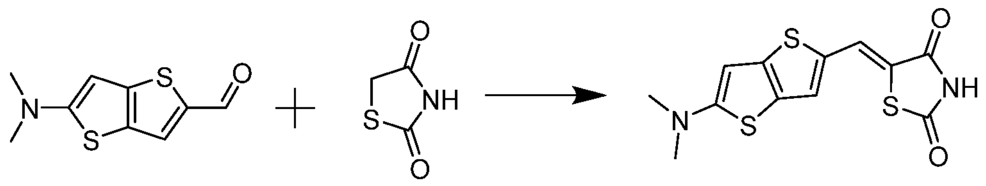

Compound 19 (I-5-53)

(I-5-53) was synthesized with reference to the synthetic method of (I-5-1), and the yield was 71.3%. 1H NMR (400 MHz, DMSO-d6) δ 11.35 (s, 1H), 7.96 (s, 1H), 7.57 (s, 1H), 6.59 (s, 1H), 3.09 (s, 6H).

(I-5-54)

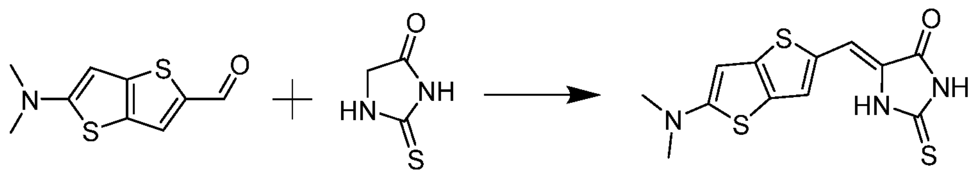

Compound 19 (I-5-54)

(I-5-54) was synthesized with reference to the synthetic method of (I-5-1), and the yield was 78.5%. 1H NMR (400 MHz, DMSO-d6) δ 11.35 (s, 1H), 7.64 (s, 1H), 7.57 (s, 1H), 6.59 (s, 1H), 3.09 (s, 6H).

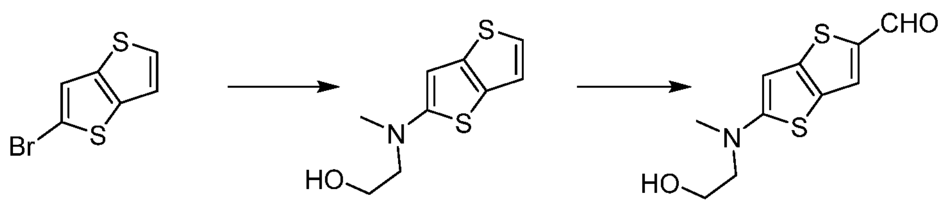

Compound 20 Compound 21

2-bromothieno[3,2-b]thiophene (0.438 g, 2 mmol) dissolved into 15 mL of N-methyl-N-hydroxyethylamine, and then copper powder (6.4 mg, 0.01 mmol), copper iodide (19 mg, 0.01 mmol) and tripotassium phosphate (0.850 g, 4 mmol) were added; they were heated to 80°C by means of oil bath under Ar atmosphere and were subject to reaction overnight; after the reaction, they were cooled to room temperature; the system was poured into 50 mL of water, and was extracted with dichloromethane for three times, 50 mL each time; the organic phase was combined and the solvent was dried by means of spinning and evaporation, as a result, 0.362 g of yellow product was obtained after column chromatography separation, and the yield was 85%. 1H-NMR (400 MHz, CDCl3): δ=7.92 (s, 1H), 7.63 (d, 1 H, J=5.2 Hz), 7.31 (d, 1 H, J=5.2 Hz), 3.85 (t, 2H, J=5.6 Hz), 3.60 (t, 2 H, J=5.6 Hz), 3.10 (s, 3H).

Compound 21

Compound 20 (0.426 g, 2 mmol) dissolved into 50 ml of anhydrous dichloromethane, 1 ml of triethylamine was added, and acetic oxide (0.3 ml, 3 mmol) was slowly and dropwise added under the condition of ice bath; after dropwise adding, the system slowly recovered to room temperature, and was stirred for 3 h; after the reaction, 100 ml of water was added, organic phase was separated, and aqueous phase was extracted twice with 50 ml of dichloromethane; the organic phase was combined and was dried with anhydrous sodium sulfate, and the solvent was dried by means of spinning and evaporation; and the residue needed no further purification and was directly used in the next step.

The above-mentioned residue was dissolved into 50 ml of dichloromethane, 5 ml of dimethylformamide was added, 2 ml of phosphorus oxychloride was added under the condition of ice bath, and stirring was performed under Ar atmosphere for 0.5 h; the system slowly recovered to room temperature, and was stirred for another 5 h; after the

reaction, saturated sodium carbonate solution was added for regulation till pH=10.0, and the stirring was performed overnight at room temperature; the organic phase was separated the next day, and the aqueous phase was extracted with 50 ml of dichloromethane for three times; the organic phase was combined and was washed twice with saturated salt solution; the organic phase was dried with anhydrous sodium sulfate, the solvent was dried by means of spinning and evaporation, and the residue was separated with column chromatography separation, as a result, 0.285 g of yellow solid was obtained, and the yield was 59%. 1H-NMR (400 MHz, CDCl3): δ=10.01 (s, 1H), 7.92 (s, 1H), 7.63 (s, 1H), 3.85 (t, 2H, J=5.6 Hz), 3.60 (t, 2 H, J=5.6 Hz), 3.10 (s, 3H).

(I-5-55)

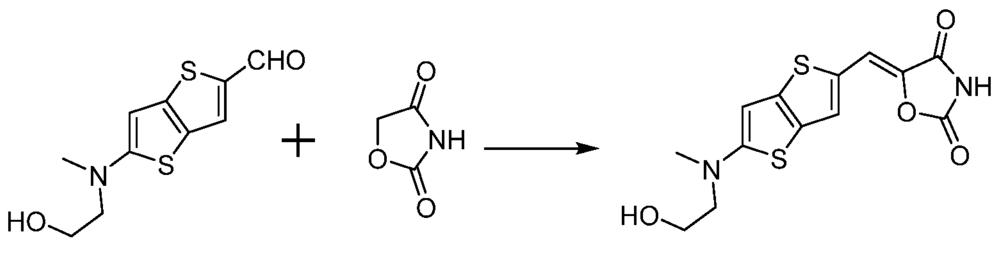

Compound 21 (I-5-55)

(I-5-55) was synthesized with reference to the synthetic method of (I-5-1), and the yield was 58.1%. 1H-NMR (400 MHz, CDCl3): δ=12.31 (s, 1H), 7.92 (s, 1H), 7.63 (s, 1H), 6.96 (s, 1H), 3.85 (t, 2H, J=5.6 Hz), 3.60 (t, 2 H, J=5.6 Hz), 3.10 (s, 3H).

(I-5-56)

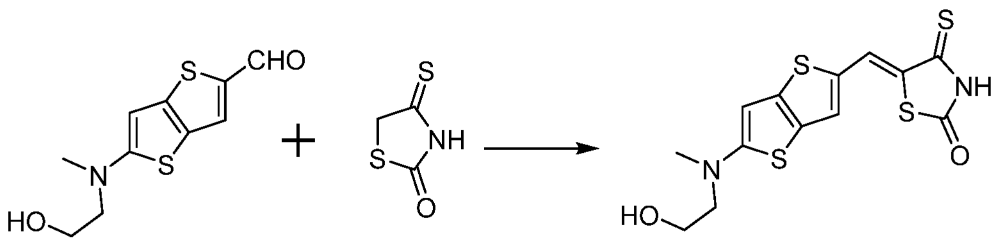

Compound 21 (I-5-56)

(I-5-56) was synthesized with reference to the synthetic method of (I-5-1), and the yield was 70.1%. 1H-NMR (400 MHz, CDCl3): δ=13.56 (s, 1H), 7.92 (s, 1H), 7.63 (s, 1H), 6.96 (s, 1H), 3.85 (t, 2H, J=5.6 Hz), 3.60 (t, 2 H, J=5.6 Hz), 3.10 (s, 3H).

(I-5-57)

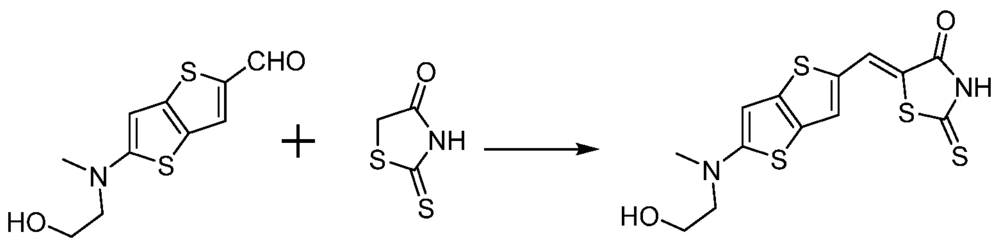

Compound 21 (I-5-57)

(I-5-57) was synthesized with reference to the synthetic method of (I-5-1), and the yield was 86.1%. 1H-NMR (400 MHz, CDCl3): δ=13.45 (s, 1H), 7.92 (s, 1H), 7.63 (s, 1H), 6.91 (s, 1H), 3.85 (t, 2H, J=5.6 Hz), 3.60 (t, 2 H, J=5.6 Hz), 3.10 (s, 3H).

(I-5-58)

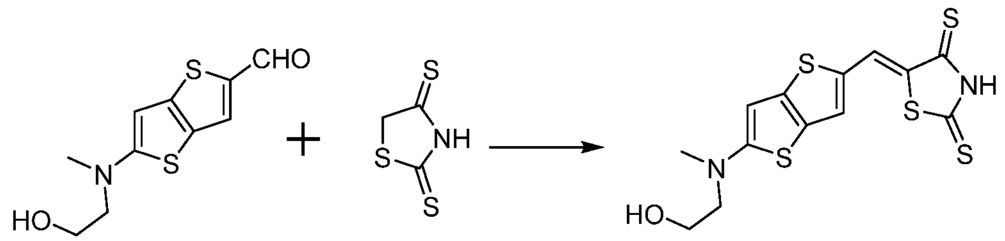

Compound 21 (I-5-58)

(I-5-58) was synthesized with reference to the synthetic method of (I-5-1), and the yield was 96.8%. 1H-NMR (400 MHz, CDCl3): δ=17.23 (s, 1H), 7.92 (s, 1H), 7.63 (s, 1H), 6.91 (s, 1H), 3.85 (t, 2H, J=5.6 Hz), 3.60 (t, 2 H, J=5.6 Hz), 3.10 (s, 3H).

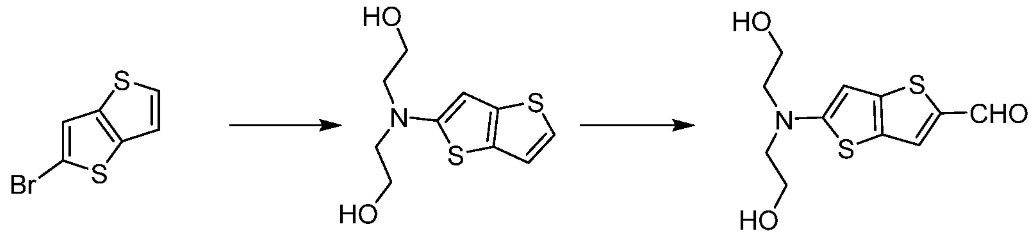

Compound 22

Compound 22 was synthesized with reference to the synthetic method of Compound 21, and the yield was 52.1%. 1H-NMR (400 MHz, CDCl3): δ=10.01 (s, 1H), 7.92 (s, 1H), 7.63 (s, 1H), 3.85 (t, J=5.6 Hz, 4H), 3.60 (t, J=5.6 Hz, 4H).

(I-5-59)

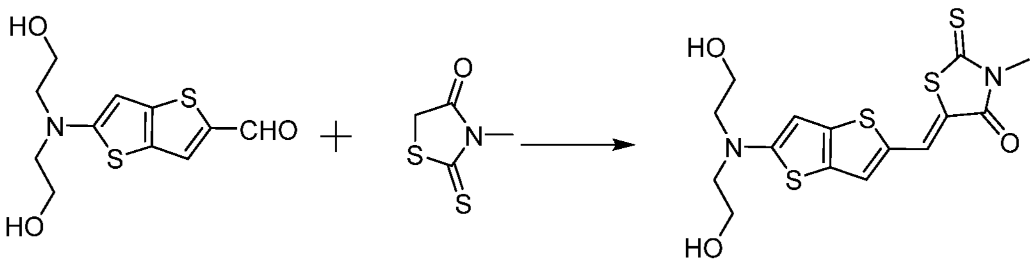

Compound 22 (I-5-59)

(I-5-59) was synthesized with reference to the synthetic method of (I-5-1), and the yield was 86.1%. 1H-NMR (400 MHz, CDCl3): δ=7.92 (s, 1H), 7.63 (s, 1H), 6.91 (s, 1H), 3.85 (t, J=5.6 Hz, 4H), 3.60 (t, J=5.6 Hz, 4H), 3.59 (s, 3H).

(I-5-60)

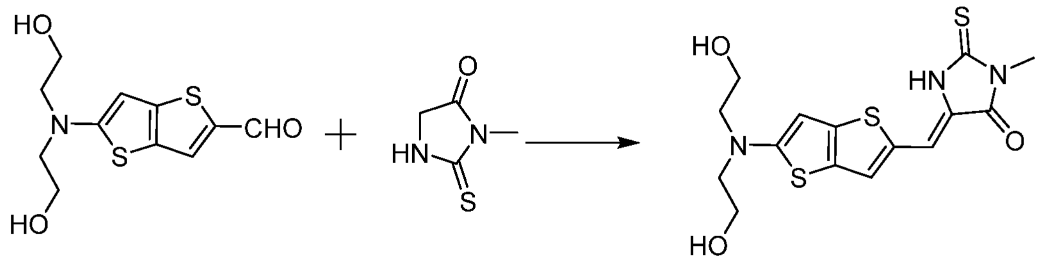

Compound 22 (I-5-60)

(I-5-60) was synthesized with reference to the synthetic method of (I-5-1), and the yield was 88.6%. 1H-NMR (400 MHz, CDCl3): δ=7.92 (s, 1H), 7.63 (s, 1H), 6.65 (s, 1H), 3.85 (t, J=5.6 Hz, 4H), 3.60 (t, J=5.6 Hz, 4H), 3.45 (s, 3H).

Compound 23

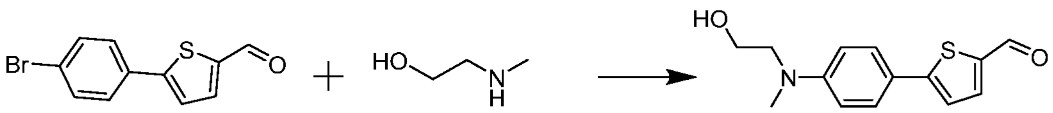

Compound 23

Compound 23 was synthesized with reference to the synthetic method of Compound 19, and the yield was 82.3%. 1H-NMR (400 MHz, CDCl3): δ=9.81 (s, 1H), 7.68 (d, 1H, J=7.88 Hz), 7.55 (d, 1H, J=7.88 Hz), 7.25 (d, 2H, J=8.00 Hz), 6.78 (d, 2H, J=8.00 Hz), 3.86 (t, 2H, J=4.80 Hz), 3.56 (t, 2H, J=4.80 Hz), 3.06 (s, 3H).

(I-5-61)

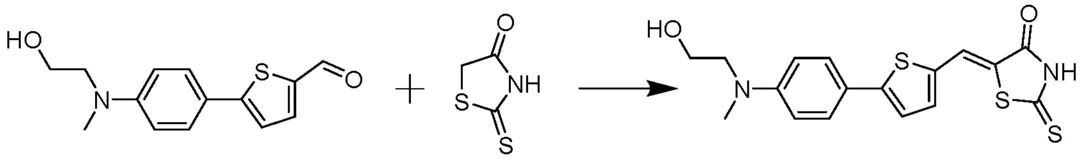

Compound 23 (I-5-61)

(I-5-61) was synthesized with reference to the synthetic method of (I-5-1), and the yield was 84.1%. 1H-NMR (400 MHz, CDCl3): δ=13.21 (s, 1H), 7.68 (d, 1H, J=7.88 Hz), 7.55 (d, 1H, J=7.88 Hz), 7.25 (d, 2H, J=8.00 Hz), 6.80 (s, 1H), 6.78 (d, 2H, J=8.00 Hz), 3.86 (t, 2H, J=4.80 Hz), 3.56 (t, 2H, J=4.80 Hz), 3.06 (s, 3H).

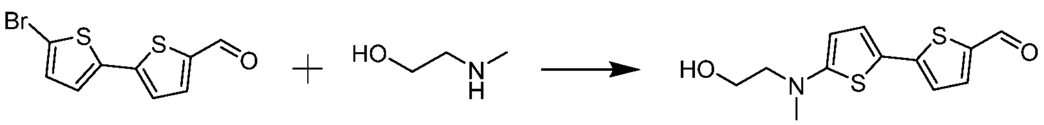

Compound 24

Compound 24 was synthesized with reference to the synthetic method of Compound 19, and the yield was 59.3%. 1H-NMR (400 MHz, CDCl3): δ=10.01 (s, 1H), 7.68 (d, 1H, J=7.88 Hz), 7.55 (d, 1H, J=7.88 Hz), 7.25 (d, 1H, J=7.65 Hz), 6.78 (d, 1H, J=7.65 Hz), 3.86 (t, 2H, J=4.80 Hz), 3.56 (t, 2H, J=4.80 Hz), 3.06 (s, 3H).

(I-5-62)

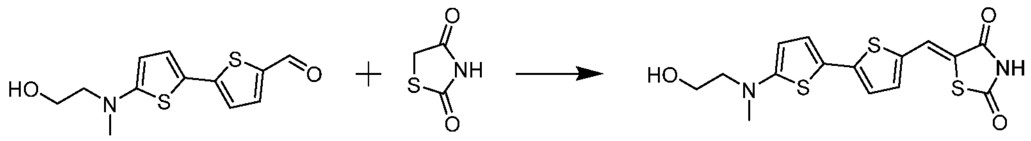

Compound 24 (I-5-62)

(I-5-62) was synthesized with reference to the synthetic method of (I-5-1), and the yield was 78.3%. 1H-NMR (400 MHz, CDCl3): δ=11.21 (s, 1H), 7.68 (d, 1H, J=7.88 Hz), 7.55 (d, 1H, J=7.88 Hz), 7.25 (d, 1H, J=7.65 Hz), 6.82 (s, 1H), 6.78 (d, 1H, J=7.65 Hz), 3.86 (t, 2H, J=4.80 Hz), 3.56 (t, 2H, J=4.80 Hz), 3.06 (s, 3H).

Compound 25

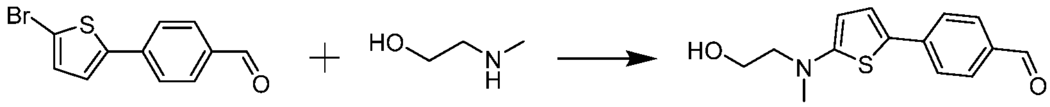

Compound 25

Compound 25 was synthesized with reference to the synthetic method of Compound 19, and the yield was 58.9%. 1H-NMR (400 MHz, CDCl3): δ=10.35 (s, 1H), 7.68 (d, 1H, J=7.88 Hz), 7.55 (d, 1H, J=7.88 Hz), 7.25(d, 2H, J=8.23 Hz), 6.78 (d, 2H, J=8.23 Hz), 3.86 (t, 2H, J=5.02 Hz), 3.56 (t, 2H, J=5.02 Hz), 3.06 (s, 3H).

(I-5-63)

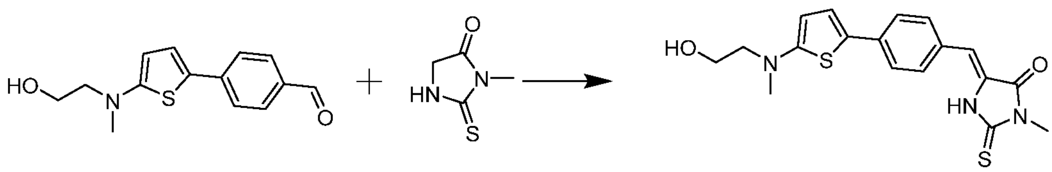

Compound 25 (I-5-63)

(I-5-63) was synthesized with reference to the synthetic method of (I-5-1), and the yield was 74.7%. 1H-NMR (400 MHz, CDCl3): δ=7.68 (d, 1H, J=7.88 Hz), 7.55 (d, 1H, J=7.88 Hz), 7.25 (d, 2H, J=8.23 Hz), 6.82 (s, 1H), 6.78 (d, 2H, J=8.23 Hz), 3.86 (t, 2H, J=5.02 Hz), 3.56 (t, 2H, J=5.02 Hz), 3.52 (s, 3H), 3.06 (s, 3H).

Compound 26

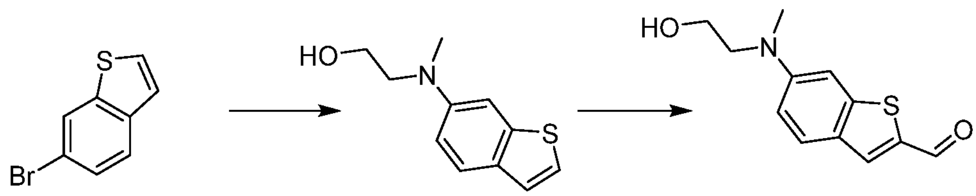

Compound 26

Compound 26 was synthesized with reference to the synthetic method of Compound 21, and the yield was 49.6%. 1H-NMR (400 MHz, CDCl3): δ=9.92 (s, 1H), 7.81 (s, 1H), 7.68 (d, J=9.0 Hz, 1H), 6.92 (s, 1H), 6.82 (d, J=9.1, 2.3 Hz, 1H), 3.61 (t, J=8.0 Hz, 2H), 3.34 (t, J=8.0 Hz, 2H), 3.10 (s, 3H).

(I-5-64)

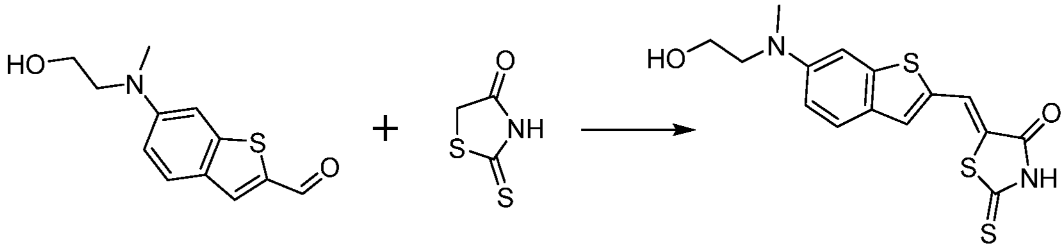

Compound 26 (I-5-64)

(I-5-64) was synthesized with reference to the synthetic method of (I-5-1), and the yield was 90.1%. 1H-NMR (400 MHz, CDCl3): δ=12.56 (s, 1H), 7.81 (s, 1H), 7.68 (d, J=9.0 Hz, 1H), 6.92 (s, 1H), 6.85 (s, 1H), 6.82 (d, J=9.1 Hz, 1H), 3.61 (t, J=8.0 Hz, 2H), 3.34 (t, J=8.0 Hz, 2H), 3.10 (s, 3H).

(I-5-65)

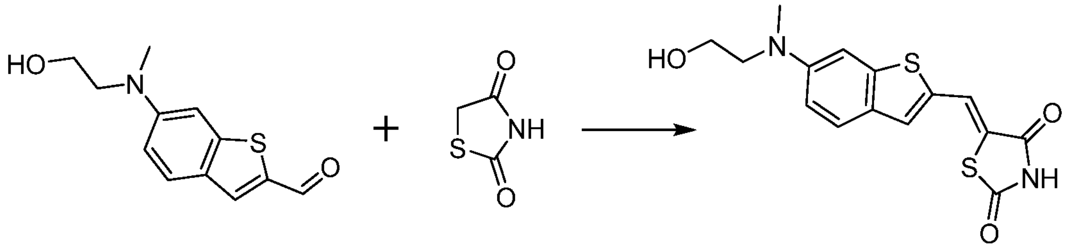

Compound 26 (I-5-65)

(I-5-65) was synthesized with reference to the synthetic method of (I-5-1), and the yield was 72.3%. 1H-NMR (400 MHz, CDCl3): δ=11.01 (s, 1H), 7.81 (s, 1H), 7.68 (d, J=9.0 Hz, 1H), 6.92 (s, 1H), 6.85 (s, 1H), 6.82 (d, J=9.1 Hz, 1H), 3.61 (t, J=8.0 Hz, 2H), 3.34 (t, J=8.0 Hz, 2H), 3.01 (s, 3H).

(I-5-66)

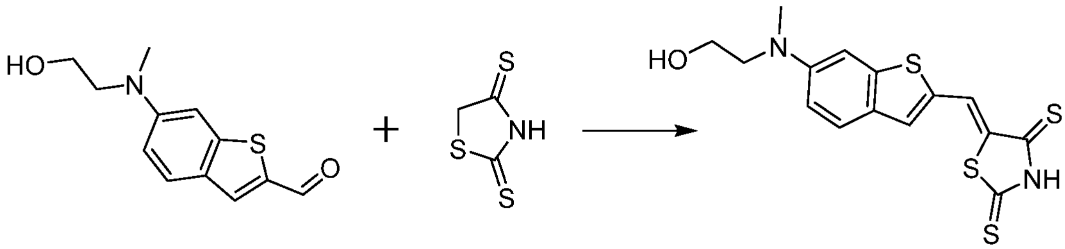

Compound 26 (I-5-65)

(I-5-66) was synthesized with reference to the synthetic method of (I-5-1), and the yield was 95.3%. 1H-NMR (400 MHz, CDCl3): δ=16.01 (s, 1H), 7.81 (s, 1H), 7.68 (d, J=9.0 Hz, 1H), 6.92 (s, 1H), 6.82 (d, J=9.1 Hz, 1H), 6.65 (s, 1H), 3.61 (t, J=8.0 Hz, 2H), 3.34 (t, J=8.0 Hz, 2H), 3.01 (s, 3H).

Compound 27

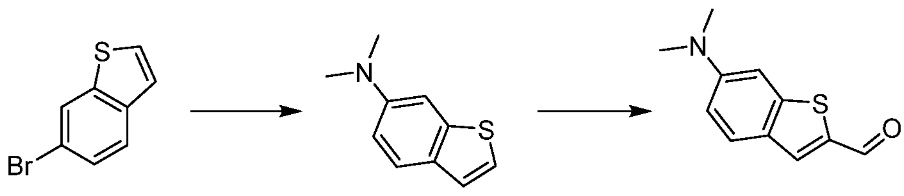

Compound 27

Compound 27 was synthesized with reference to the synthetic method of Compound 21, and the yield was 60.3%. 1H-NMR (400 MHz, CDCl3): δ=9.92 (s, 1H), 7.81 (s, 1H), 7.68 (d, J=9.0 Hz, 1H), 6.92 (d, J=2.0 Hz, 1H), 6.82 (d, J=9.1, 2.3 Hz, 1H), 3.05 (s, 6H).

(I-5-67)

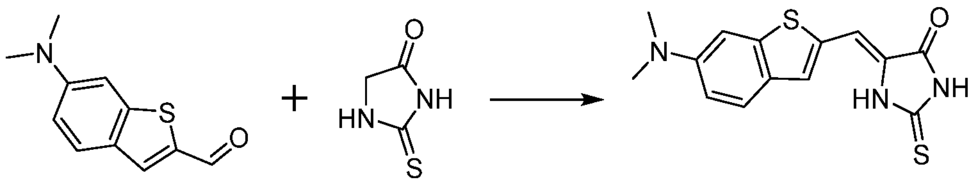

Compound 27 (I-5-67)

(I-5-67) was synthesized with reference to the synthetic method of (I-5-1), and the yield was 75.5%. 1H-NMR (400 MHz, CDCl3): δ=12.06 (s, 1H), 7.81 (s, 1H), 7.68 (d, J=9.0 Hz, 1H), 6.92 (d, J=2.0 Hz, 1H), 6.85 (s, 1H), 6.82 (d, J=9.1, 2.3 Hz, 1H), 3.05 (s, 6H).

Compound 28

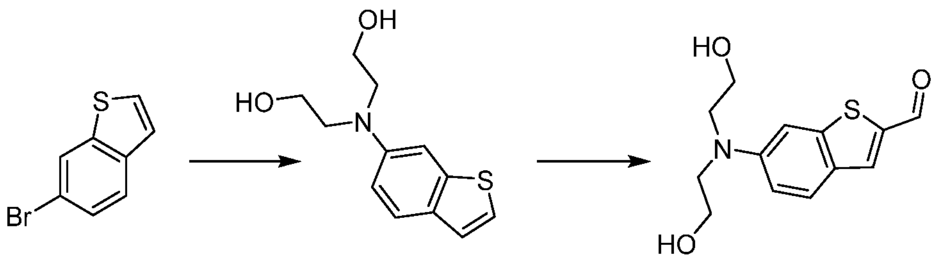

Compound 28 was synthesized with reference to the synthetic method of Compound 21, and the yield was 50.6%. 1H-NMR (400 MHz, CDCl3): δ=10.02 (s, 1H), 7.81 (s, 1H), 7.68 (d, J=9.0 Hz, 1H), 6.92 (d, J=2.0 Hz, 1H), 6.82 (d, J=9.1, 2.3 Hz, 1H), 3.61 (t, J=8.0 Hz, 4H), 3.34 (t, J=8.0 Hz, 4H).

(I-5-68)

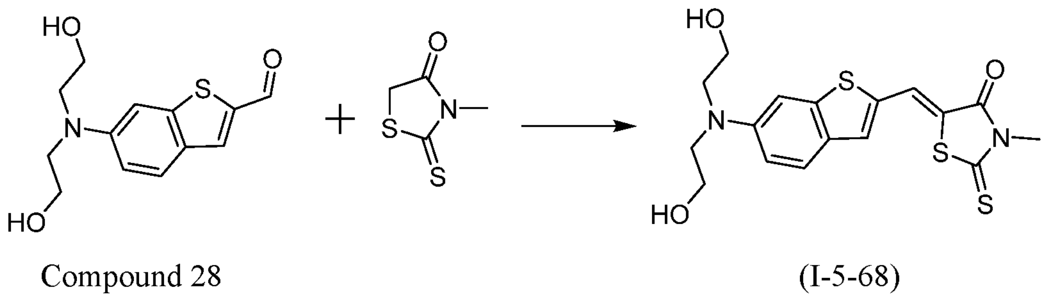

Compound 28 (I-5-68)

(I-5-68) was synthesized with reference to the synthetic method of (I-5-1), and the yield was 88.3%. 1H-NMR (400 MHz, CDCl3): δ=7.81 (s, 1H), 7.68 (d, J=9.0 Hz, 1H), 6.92 (d, J=2.0 Hz, 1H), 6.85 (s, 1H), 6.82 (d, J=9.1, 2.3 Hz, 1H), 3.61 (t, J=8.0 Hz, 4H), 3.52 (s, 3H), 3.34 (t, J=8.0 Hz, 4H).

Compound 29

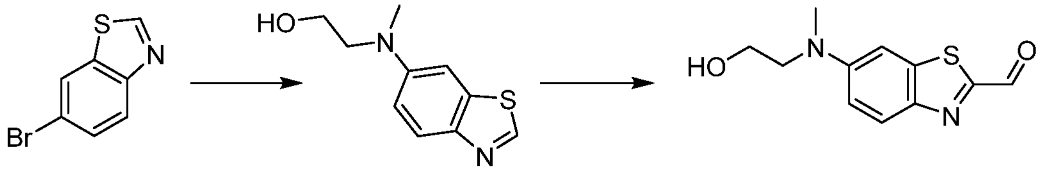

Compound 29

Compound 29 was synthesized with reference to the synthetic method of Compound 21, and the yield was 49.9%. 1H-NMR (400 MHz, CDCl3): δ=9.92 (s, 1H), 7.68 (d, J=9.0 Hz, 1H), 6.92 (s, 1H), 6.82 (d, J=9.1, 2.3 Hz, 1H), 3.61 (t, J=8.0 Hz, 2H), 3.34 (t, J=8.0 Hz, 2H), 3.10 (s, 3H).

(I-5-69)

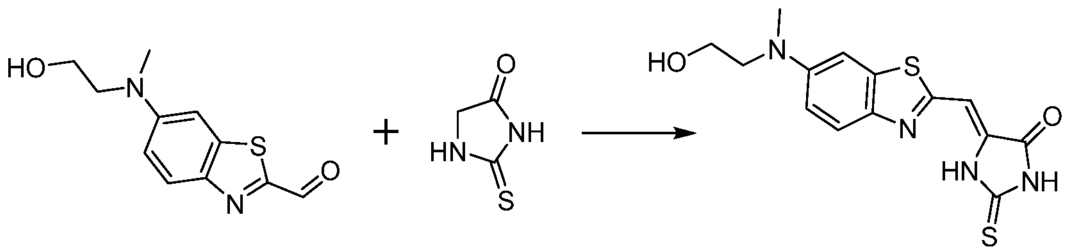

Compound 29 (I-5-69)

(I-5-69) was synthesized with reference to the synthetic method of (I-5-1), and the yield was 75.3%. 1H-NMR (400 MHz, CDCl3): δ=12.31 (s, 1H), 7.68 (d, J=9.0 Hz, 1H), 6.92 (s, 1H), 6.85 (s, 1H), 6.82 (d, J=9.1, 2.3 Hz, 1H), 3.61 (t, J=8.0 Hz, 2H), 3.34 (t, J=8.0 Hz, 2H), 3.10 (s, 3H).

(I-5-70)

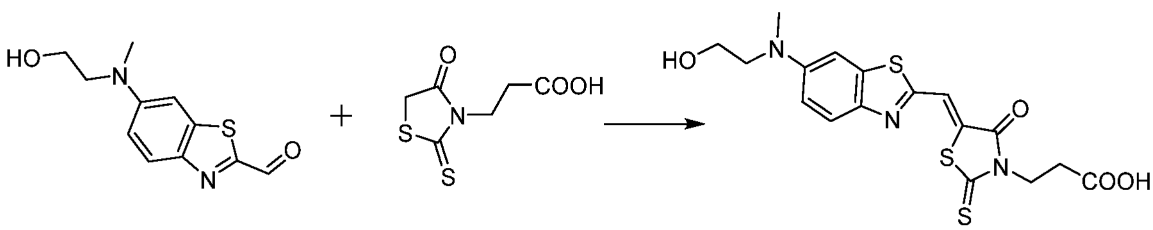

Compound 29 (I-5-70)

(I-5-70) was synthesized with reference to the synthetic method of (I-5-1), and the yield was 48.8%. 1H-NMR (400 MHz, CDCl3): δ=7.68 (d, J=9.0 Hz, 1H), 6.92 (s, 1H), 6.85 (s, 1H), 6.82 (d, J=9.1, 2.3 Hz, 1H), 4.24 (t, J=5.2 Hz, 2H), 3.61 (t, J=8.0 Hz, 2H), 3.34 (t, J=8.0 Hz, 2H), 3.10 (s, 3H), 2.63 (t, J=5.2 Hz, 2H).

(I-5-71)

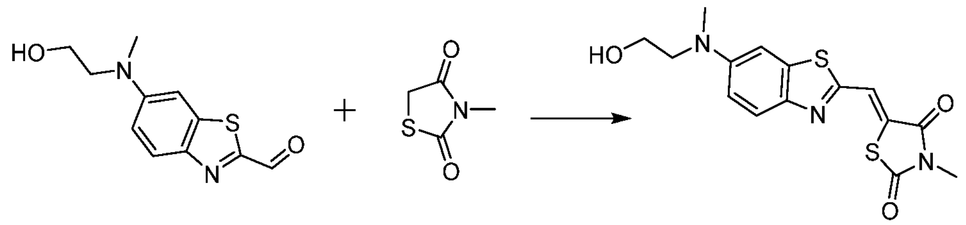

Compound 29 (I-5-71)

(I-5-71) was synthesized with reference to the synthetic method of (I-5-1), and the yield was 78.9%. 1H-NMR (400 MHz, CDCl3): δ=7.68 (d, J=9.0 Hz, 1H), 6.92 (s, 1H), 6.85 (s, 1H), 6.82 (d, J=9.1, 2.3 Hz, 1H), 3.61 (t, J=8.0 Hz, 2H), 3.52 (s, 3H), 3.34 (t, J=8.0 Hz, 2H), 3.10 (s, 3H).

(I-5-72)

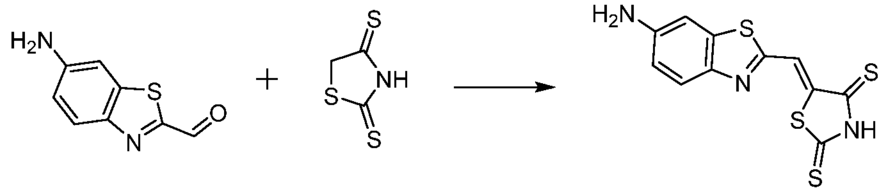

(I-5-72)

(I-5-72) was synthesized with reference to the synthetic method of (I-5-1), and the yield was 98.4%. 1H-NMR (400 MHz, CDCl3): δ=7.68 (d, J=9.0 Hz, 1H), 6.92 (s, 1H), 6.85 (s, 1H), 6.82 (d, J=9.1, 2.3 Hz, 1H).

Compound 30

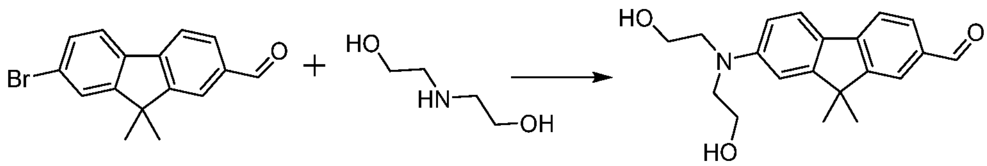

Compound 30

Compound 30 was synthesized with reference to the synthetic method of Compound 19, and the yield was 42.5%. 1H-NMR (400 MHz, CDCl3): δ=9.92 (s, 1H), 7.82 (s, 1H), 7.71 (m, 1H), 7.60 (m, 2H), 6.78 (m, 2H), 3.82 (t, 4H, J=5.6 Hz), 3.54 (t, 4H, J=5.6 Hz), 1.42 (s, 6H).

(I-5-73)

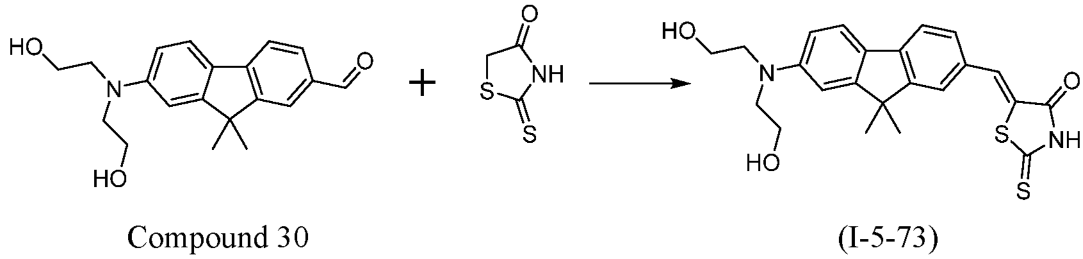

Compound 30 (I-5-73)

(I-5-73) was synthesized with reference to the synthetic method of (I-5-1), and the yield was 88.9%. 1H-NMR

(400 MHz, CDCl3): δ=12.31 (s, 1H), 7.82 (s, 1H), 7.71 (m, 1H), 7.60 (m, 2H), 6.85 (s, 1H), 6.78 (m, 2H), 3.82 (t, 4H, J=5.6 Hz), 3.54 (t, 4H, J=5.6 Hz), 1.42 (s, 6H).

Compound 31

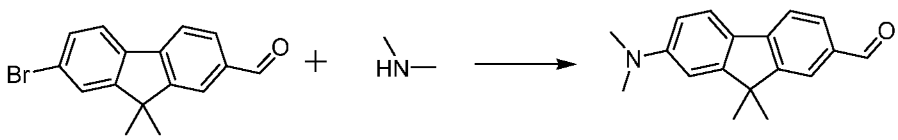

Compound 31

Compound 31 was synthesized with reference to the synthetic method of Compound 19, and the yield was 82.7%. 1H-NMR (400 MHz, CDCl3): δ=9.92 (s, 1H), 7.82 (s, 1H), 7.71 (m, 1H), 7.60 (m, 2H), 6.78 (m, 2H), 3.62 (s, 6H), 1.42 (s, 6H).

(I-5-74)

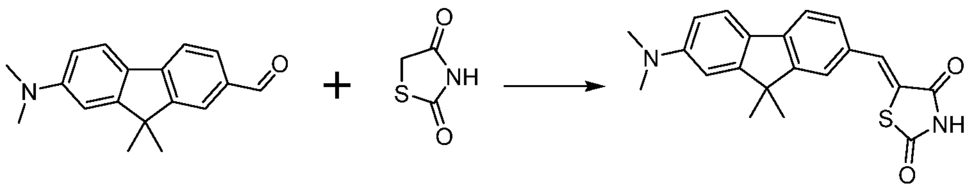

Compound 31 (I-5-74)

(I-5-74) was synthesized with reference to the synthetic method of (I-5-1), and the yield was 78.3%. 1H-NMR (400 MHz, CDCl3): δ= 11.23 (s, 1H), 7.82 (s, 1H), 7.71 (m, 1H), 7.60 (m, 2H), 6.85 (s, 1H), 6.78 (m, 2H), 3.62 (s, 6H), 1.42 (s, 6H).

Compound 32

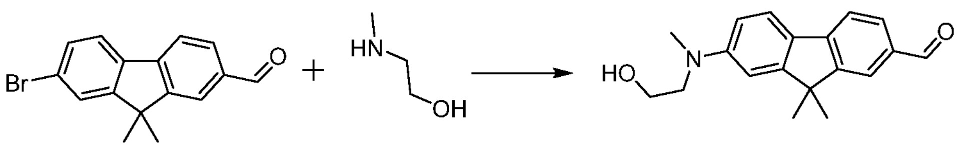

Compound 32

Compound 32 was synthesized with reference to the synthetic method of Compound 19, and the yield was 63.0%. 1H-NMR (400 MHz, CDCl3): δ=9.92 (s, 1H), 7.82 (s, 1H), 7.71 (m, 1H), 7.60 (m, 2H), 6.78 (m, 2H), 3.82 (t, J=5.6 Hz, 2H), 3.54 (t, J=5.6 Hz, 2H), 3.05 (s, 3H), 1.42 (s, 6H).

(I-5-75)

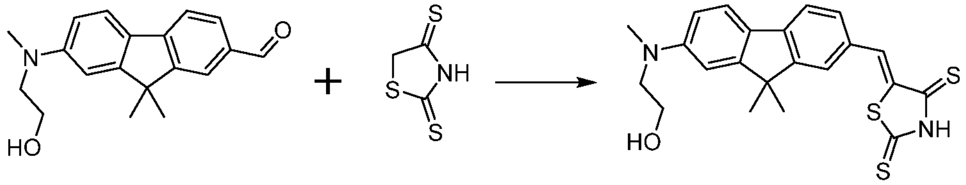

Compound 32 (I-5-75)

(I-5-75) was synthesized with reference to the synthetic method of (I-5-1), and the yield was 98.3%. 1H-NMR (400 MHz, CDCl3): δ=14.89 (s, 1H), 7.82 (s, 1H), 7.71 (m, 1H), 7.60 (m, 2H), 6.85 (s, 1H), 6.78 (m, 2H), 3.82 (t, J=5.6 Hz, 2H), 3.54 (t, J=5.6 Hz, 2H), 3.05 (s, 3H), 1.42 (s, 6H).

(I-5-76)

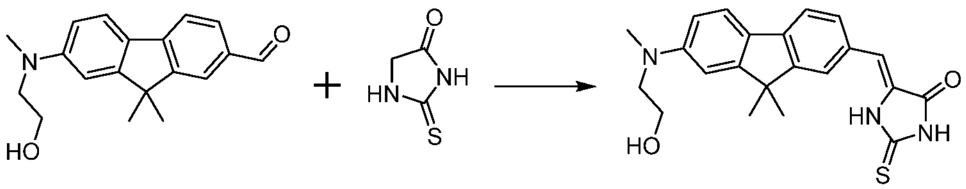

Compound 32 (I-5-76)

(I-5-76) was synthesized with reference to the synthetic method of (I-5-1), and the yield was 72.8%. 1H-NMR (400 MHz, CDCl3): δ=12.03 (s, 1H), 7.82 (s, 1H), 7.71 (m, 1H), 7.60 (m, 2H), 6.82 (s, 1H), 6.78 (m, 2H), 3.82 (t, J=5.6 Hz, 2H), 3.54 (t, J=5.6 Hz, 2H), 3.05 (s, 3H), 1.42 (s, 6H).

Compound 33

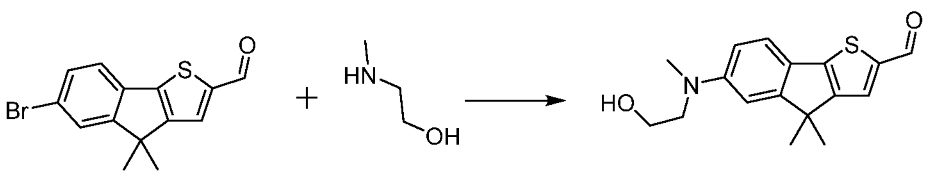

Compound 33

Compound 33 was synthesized with reference to the synthetic method of Compound 19, and the yield was 68.1%. 1H-NMR (400 MHz, CDCl3): δ=9.92 (s, 1H), 7.82 (s, 1H), 7.71 (m, 1H), 7.60 (m, 2H), 3.82 (t, J=5.6 Hz, 2H), 3.54 (t, J=5.6 Hz, 2H), 3.05 (s, 3H), 1.42 (s, 6H).

(I-5-77)

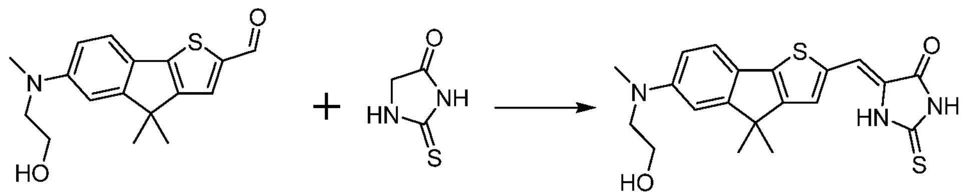

Compound 33 (I-5-77)

(I-5-77) was synthesized with reference to the synthetic method of (I-5-1), and the yield was 70.1%. 1H-NMR (400 MHz, CDCl3): δ=12.05 (s, 1H), 7.82 (s, 1H), 7.71 (m, 1H), 7.60 (m, 2H), 6.82 (s, 1H), 3.82 (t, J=5.6 Hz, 2H), 3.54 (t, J=5.6 Hz, 2H), 3.05 (s, 3H), 1.42 (s, 6H).

(I-5-78)

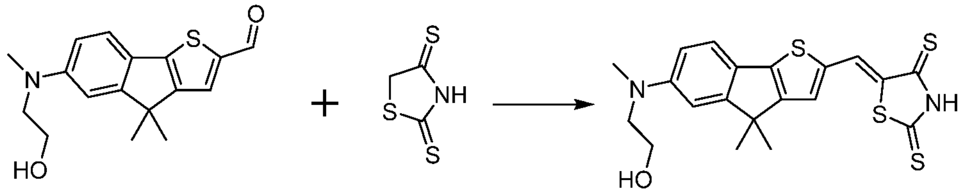

Compound 33 (I-5-78)

(I-5-78) was synthesized with reference to the synthetic method of (I-5-1), and the yield was 70.2%. 1H-NMR (400 MHz, CDCl3): δ=11.25 (s, 1H), 7.82 (s, 1H), 7.71 (m, 1H), 7.60 (m, 2H), 6.82 (s, 1H), 3.82 (t, J=5.6 Hz, 2H), 3.54 (t, J=5.6 Hz, 2H), 3.05 (s, 3H), 1.42 (s, 6H).

(I-5-79)

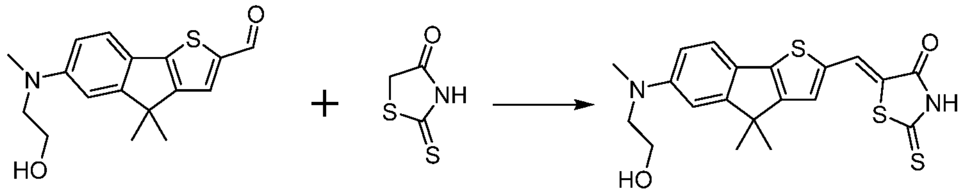

Compound 33 (I-5-79)

(I-5-79) was synthesized with reference to the synthetic method of (I-5-1), and the yield was 80.6%. 1H-NMR (400 MHz, CDCl3): δ=13.01 (s, 1H), 7.82 (s, 1H), 7.71 (m, 1H), 7.60 (m, 2H), 6.82 (s, 1H), 3.82 (t, J=5.6 Hz, 2H), 3.54 (t, J=5.6 Hz, 2H), 3.05 (s, 3H), 1.42 (s, 6H).

Compound 34

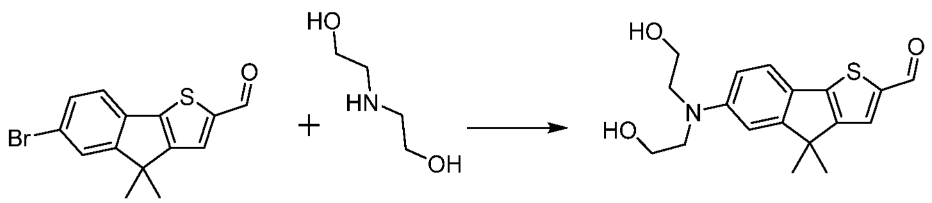

Compound 34

Compound 34 was synthesized with reference to the synthetic method of Compound 19, and the yield was 58.9%. 1H-NMR (400 MHz, CDCl3): δ=9.92 (s, 1H), 7.82 (s, 1H), 7.71 (m, 1H), 7.60 (m, 2H), 3.82 (t, J=5.6 Hz, 4H), 3.54 (t, J=5.6 Hz, 4H), 3.05 (s, 3H), 1.42 (s, 6H).

(I-5-80)

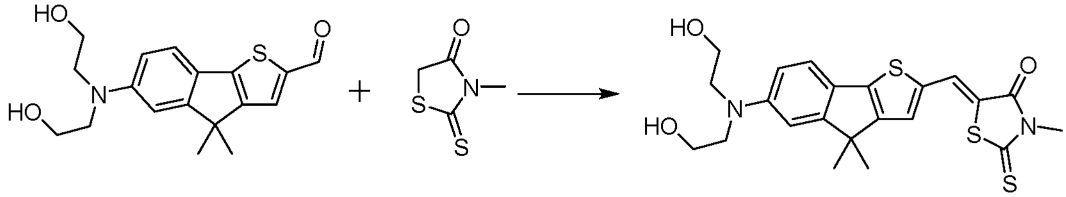

Compound 34 (I-5-80)

(I-5-80) was synthesized with reference to the synthetic method of (I-5-1), and the yield was 90.6%. 1H-NMR (400 MHz, CDCl3): δ=7.82 (s, 1H), 7.71 (m, 1H), 7.60 (m, 2H), 3.82 (t, J=5.6 Hz, 4H), 3.54 (t, J=5.6 Hz, 4H), 3.55 (s, 3H), 1.42 (s, 6H).

Compound 35

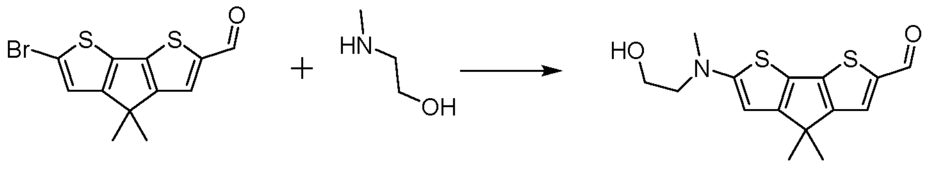

Compound 35

Compound 35 was synthesized with reference to the synthetic method of Compound 19, and the yield was 59.7%. 1H-NMR (400 MHz, CDCl3): δ=10.02 (s, 1H), 7.82 (s, 1H), 7.71 (s, 1H), 3.82 (t, J=5.6 Hz, 2H), 3.54 (t, J=5.6 Hz, 2H), 3.05 (s, 3H), 1.42 (s, 6H).

(I-5-81)

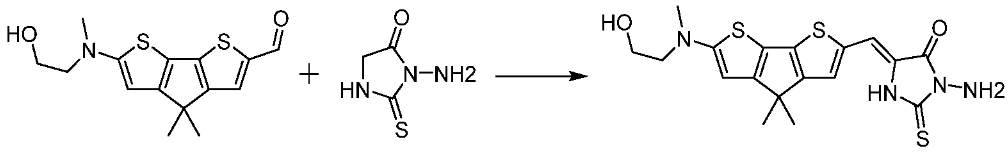

Compound 35 (I-5-81)

(I-5-81) was synthesized with reference to the synthetic method of (I-5-1), and the yield was 70.2%. 1H-NMR (400 MHz, CDCl3): δ=7.82 (s, 1H), 7.71 (s, 1H), 3.82 (t, J=5.6 Hz, 2H), 3.54 (t, J=5.6 Hz, 2H), 3.05 (s, 3H), 1.42 (s, 6H).

(I-5-82)

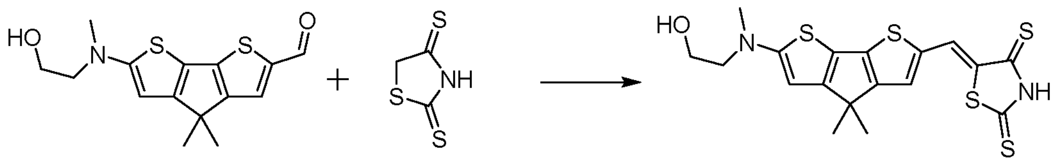

Compound 35 (I-5-82)

(I-5-82) was synthesized with reference to the synthetic method of (I-5-1), and the yield was 95.4%. 1H-NMR (400 MHz, CDCl3): δ=14.21(s, 1H), 7.82 (s, 1H), 7.71 (s, 1H), 3.82 (t, J=5.6 Hz, 2H), 3.54 (t, J=5.6 Hz, 2H), 3.05 (s, 3H), 1.42 (s, 6H).

(I-5-83)

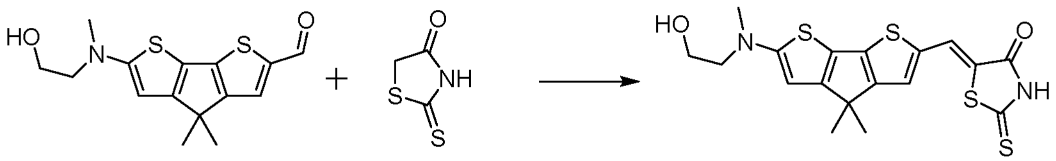

Compound 35 (I-5-83)

(I-5-83) was synthesized with reference to the synthetic method of (I-5-1), and the yield was 95.1%. 1H-NMR (400 MHz, CDCl3): δ=12.21(s, 1H), 7.82 (s, 1H), 7.71 (s, 1H), 3.82 (t, J=5.6 Hz, 2H), 3.54 (t, J=5.6 Hz, 2H), 3.05 (s, 3H), 1.42 (s, 6H).

Compound 36

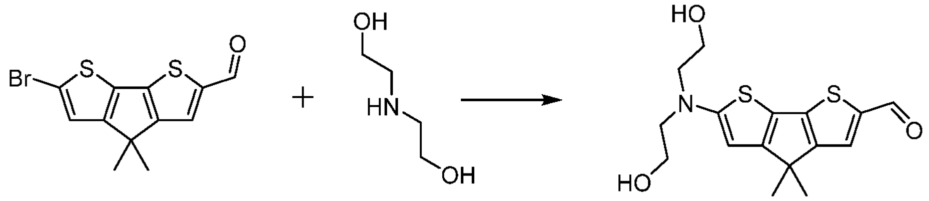

Compound 36

Compound 36 was synthesized with reference to the synthetic method of Compound 19, and the yield was 63.5%. 1H-NMR (400 MHz, CDCl3): δ=10.02 (s, 1H), 7.82 (s, 1H), 7.71 (s, 1H), 3.82 (t, J=5.6 Hz, 4H), 3.54 (t, J=5.6 Hz, 4H), 1.42 (s, 6H).

(I-5-84)

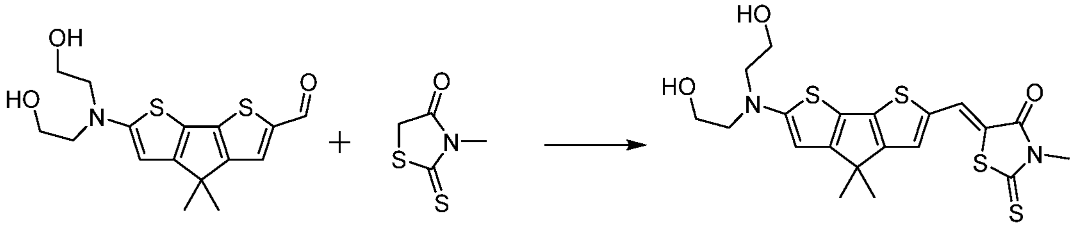

Compound 36 (I-5-84)

(I-5-84) was synthesized with reference to the synthetic method of (I-5-1), and the yield was 88.3%. 1H-NMR (400 MHz, CDCl3): δ=7.82 (s, 1H), 7.71 (s, 1H), 3.82 (t, J=5.6 Hz, 4H), 3.54 (t, J=5.6 Hz, 4H), 3.55 (s, 3H), 1.42 (s, 6H).

It should be understood that, unless otherwise specifically noted, the dosages, reaction conditions, etc. in the embodiments of the present specification are approximations and may be slightly changed according to actual conditions so as to obtain similar results. Unless otherwise specifically limited, all professional and scientific terms used herein have the same meaning as those understood by those skilled in the art. All references mentioned herein are included in this application. The present description describes preferable embodiments for the purpose of demonstration. Those skilled in the art may carry out the present discourse by using methods and materials similar to those described herein, so as to obtain the same or similar results. All alterations or modifications of the present disclosure shall remain within the scope limited by the attached claims of the present disclosure.

## Claims

1. A nucleic acid aptamer molecule, comprising following nucleotide sequences (a), (b) or (c):

   (a): a nucleotide sequence $N_1$CUCCAGGUG$N_{11}$-$N_{12}$-$N_{13}$GUAACGGACUUAGAUCCACUGUACG$N_{39}$, wherein $N_1$, $N_{11}$, $N_{12}$, $N_{13}$ and $N_{39}$ represent nucleotide fragments greater than or equal to 1 in length, and at least one base pair in $N_1$ and $N_{39}$ nucleotide sequences forms a complementary pair, and at least one base pair in $N_{11}$ and $N_{13}$ nucleotide sequences forms a complementary pair;
   (b): a nucleotide sequence with an identity of at least 71% to the nucleotide sequence limited by (a); and
   (c): a nucleic acid aptamer molecule derived from (a) at a position not including $N_1$, $N_{11}$, $N_{12}$, $N_{13}$ and $N_{39}$ in the nucleotide sequence limited by (a), with substitution, missing and/or addition of one or several nucleotides, and having aptamer functions.

2. The nucleic acid aptamer molecule according to claim 1, a sequence having an identity of at least 71%, 74%, 76%, 79%, 82%, 85%, 88%, 91%, 94%, 97% or 100% to the N16 structure nucleotide sequence of (a).

3. The nucleic acid aptamer molecule according to claim 1, wherein the nucleotide sequence (c) is nucleic acid aptamer molecules obtained with substitution, missing and/or addition of 10, 9, 8, 7, 6, 5, 4, 3, 2 or 1 nucleotide at a position

in the N16 structure nucleotide sequence limited by (a) not including $N_1$, $N_{11}$, $N_{12}$, $N_{13}$ and $N_{39}$.

4. The nucleic acid aptamer molecule according to claim 3, wherein the nucleotide sequence (c) is nucleic acid aptamer molecules obtained with substitution of 7, 6, 5, 4, 3, 2 or 1 nucleotide at a position in the nucleotide sequence limited by (a) not including $N_1$, $N_{11}$, $N_{12}$, $N_{13}$ and $N_{39}$.

5. The nucleic acid aptamer molecule according to any one of claims 1 to 4, wherein: when $N_1$ and $N_{39}$ in the nucleotide sequence (a) form a complementary pair, a direction of $N_1$ nucleotide sequence is 5'-3', and a direction of $N_{39}$ nucleotide sequence is 3'-5'; and, when $N_{11}$ and $N_{13}$ form a complementary pair, a direction of $N_{11}$ nucleotide sequence is 5'-3', and a direction of $N_{13}$ nucleotide sequence is 3'-5'.

6. The nucleic acid aptamer molecule according to claim 5, wherein: when at least one fragment of $N_1$ and $N_{39}$ is greater than or equal to 5 nucleotide bases in length, at least two pairs of nucleotide bases in $N_1$ and $N_{39}$ nucleotide sequences form complementary pairs; when at least one fragment of $N_{11}$ and $N_{13}$ is greater than or equal to 5 nucleotide bases in length, at least two pairs of nucleotide bases in $N_{11}$ and $N_{13}$ nucleotide sequences form complementary pairs.

7. The nucleic acid aptamer molecule according to any one of claims 1 to 6, wherein the nucleotide substitution for the General Formula N16 structure is selected from one of the following groups: C2A, C2U, C2G, U3A, U3C, U3G, C4A, C4U, C4G, C5A, C5U, C5G, A6U, A6C, A6G, G7A, G7U, G7C, G8A, G8U, G8C, U9A, U9C, U9G, G10A, G10U, G10C, G14A, G14U, G14C, U15A, U15C, U15G, A16U, A16C, A16G, A17U, A17C, A17G, C18A, C18U, C18G, G19A, G19U, G19C, G20A, G20U, G20C, A21U, A21C, A21G, C22A, C22U, C22G, U23A, U23C, U23G, U24A, U24C, U24G, A25U, A25C, A25G, G26A, G26U, G26C, A27U, A27C, A27G, U28A, U28C, U28G, C29A, C29U, C29G, C30A, C30U, C30G, A31U, A31C, A31G, C32A, C32U, C32G, U33A, U33C, U33G, G34A, G34U, G34C, U35A, U35C, U35G, A36U, A36C, A36G, C37A, C37U, C37G, G38A, G38U, G38C, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U3 5G.

8. The nucleic acid aptamer molecule according to claim 7, wherein the nucleotide substitution for the General Formula N16 structure is selected from one of the following groups: C2U, U3A, U3C, U3G, C4A, C4U, C4G, C5A, C5U, C5G, A6U, A6C, A6G, G8A, U9A, U9C, U9G, G10A, U15C, A16U, A16C, A16G, A17G, C18A, C18U, C18G, A21C, A21G, C22A, C22U, C22G, U23A, U23C, U23G, U24A, U24C, U24G, A25U, A25C, A25G, G26A, G26U, G26C, A27U, A27C, A27G, U28A, U28C, U28G, C29U, C29G, C30U, A31U, A31C, A31G, C32A, C32U, C32G, U33A, U33G, G34A, G34U, G34C, U35A, U35C, U35G, A36U, A36C, A36G, C37A, C37U, C37G, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G, C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/A16G/U35G, C4G/C22U/U24G/A17G/U35G.

9. The nucleic acid aptamer molecule according to claim 8, wherein the nucleotide substitution for the General Formula N16 structure is selected from one of the following groups: U3A, U3C, U3G, C4A, C4U, C4G, C5A, C5U, C5G, A6U, A6G, G8A, U9A, U9C, U9G, G10A, U15C, A16U, A16C, A16G, A17G, C18A, C18G, A21C, A21G, C22A, C22U, C22G, U23A, U23C, U24A, U24C, U24G, A25U, A25C, A25G, G26A, G26U, G26C, A27U, A27C, A27G, U28A, U28C, U28G, C29U, C29G, A31C, A31G, C32A, C32U, C32G, G34A, G34U, G34C, U35A, U35C, U35G, A36U, A36C, A36G, C37A, C37U, C37G, C4G/U9A, C4G/A16G, C4G/A17G, C4G/C22U, C4G/U24G, C4G/U35G, U9A/A16G, U9A/A17G, U9A/C22U, U9A/U24G, U9A/U35G, A16G/A17G, A16G/C22U, A16G/U24G, A16G/U35G, A17G/C22U, A17G/U24G, A17G/U35G, C22U/U24G, C22U/U35G, U24G/U35G, C4G/U9A/A16G, C4G/U9A/A17G, C4G/U9A/C22U, C4G/U9A/U24G, C4G/U9A/U35G, C4G/A16G/A17G, C4G/A16G/C22U, C4G/A16G/U24G,

C4G/A16G/U35G, C4G/A17G/C22U, C4G/A17G/U24G, C4G/A17G/U35G, C4G/C22U/U24G, C4G/C22U/U35G, C4G/U24G/U35G, C4G/C22U/U24G/U9A, C4G/C22U/U24G/A16G, C4G/C22U/U24G/A17G, C4G/C22U/U24G/U35G, C4G/C22U/U24G/U9A/A16G, C4G/C22U/U24G/U9A/A17G, C4G/C22U/U24G/U9A/U35G, C4G/C22U/U24G/A16G/A17G, C4G/C22U/U24G/ A 16G/U3 5G, C4G/C22U/U24G/A17G/U3 5G.

**10.** The nucleic acid aptamer molecule according to any one of claims 1 to 9, wherein nucleotide sequences at $N_1$ and $N_{39}$ in the nucleotide sequence (a) are F30 or tRNA scaffold RNA sequences.

**11.** The nucleic acid aptamer molecule according to any one of the preceding claims, wherein the aptamer molecules are RNA molecules or base-modified RNA molecules.

**12.** The nucleic acid aptamer molecule according to any one of the preceding claims, wherein the aptamer molecules are DNA-RNA hybrid molecules or base-modified DNA-RNA molecules.

**13.** The nucleic acid aptamer molecule according to any one of the preceding claims, wherein the aptamer function refers to that the nucleic acid aptamer can enhance fluorescence intensity of fluorophore molecules under excitation light of appropriate wavelength by at least 2 times, at least 5 to 10 times, at least 20 to 50 times, at least 100 to 200 times or at least 200 to 1,000 times.

**14.** The nucleic acid aptamer molecule according to claim 1, further comprising concatemers that can bind with multiple fluorophore molecules, wherein the concatemers are connected by spacer sequences of appropriate length that may be 2, 3, 4, 5, 6, 7, 8 or more. Nucleotides of the concatemers can be selected from but are not limited to sequences SEQ ID No: 1, 2, 3, 4, 5, 6, 7, 8, 9, 10, 11, 12 and 13.

**15.** A complex of nucleic acid aptamer molecules and fluorophore molecules, wherein the nucleic acid aptamer molecule is the nucleic acid aptamer molecule according to any one of claims 1 to 14 and the fluorophore molecule has a structure shown by Formula (I) below:

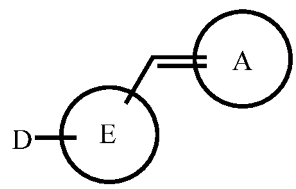

(I)

an electron donor part-D is -NX1-X2, X1 is selected from hydrogen, alkyl or modified alkyl, and X2 is selected from hydrogen, alkyl or modified alkyl; X1 and X2 are optionally interconnected, and form an aliphatic heterocycle with N atoms;
a conjugate system E is formed by at least one conjugate connection selected from double bond, triple bond, aromatic ring and aromatic heterocycle, wherein each hydrogen atom contained therein is optionally and independently substituted by a substituent group selected from halogen atom, hydroxyl group, amino group, primary amino group, secondary amino group, hydrophilic group, alkyl and modified alkyl, and the substituent group is optionally interconnected to form an aliphatic ring or an aliphatic heterocycle;
X1 and X2 are independently connected with the conjugate system E to form an aliphatic heterocycle;
an electron acceptor A optionally forms a ring structure represented by Formula (I-1-a) below:

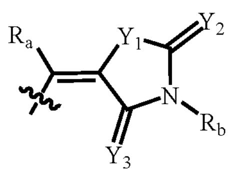

(I-1-a)

Ra is independently selected from hydrogen, halogen atom, nitro group, alkyl group, aryl group, heteroaryl group, hydrophilic group or modified alkyl; $R_b$ is independently selected from hydrogen, halogen atom, hydroxyl group, carboxyl group, amino group, nitro group, alkyl group, aryl group, heteroaryl group, hydrophilic group or modified alkyl, or is a group formed by conjugate connection of the double bond with at least one of the aromatic ring and aromatic heterocycle;
each $Y_1$ is independently selected from -O-, -S-, -(S=O)- and -($NR_i$)-, wherein $R_i$ is selected from hydrogen, amino group, alkyl or modified alkyl;
each $Y_2$ is independently selected from =O, =S, =S=O and =$NR_i$, wherein $R_i$ is selected from hydrogen, alkyl or modified alkyl;
each $Y_3$ is independently selected from =O, =S, =S=O and =$NR_i$, wherein $R_i$ is selected from hydrogen, alkyl or modified alkyl;
or, each $Y_3$ independently is =C ($R_e$)(CN); wherein $R_e$ is selected from hydrogen, ester group, amide group, sulfonic group, sulfamine, and sulfonate ester group;
wherein:

the "alkyl" is $C_1$-$C_{30}$ straight or branched alkyl; preferably, the "alkyl" is $C_1$-$C_{10}$ straight or branched alkyl; preferably, the "alkyl" is $C_1$-$C_7$ straight or branched alkyl; preferably, the "alkyl" is $C_1$-$C_5$ straight or branched alkyl; preferably, the "alkyl" is selected from methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, n-heptyl, 2-methylhexyl, 3-methylhexyl, 2,2-dimethylpentyl, 3,3-dimethylpentyl, 2,3-dimethylpentyl, 2,4-dimethylpentyl, 3-ethylpentyl or 2,2,3-trimethylbutyl;
the "modified alkyl group" is a group obtained by replacement of any carbon atom of an alkyl group with at least one group selected from a halogen atom, -O-, -OH, -CO-, -CS-, -$NO_2$, -CN, -S-, -$SO_2$-, -(S=O)-,

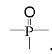,

phenyl group, phenylene group, primary amino group, secondary amino group, tertiary amino group, quaternary ammonium group, saturated or unsaturated monocyclic or bicyclic cyclic-hydrocarbon group, biaryl heterocyclic group, and a bridged aliphatic heterocyclic group, the modified alkyl group having 1 to 30 carbon atoms, and the carbon-carbon single bond is optionally and independently replaced with a carbon-carbon double bond or a carbon-carbon triple bond;
the replacement of carbon atoms refers to that carbon atoms or the carbon atoms and hydrogen atoms thereon together are replaced by corresponding groups;
the "aliphatic ring" is a saturated or unsaturated 4- to 10-membered monocyclic or polycyclic aliphatic ring;
the "aliphatic heterocycle" is a saturated or unsaturated 4- to 10-membered monocyclic or polycyclic aliphatic heterocycle containing at least one heteroatom selected from N, O, S, or Si; if contained on the aliphatic heterocycle, S atoms are in the form of -S-, -SO-, or -$SO_2$-; the aliphatic heterocycle is optionally substituted with halogen atom, nitro group, alkyl group, aromatic group, hydrophilic group, and modified alkyl group;
the "aromatic group or aromatic ring" is a 5- to 10-membered monocyclic or fused bicyclic aromatic group;
the "heteroaryl or aromatic heterocyclic ring" is a 5- to 10-membered monocyclic or fused bicyclic heteroaromatic group containing at least one heteroatom selected from N, O, S or Si on the ring;
the "halogen atom" is respectively and independently selected from F, Cl, Br, I;
the "hydrophilic group" is hydroxyl group, sulfonic acid group, carboxyl group, phosphite group, primary amino group, secondary amino group, or tertiary amino group;
the "bridged aliphatic heterocycle" is a 5- to 20-membered bridged aliphatic heterocycle containing at least one heteroatom selected from N, O, or S on the ring;
the "ester group" is R(C=O)OR’ group;
the "phosphite group" is RP(=O)$(OH)_2$ group;
the "sulfonic group" is $RSO_3H$ group;
the "sulfonate ester group" is $RSO_3R$’ group;
the "sulfamine" is $RSO_2NR$’R" group;
the "primary amino group" is $RNH_2$ group;
the "secondary amino group" is RNHR’ group;
the "tertiary amino group" is RNR’R" group;
the "quaternary ammonium group" is R R’R" R‴$N^+$ group;

each R, R', R", R‴ respectively and independently is single bond, alkyl group, alkylene group, modified alkyl group, or modified alkylene group, and the modified alkyl group or modified alkylene group is a group obtained by replacement of any carbon atom of $C_1$-$C_{10}$ (preferably $C_1$-$C_6$) alkyl or alkylene group with a group selected from -O-, -OH, -CO-, -CS-, -(S=O)-;
optionally, the modified alkyl group or modified alkylene group respectively and independently is a group containing at least one group selected from -OH, -O-, ethylene glycol unit ($-(CH_2CH_2O)n-$), Ci-Cs alkyl group, Ci-Cs alkoxy group, Ci-Cs acyloxy group, Ci-Cs haloalkyl group, monosaccharide group, disaccharide group, polysaccharide group, -O-CO-, -NH-CO-, $-(-NH-CHR''''-CO-)_n-$, $-SO_2-O-$, -SO-, $-SO_2-NH-$, -S-S-, -CH=CH-, halogen atom, cyano group, nitro group, o-nitrophenyl group, benzoylmethyl group, and phosphate easter group, wherein n is 1 to 100, preferably 1 to 50, more preferably 1 to 30, more preferably 1 to 10; R"" is H or residue of $\alpha$ amino acid; the "phosphate easter group" is defined as above;
the "monosaccharide unit" is a saccharide unit that can no longer be simply hydrolyzed into smaller sugar molecules;
the "disaccharide unit" is a saccharide unit formed by dehydration of two monosaccharides;
the "polysaccharide unit" is a saccharide unit formed by dehydration of 10 or more monosaccharides;
optionally, the Ci-Cs alkyl group is methyl, ethyl, propyl, or isopropyl, the Ci-Cs alkoxy group is methoxy, ethoxy, propoxy, or isopropoxy, the Ci-Cs acyloxy is acetoxy, ethyl, propyl, or isopropyl, and the Ci-Cs haloalkyl is trifluoromethyl, chloromethyl, or bromomethyl;
optionally, the aliphatic heterocycle is selected from azetidine, pyrrolidine, piperidine, tetrahydrofuran, tetrahydropyran, morpholine, and thiomorpholine;
optionally, the conjugate system E is a structure selected from following Formulae (I-2-1) to (I-2-39):

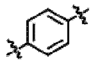 (I-2-1), 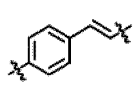 (I-2-2), 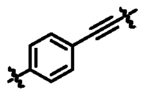 (I-2-3), 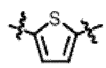 (I-2-4),

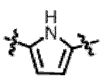 (I-2-5), 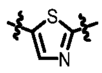 (I-2-6), 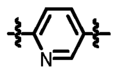 (I-2-7), 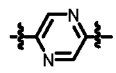 (I-2-8),

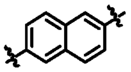 (I-2-9), 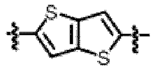 (I-2-10), 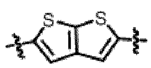 (I-2-11), 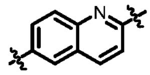 (I-2-12),

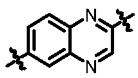 (I-2-13), 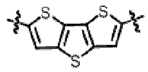 (I-2-14), 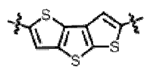 (I-2-15),

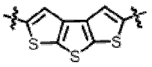 (I-2-16), 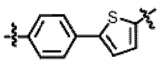 (I-2-17), 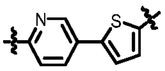 (I-2-18),

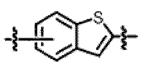 (I-2-19), 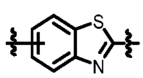 (I-2-20), 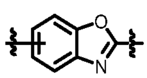 (I-2-21),

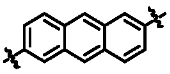 (I-2-22), 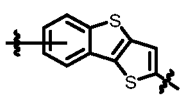 (I-2-23), 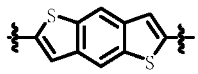 (I-2-24),

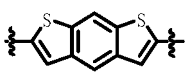 (I-2-25), 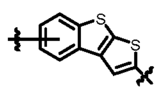 (I-2-26), 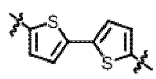 (I-2-27),

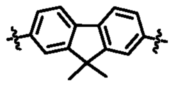 (I-2-28), 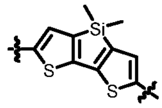 (I-2-29), 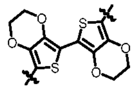 (I-2-30),

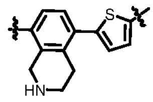 (I-2-31), 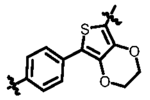 (I-2-32), 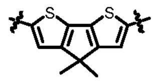 (I-2-33),

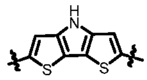 (I-2-34), 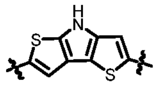 (I-2-35), 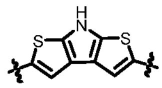 (I-2-36),

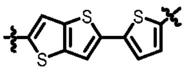 (I-2-37), 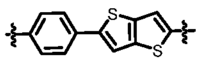 (I-2-38), 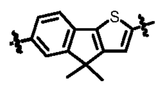 (I-2-39);

optionally, the conjugate system E and $-NX_1-X_2$ form an aliphatic heterocycle represented by Formulae (I-3-1) to (I-3-5):

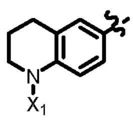 (I-3-1), 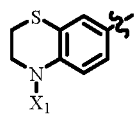 (I-3-2), 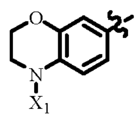 (I-3-3),

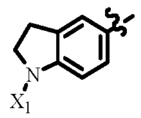 (I-3-4), 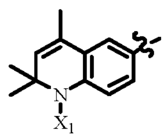 (I-3-5)

or, the conjugate system E and $-NX_1-X_2$ form a structure represented by Formula (I-3-6):

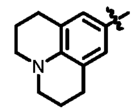 (I-3-6);

and
optionally, the electron acceptor part is one selected from Formulae (I-4-1) to (I-4-42) below:

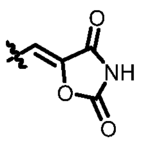 (I-4-1) 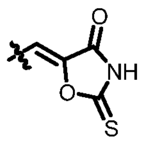 (I-4-2) 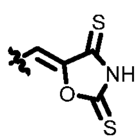 (I-4-3) 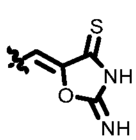 (I-4-4) 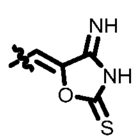 (I-4-5) 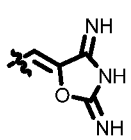 (I-4-6)

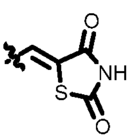 (I-4-7) 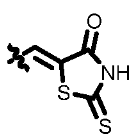 (I-4-8) 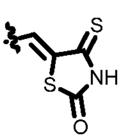 (I-4-9) 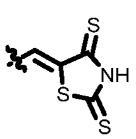 (I-4-10) 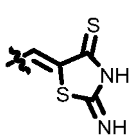 (I-4-11) 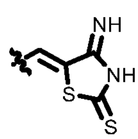 (I-4-12)

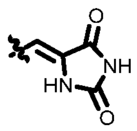 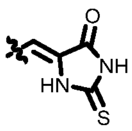 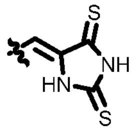 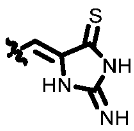 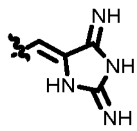 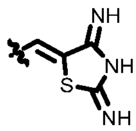

(I-4-13) (I-4-14) (I-4-15) (I-4-16) (I-4-17) (I-4-18)

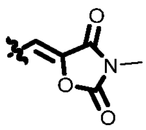 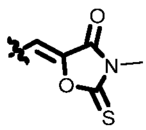 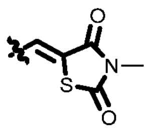 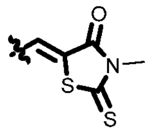 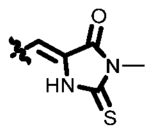 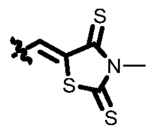

(I-4-19) (I-4-20) (I-4-21) (I-4-22) (I-4-23) (I-4-24)

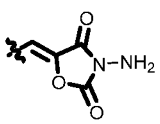 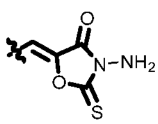 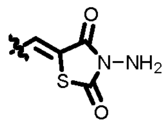 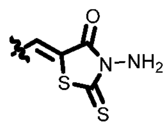 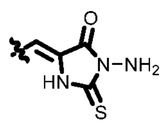 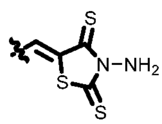

(I-4-25) (I-4-26) (I-4-27) (I-4-28) (I-4-29) (I-4-30)

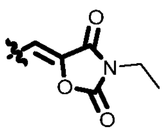 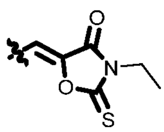 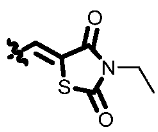 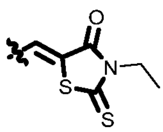 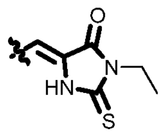 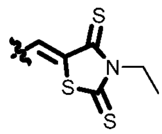

(I-4-31) (I-4-32) (I-4-33) (I-4-34) (I-4-35) (I-4-36)

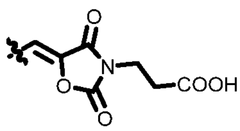 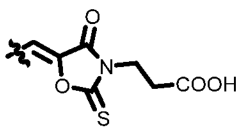 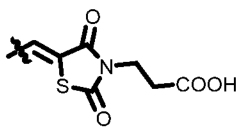 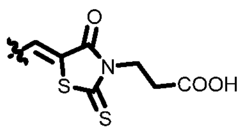

(I-4-37) (I-4-38) (I-4-39) (I-4-40)

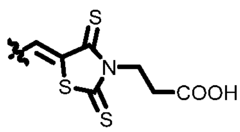 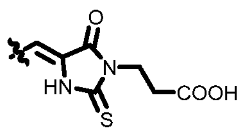

(I-4-41) (I-4-42).

**16.** Optionally, the above-mentioned fluorescent probe, wherein the fluorescent probe formula (I) is selected from compounds represented by the formulae below:

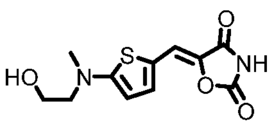 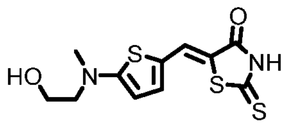 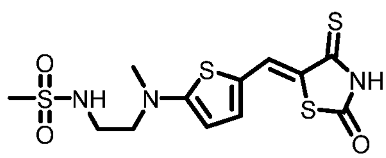

(I-5-1) (I-5-2) (I-5-3)

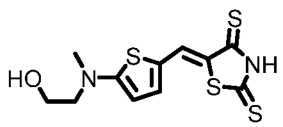

(I-5-4)

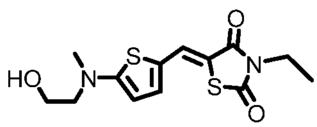

(I-5-5)

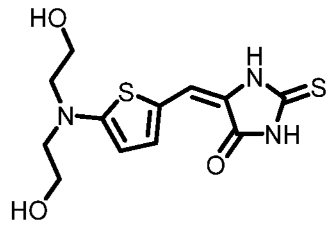

(I-5-6)

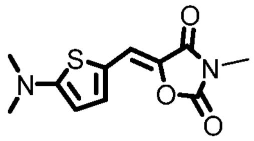

(I-5-7)

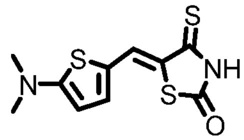

(I-5-8)

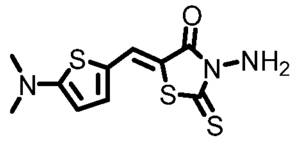

(I-5-9)

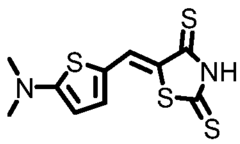

(I-5-10)

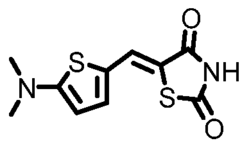

(I-5-11)

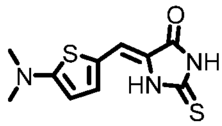

(I-5-12)

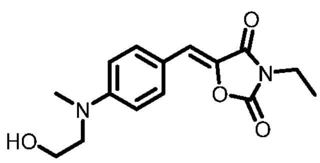

(I-5-13)

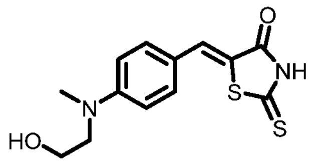

(I-5-14)

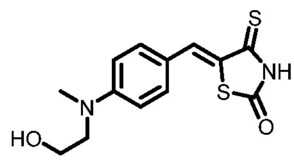

(I-5-15)

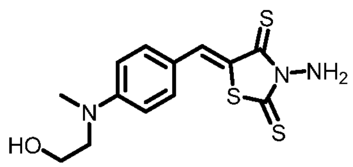

(I-5-16)

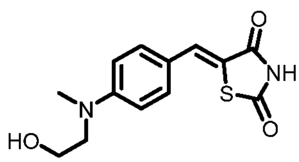

(I-5-17)

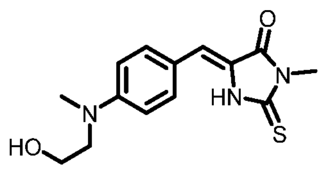

(I-5-18)

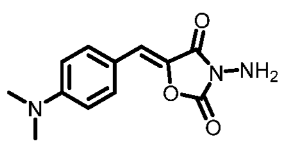

(I-5-19)

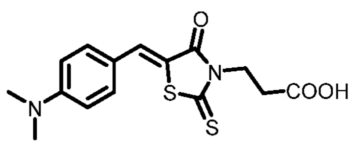

(I-5-20)

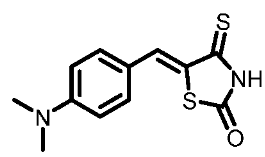

(I-5-21)

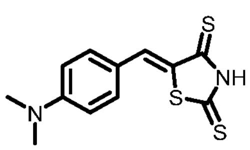

(I-5-22)

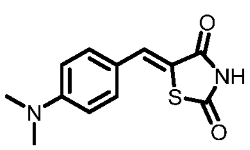

(I-5-23)

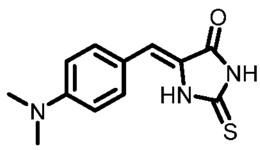

(I-5-24)

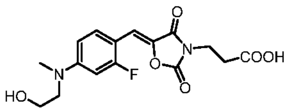
(I-5-25)

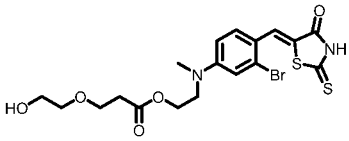
(I-5-26)

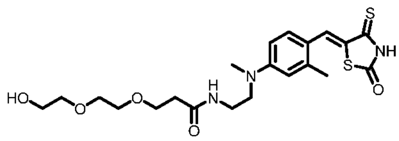
(I-5-27)

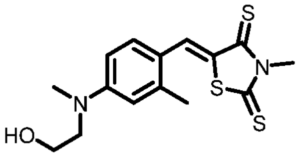
(I-5-28)

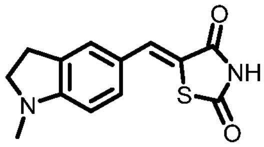
(I-5-29)

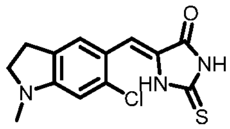
(I-5-30)

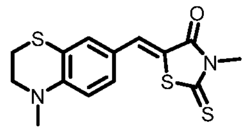
(I-5-31)

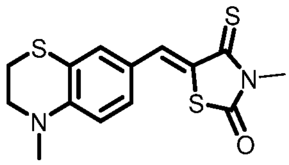
(I-5-32)

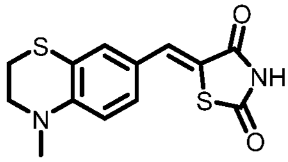
(I-5-33)

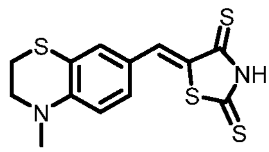
(I-5-34)

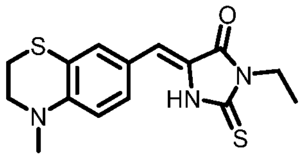
(I-5-35)

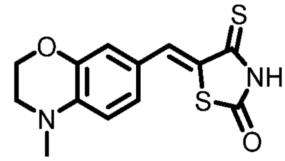
(I-5-36)

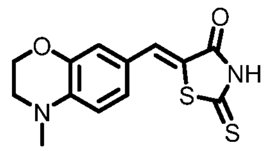
(I-5-37)

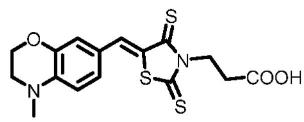
(I-5-38)

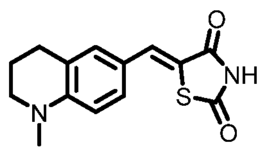
(I-5-39)

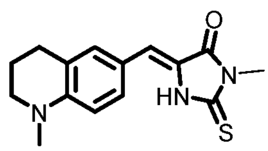
(I-5-40)

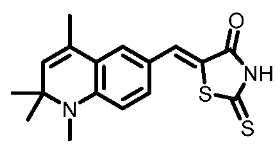
(I-5-41)

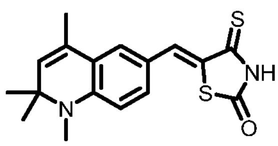
(I-5-42)

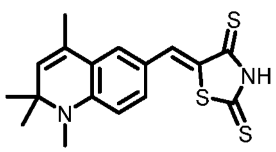
(I-5-43)

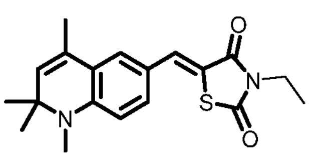
(I-5-44)

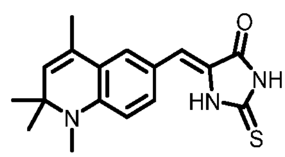
(I-5-45)

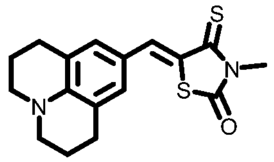
(I-5-46)

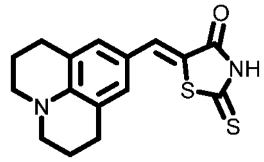
(I-5-47)

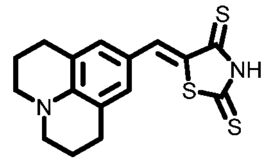
(I-5-48)

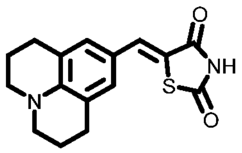
(I-5-49)

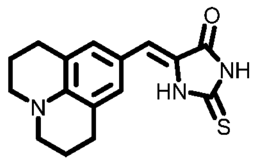
(I-5-50)

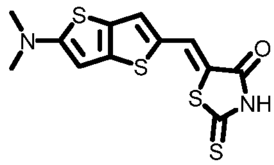
(I-5-51)

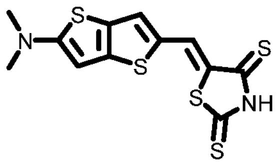
(I-5-52)

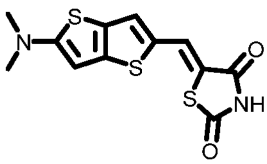
(I-5-53)

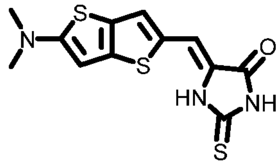
(I-5-54)

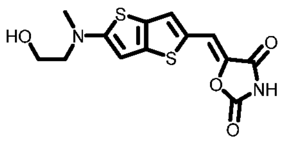
(I-5-55)

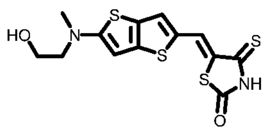
(I-5-56)

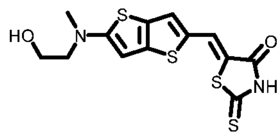
(I-5-57)

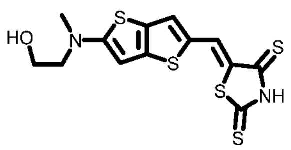
(I-5-58)

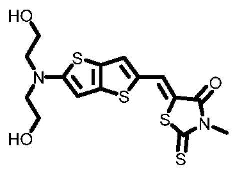
(I-5-59)

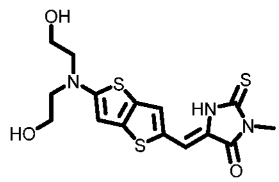
(I-5-60)

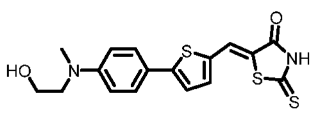
(I-5-61)

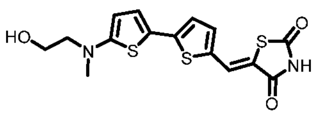
(I-5-62)

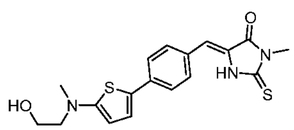
(I-5-63)

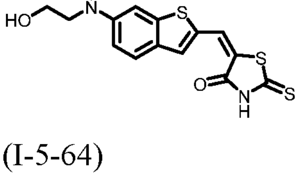
(I-5-64)

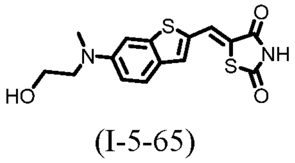
(I-5-65)

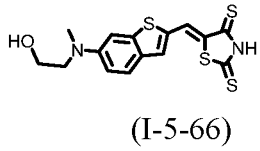
(I-5-66)

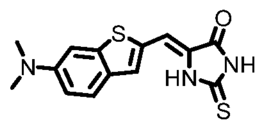
(I-5-67)

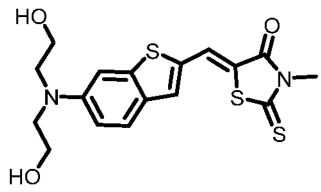
(I-5-68)

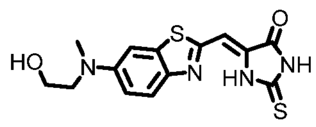
(I-5-69)

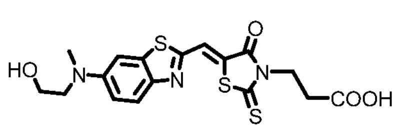
(I-5-70)

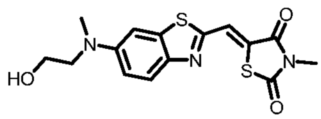
(I-5-71)

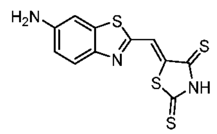
(I-5-72)

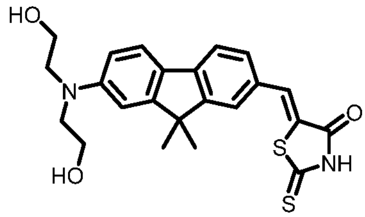
(I-5-73)

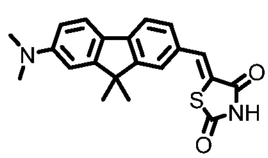
(I-5-74)

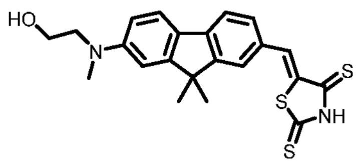
(I-5-75)

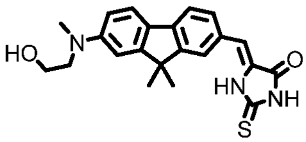
(I-5-76)

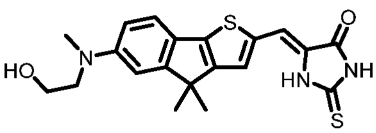
(I-5-77)

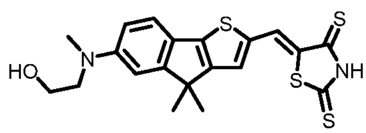
(I-5-78)

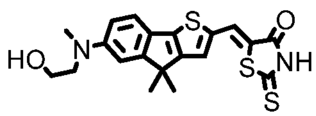
(I-5-79)

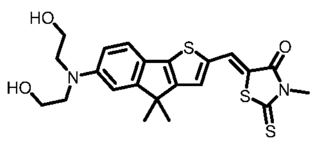
(I-5-80)

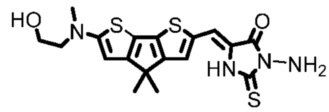
(I-5-81)

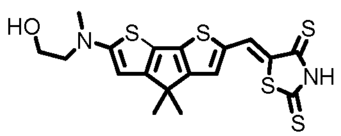
(I-5-82)

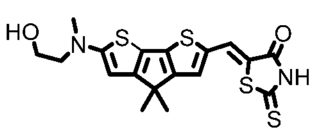
(I-5-83)

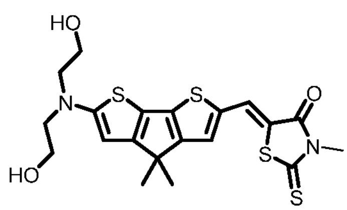
(I-5-84).

**17.** The complex according to claim 16, wherein the fluorophore molecules in the complex are selected from I-5-1, I-5-2, I-5-3, I-5-4, I-5-5, I-5-6, I-5-7, I-5-8, I-5-9, I-5-10, I-5-11, I-5-12, I-5-13, I-5-14, I-5-15, I-5-16, I-5-17, I-5-18, I-5-19, I-5-20, I-5-21, I-5-22, I-5-23, I-5-24, I-5-25, I-5-26, I-5-27, I-5-28, I-5-29, I-5-30, I-5-31, I-5-32, I-5-33, I-5-34, I-5-35, I-5-36, I-5-37, I-5-38, I-5-39, I-5-40, I-5-41, I-5-42, I-5-43, I-5-44, I-5-45, I-5-46, I-5-47, I-5-48, I-5-49, I-5-50, I-5-51, I-5-52, I-5-53, I-5-54, I-5-55, I-5-56, I-5-57, I-5-58, I-5-59, I-5-60, I-5-61, I-5-62, I-5-63, I-5-64, I-5-65, I-5-66,

I-5-67, I-5-68, I-5-69, I-5-70, I-5-71, I-5-72, I-5-73, I-5-74, I-5-75, I-5-76, I-5-77, I-5-78, I-5-79, I-5-80, I-5-81, I-5-82, I-5-83 and I-5-84.

**18.** The complex according to any one of claims 15 to 17, wherein the aptamer molecules in the complex contain a nucleotide sequence SEQ ID No: 1, 2, 3, 4, 5, 6, 7, 8, 9, 10, 11, 12 and 13.

**19.** The complex according to any one of claims 15 to 18 for detection or labeling of objective nucleic acid molecules in vitro or in vivo.

**20.** A DNA molecule, which transcribes the nucleic acid aptamer molecules according to any one of claims 1 to 14.

**21.** An expression vector containing the DNA molecule according to claim 20.

**22.** A host cell containing the expression system according to claim 21.

**23.** A kit, containing the nucleic acid aptamer molecule according to any one of claims 1 to 14 and/or the expression vector according to claim 21 and/or the host cell according to claim 22 and/or the complex according to any one of claims 15 to 18.

**24.** A method for detecting target molecules, including:

a) adding the complex according to any one of claims 15 to 18 to a solution containing the target molecules;
b) exciting the complex with light of appropriate wavelength; and
c) detecting fluorescence of the complex.

**25.** A method for extracting and purifying RNA, including extracting and purifying RNA with the complex according to any one of claims 15 to 18.

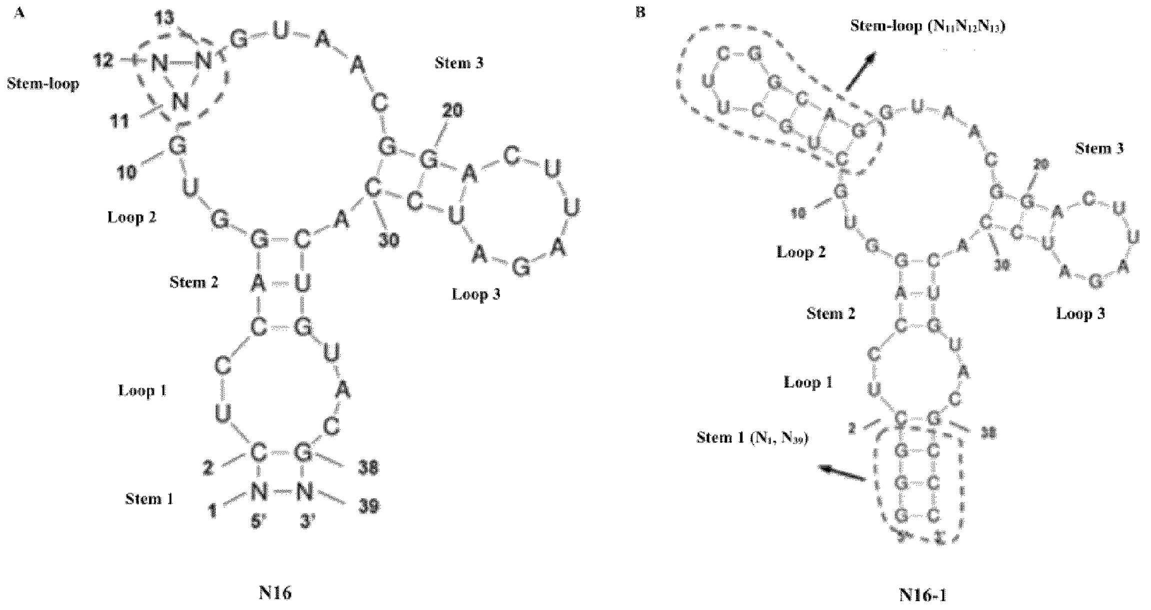
A
13
12
11
10
Stem-loop
Loop 2
Stem 2
Loop 1
Stem 1
Stem 3
Loop 3
20
30
2
38
1
39
5'
3'
N16
B
Stem-loop (N11N12N13)
Stem 3
Loop 2
Stem 2
Loop 1
Stem 1 (N1, N39)
Loop 3
N16-1

FIG. 1

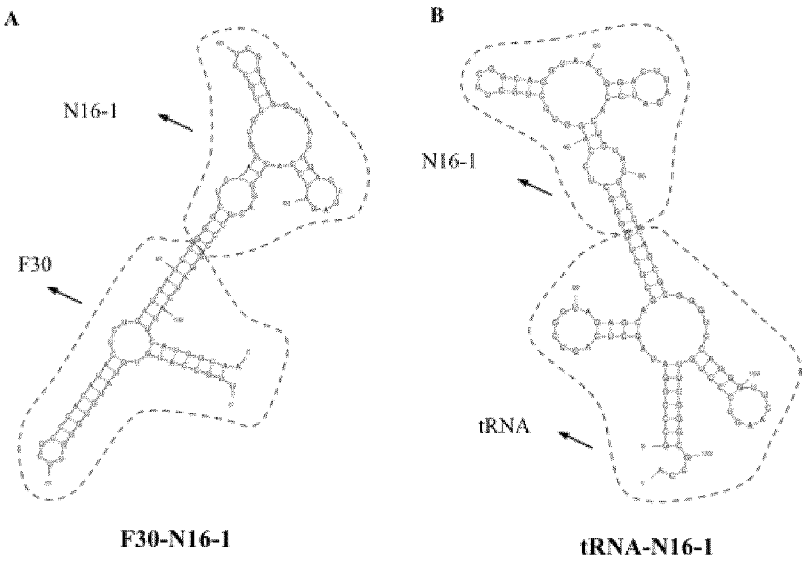
A
N16-1
F30
F30-N16-1
B
N16-1
tRNA
tRNA-N16-1

FIG. 2

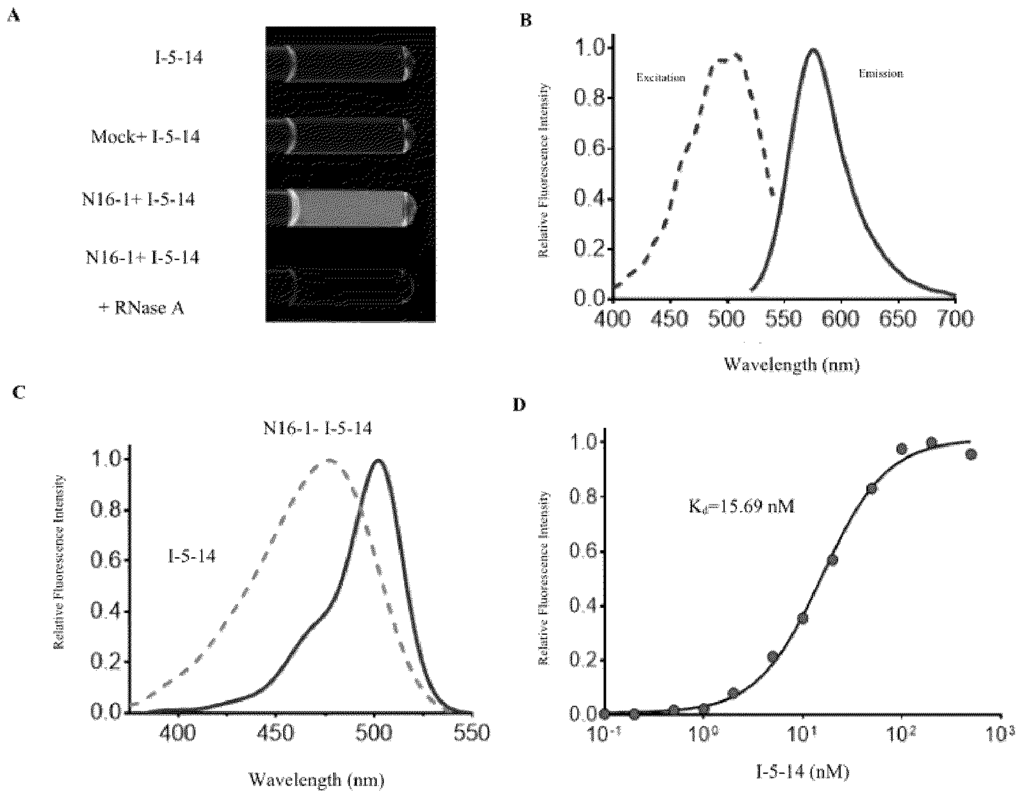
A
I-5-14
Mock+ I-5-14
N16-1+ I-5-14
N16-1+ I-5-14
+ RNase A
B
Excitation
Emission
Relative Fluorescence Intensity
Wavelength (nm)
C
N16-1- I-5-14
I-5-14
Relative Fluorescence Intensity
Wavelength (nm)
D
$K_d$=15.69 nM
Relative Fluorescence Intensity
I-5-14 (nM)

FIG. 3

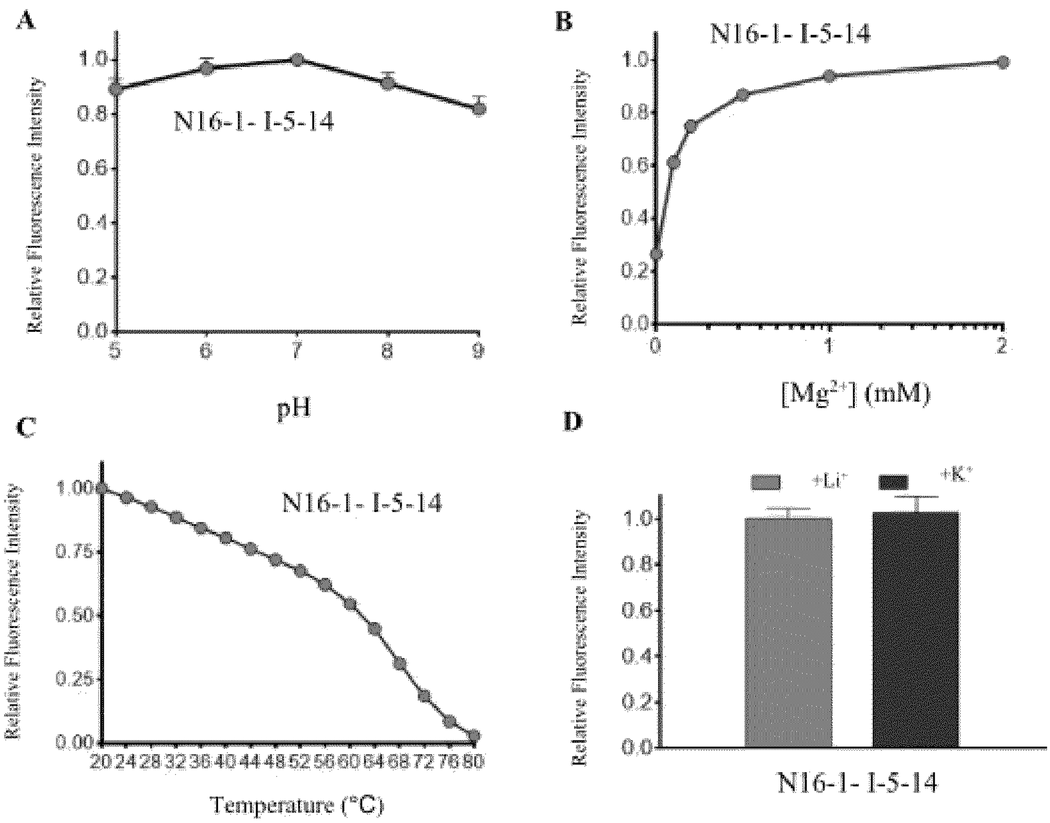
A
N16-1- I-5-14
Relative Fluorescence Intensity
1.0
0.8
0.6
0.4
0.2
0.0
5
6
7
8
9
pH
B
N16-1- I-5-14
Relative Fluorescence Intensity
1.0
0.8
0.6
0.4
0.2
0.0
0
1
2
[Mg2+] (mM)
C
N16-1- I-5-14
Relative Fluorescence Intensity
1.00
0.75
0.50
0.25
0.00
20 24 28 32 36 40 44 48 52 56 60 64 68 72 76 80
Temperature (°C)
D
+Li+
+K+
Relative Fluorescence Intensity
1.0
0.8
0.6
0.4
0.2
0.0
N16-1- I-5-14

FIG. 4

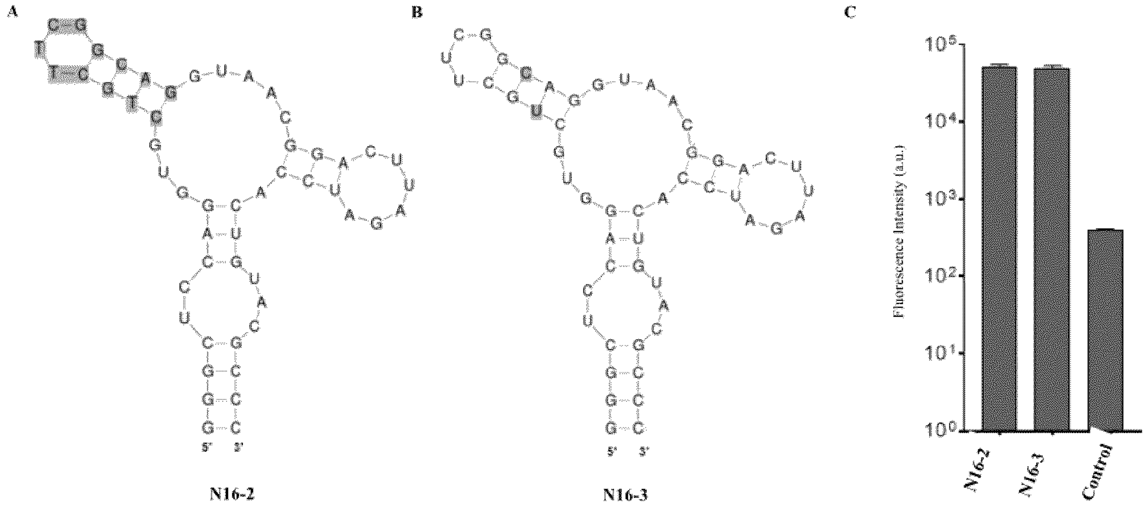


FIG. 5

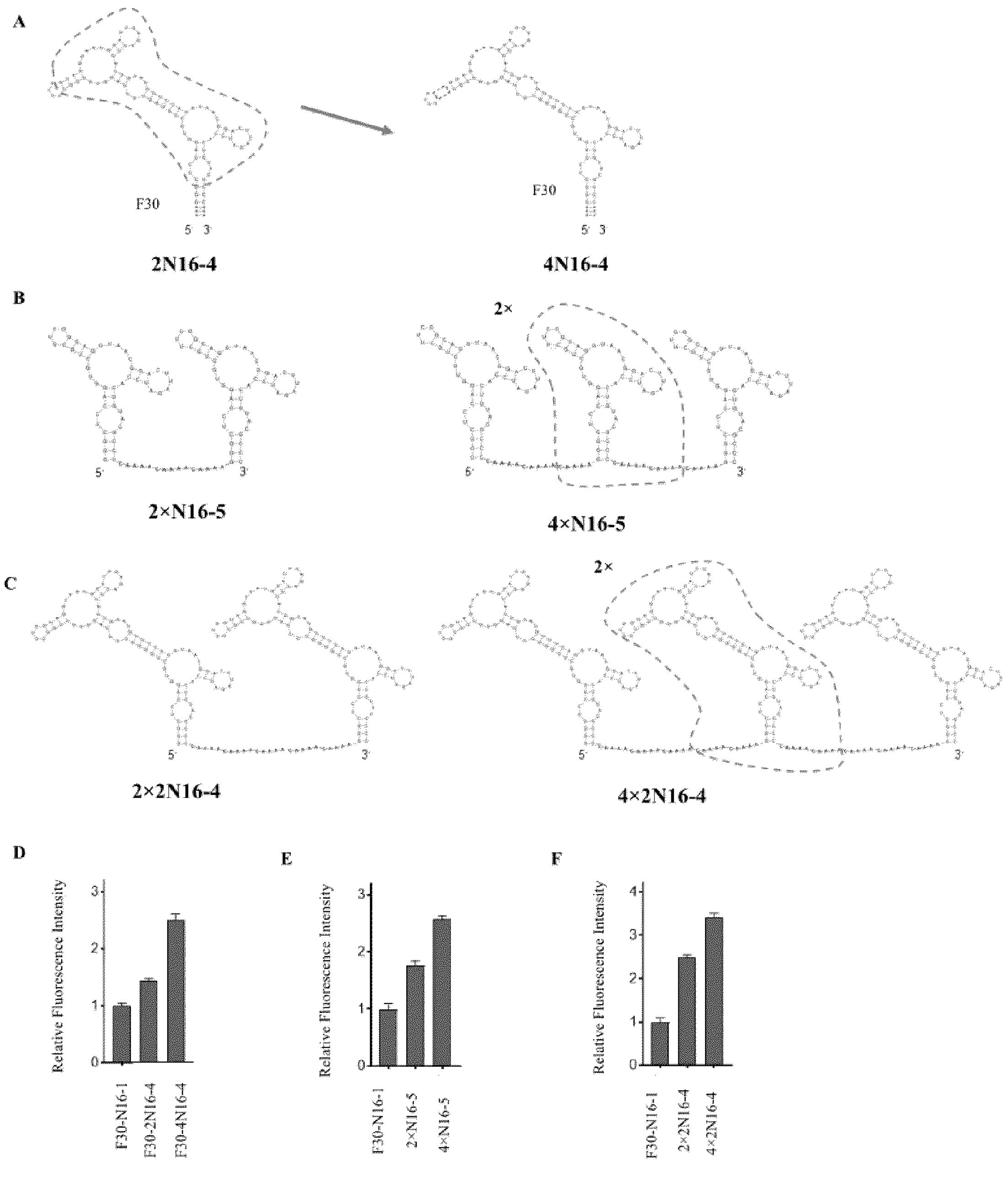
A
F30
5' 3'
2N16-4
F30
5' 3'
4N16-4
B
5' 3'
2×N16-5
2×
5' 3'
4×N16-5
C
5' 3'
2×2N16-4
2×
5' 3'
4×2N16-4
D
Relative Fluorescence Intensity
F30-N16-1
F30-2N16-4
F30-4N16-4
E
Relative Fluorescence Intensity
F30-N16-1
2×N16-5
4×N16-5
F
Relative Fluorescence Intensity
F30-N16-1
2×2N16-4
4×2N16-4

FIG. 6

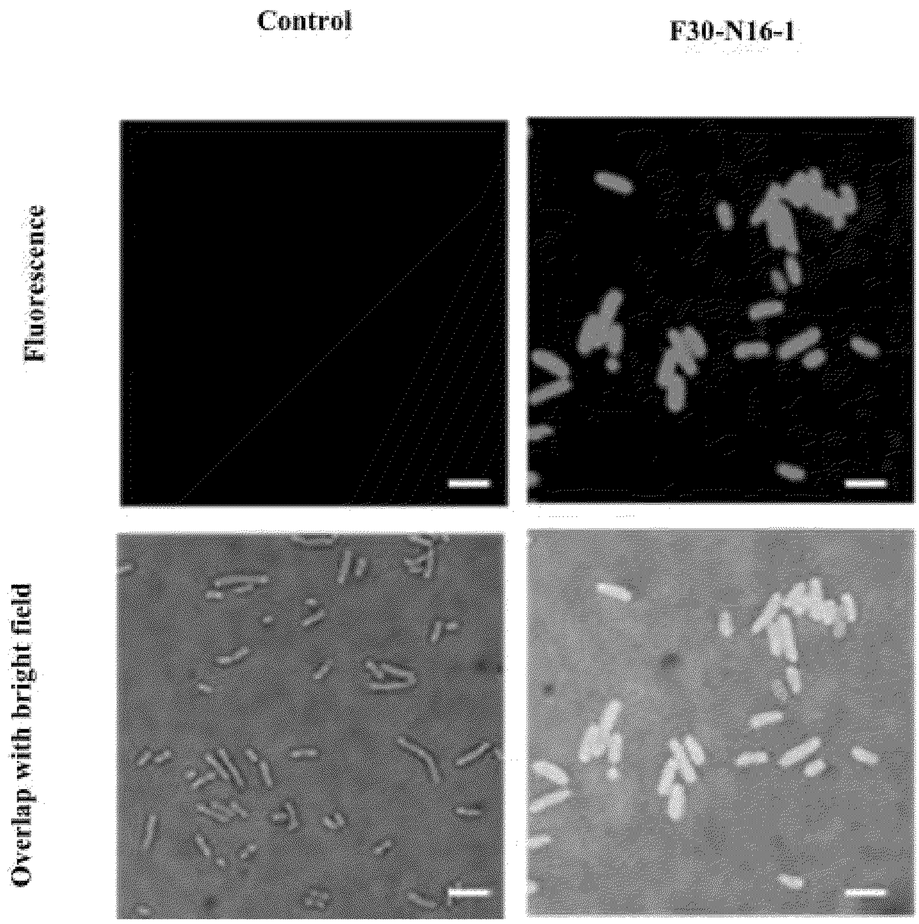
Control
F30-N16-1
Fluorescence
Overlap with bright field

FIG. 7

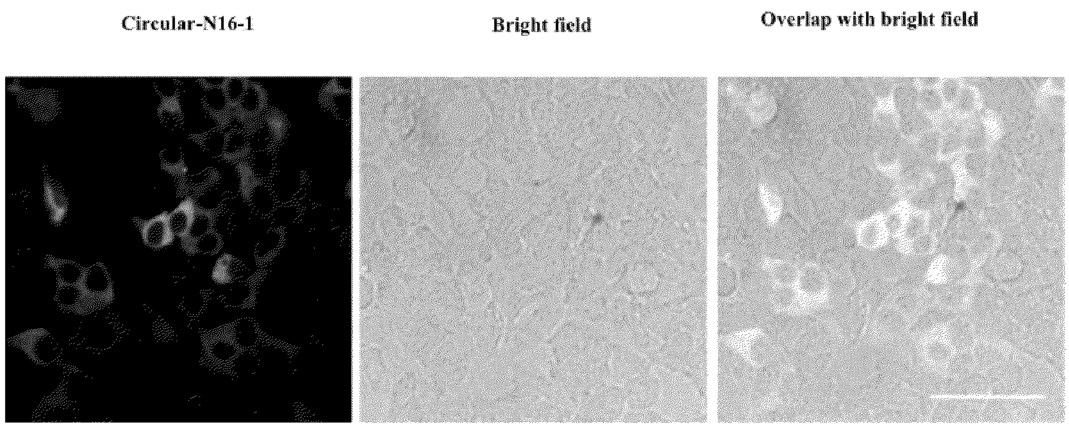
Circular-N16-1
Bright field
Overlap with bright field

FIG. 8

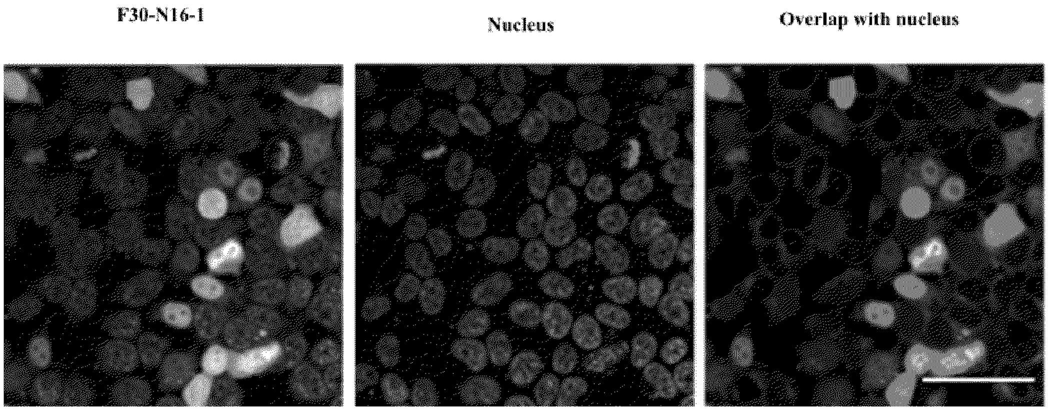
F30-N16-1
Nucleus
Overlap with nucleus

FIG. 9

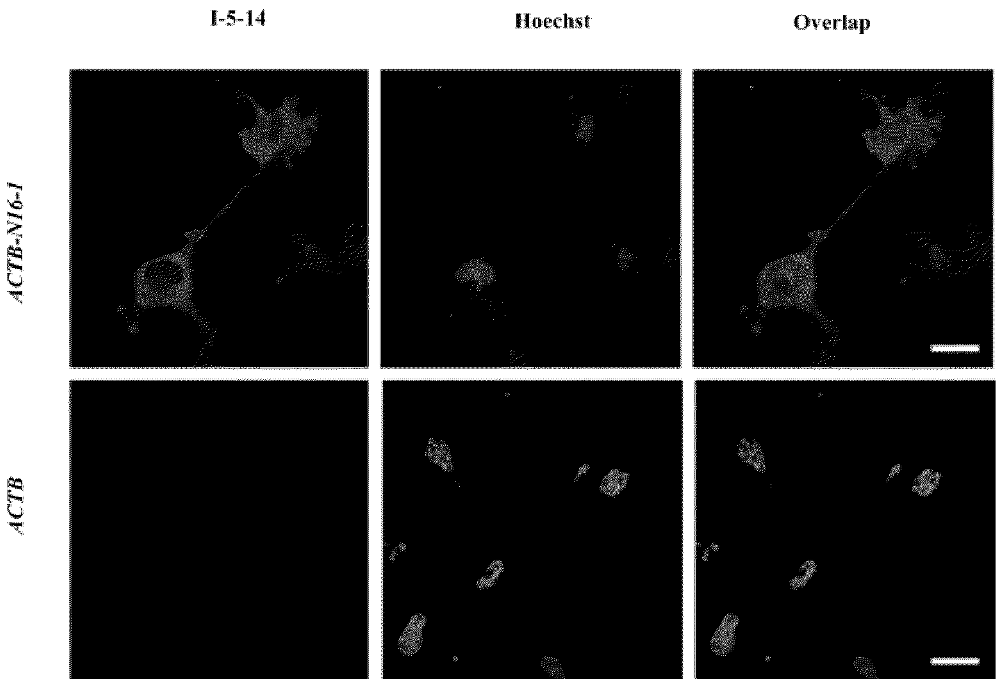
I-5-14
Hoechst
Overlap
ACTB-N16-1
ACTB

FIG. 10

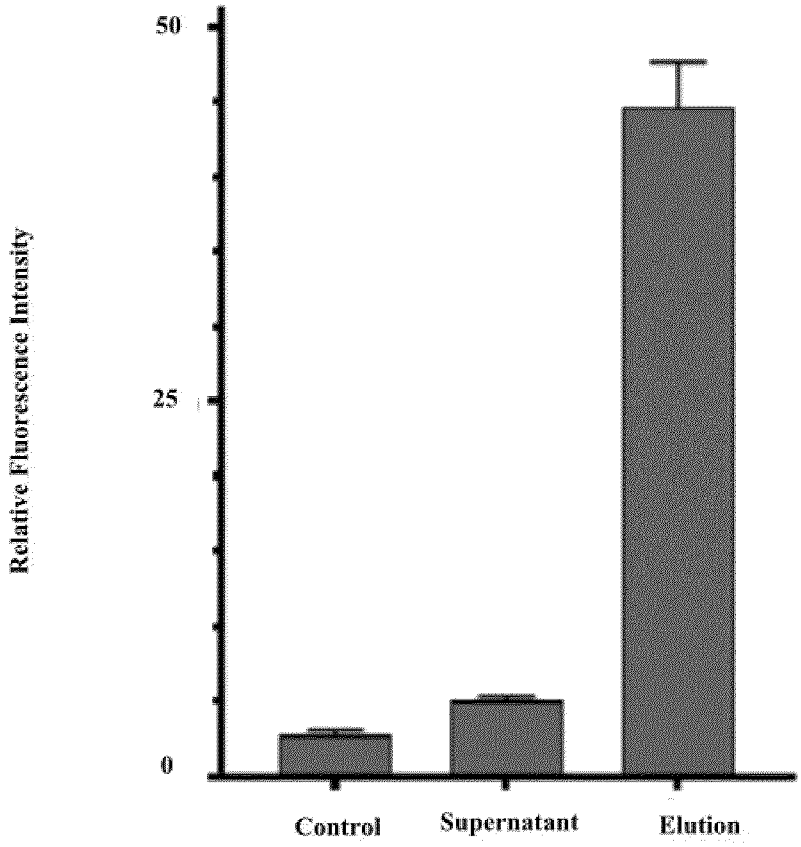
50
25
0
Relative Fluorescence Intensity
Control
Supernatant
Elution

FIG. 11

## INTERNATIONAL SEARCH REPORT

International application No.
**PCT/CN2022/110700**

**A. CLASSIFICATION OF SUBJECT MATTER**

C12N 15/115(2010.01)i; C09K 11/06(2006.01)i; C12N 15/10(2006.01)i; G01N 21/64(2006.01)i

According to International Patent Classification (IPC) or to both national classification and IPC

**B. FIELDS SEARCHED**

Minimum documentation searched (classification system followed by classification symbols)

C12N; C09K; C12N; G01N

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CNKI, PubMed, GenBank, STN, 中国专利生物序列数据库, Chinese Patent Biological Sequence Retrieval System: 适配体, 荧光RNA, 互补, 配对, 复合体, 检测, 定量, aptamers, RNA, fluorescent RNA, complementary, stem, pepper, complex, detect+, quantitat+, SEQ ID NOs: 1-13, I-2-1-I-2-39, I-3-1-I-3-6, I-4-1-I-4-42, I-5-1-I-5-84

**C. DOCUMENTS CONSIDERED TO BE RELEVANT**

| Category* | Citation of document, with indication, where appropriate, of the relevant passages | Relevant to claim No. |
|---|---|---|
| A | CN 112538482 A (EAST CHINA UNIVERSITY OF SCIENCE AND TECHNOLOGY) 23 March 2021 (2021-03-23)<br>entire document | 1-25 |
| A | CN 111593052 A (EAST CHINA UNIVERSITY OF SCIENCE AND TECHNOLOGY) 28 August 2020 (2020-08-28)<br>entire document | 1-25 |
| A | CN 111592472 A (SHANGHAI GAOCHI ASSET MANAGEMENT CO., LTD.) 28 August 2020 (2020-08-28)<br>entire document | 1-25 |
| A | TRUONG, L. et al. "From fluorescent proteins to fluorogenic RNAs: Tools for imaging cellular macromolecules."<br>*PROTEIN SCIENCE.*, Vol. 28, 11 May 2019 (2019-05-11),<br>pp. 1374-1386 | 1-25 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 October 2022** | **26 October 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No. **PCT/CN2022/110700**

C. DOCUMENTS CONSIDERED TO BE RELEVANT

| Category* | Citation of document, with indication, where appropriate, of the relevant passages | Relevant to claim No. |
|---|---|---|
| A | FILONOV, G. S. et al. "Broccoli: Rapid Selection of an RNA Mimic of Green Fluorescent Protein by Fluorescence-Based Selection and Directed Evolution." *J.A.C.S.*, Vol. 136, 22 October 2014 (2014-10-22), pp. 16299-16308 | 1-25 |
| A | CHEN, Xianjun et al. "Visualizing RNA dynamics in live cells with bright and stable fluorescent RNAs." *Nature Biotechnology*, Vol. 37, 30 November 2019 (2019-11-30), pp. 1287-1293 | 1-25 |
| A | 左方婷等. (ZUO, Fangting et al.). "荧光RNA: 点亮神秘的基因组暗物质. ( Fluorescent RNA: Illuminating the Genomic Mystic Matter)" *SCIENCE (KEXUE). www.kexuemag.cn*, Vol. 73, No. 4, 25 July 2021 (2021-07-25), pp. 25-29 | 1-25 |

**INTERNATIONAL SEARCH REPORT**
**Information on patent family members**

International application No.
**PCT/CN2022/110700**

| Patent document cited in search report | Publication date (day/month/year) | Patent family member(s) | Publication date (day/month/year) |
|---|---|---|---|
| CN 112538482 A | 23 March 2021 | None | |
| CN 111593052 A | 28 August 2020 | None | |
| CN 111592472 A | 28 August 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **OZAWA et al.** *Nature Methods,* 2007, vol. 4, 413-419
- **PAIGE et al.** *Science,* 2011, vol. 333, 642-646
- **FILONOV et al.** *Journal of the American Chemical Society,* 2014, vol. 136, 16299-16308
- **LI et al.** *Angewandte Chemie International Edition in English,* 2020, vol. 59, 4511-4518
- **SONG et al.** *Nature Chemical Biology,* 2017, vol. 13, 1187-1194
- **NEEDLEMAN ; WUNSCH.** *J.Mol.Biol.,* 1970, vol. 48, 443-453
- **RICE.** EMBOSS: The European Molecular Biology Open Software Suite. *Trends in Genetics,* 2000, vol. 16, 276-277
- **JANE ROSKAMS.** A Handbook of Recipes, Reagents, and Other Reference Tools for Use at the Bench
- **SAMBROOK.J ; D. W. RUSSELL.** Molecular Cloning-A Laboratory Manual. Science Press, August 2002
- **CHEN et al.** *Nature biotechnology,* 2019, vol. 37, 1287-1293